(19) Europäisches Patentamt
European Patent Office
Office européen des brevets

(11) EP 3 978 503 A1

(12) **EUROPEAN PATENT APPLICATION**
published in accordance with Art. 153(4) EPC

(43) Date of publication:
**06.04.2022 Bulletin 2022/14**

(21) Application number: **20818731.0**

(22) Date of filing: **05.03.2020**

(51) International Patent Classification (IPC):
***C07D 498/14*** $^{(2006.01)}$ ***C07D 498/18*** $^{(2006.01)}$
***A61K 31/5365*** $^{(2006.01)}$ ***A61K 31/537*** $^{(2006.01)}$
***A61K 31/553*** $^{(2006.01)}$ ***A61K 31/4985*** $^{(2006.01)}$
***A61P 31/18*** $^{(2006.01)}$

(86) International application number:
**PCT/RU2020/000118**

(87) International publication number:
**WO 2020/246910 (10.12.2020 Gazette 2020/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.06.2019 RU 2019117193**

(71) Applicants:
- **Ivachtchenko, Alena Alexandrovna**
  **Hallandale, Florida 33009 (US)**
- **Alla Chem, LLC**
  **Hallandale Beach, FL 33009 (US)**
- **Ivachtchenko, Alexandre Vasilievich**
  **Hallandale Beach, FL 33009 (US)**
- **Savchuk, Nikolay Filippovich**
  **Rancho Santa Fe, CA 92067 (US)**
- **Ivashchenko, Andrey Alexandrovich**
  **Moscow 127576 (RU)**

(72) Inventors:
- **MITKIN, Oleg Dmitrievich**
  **Khimki, 141401 (RU)**
- **IVACHTCHENKO, Alexandre Vasilievich**
  **Hallandale, Florida 33009 (US)**
- **SAVCHUK, Nikolay Filippovich**
  **Rancho Santa Fe, California 92067 (US)**
- **IVASHCHENKO, Andrey Alexandrovich**
  **Moscow, 127576 (RU)**

(74) Representative: **Zellentin & Partner mbB**
**Patentanwälte**
**Rubensstraße 30**
**67061 Ludwigshafen (DE)**

(54) **ANNELATED 9-HYDROXY-1,8-DIOXO-1,3,4,8-TETRAHYDRO-2H-PYRIDO[1,2-a]PYRAZINE-7-CARBOXAMIDES AS HIV INTEGRASE INHIBITORS**

(57) The present invention relates to a novel compound that has antiviral activity, in particular, inhibitory activity against the integrase of the human immunodeficiency virus (HIV).

The subject of this invention is a novel annelated 9-hydroxy-1,8-dioxo-1,3,4,8-tetrahydro-2H-pyrido[1,2-a]pyrazine-7-carboxamide of general formula 1 or 2, or any stereoisomer, any pharmaceutically acceptable salt, any solvate, or any crystalline or polycrystalline form thereof

EP 3 978 503 A1

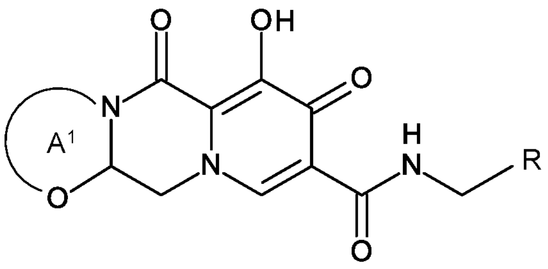

,

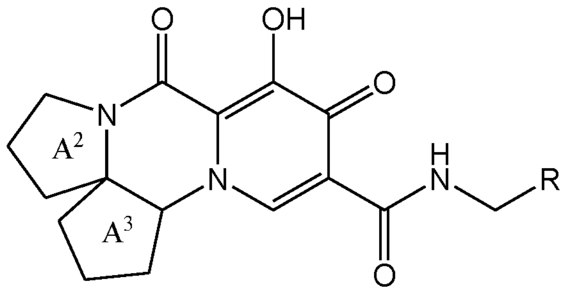

1

2

wherein

ring $A^1$ is an optionally methyl-substituted 5-7 membered saturated heterocycle or heterobicycle;

ring $A^2$ is a 5-6 membered optionally methyl-substituted saturated or partially saturated monocyclic heterocycle;

ring $A^3$ is a 5-6 membered monocyclic saturated cycloalkane or tetrahydro-2H-pyran;

R is a 5-7 membered optionally substituted with one, two, or three optionally identical substituents monocyclic or bicyclic heterocyclic radical comprising 1-4 heteroatoms selected from the series O, S, and N except (2*S*,5*R*,13a*S*)-8-hydroxy-7,9-dioxo-*N*-{[3-(trifluoromethyl)-pyridin-2-yl]methyl}-2,3,4,5,7,9,13,13a-octahydro-2,5-methanopurido[1',2':4,5]pyrazino[2,1-*b*][1,3]oxazepine-10-carboxamide (formula A4) and (1*R*,4*S*,12a*R*)-*N*-[(3,5-difluoropyridin-2-yl)methyl]-7-hydroxy-6,8-dioxo-1,2,3,4,6,8,12,12a-octahydro-1,4-methanodipyrido[1,2-*a*:1',2'-*d*]pyrazine-9-carboxamide (formula A5).

**Description**

**FIELD OF THE INVENTION**

The present invention relates to a novel compound that has antiviral activity, in particular, inhibitory activity against the integrase of the human immunodeficiency virus (HIV).

**BACKGROUND OF THE INVENTION**

HIV is a lentivirus (a subgroup of retroviruses) that causes an indolent disease-HIV-infection [Weiss R.A. How does HIV cause AIDS. Science 1993, 260 (5112), 1273-1279. Douek D.C., Roederer M., Koup R.A. Emerging Concepts in the Immunopathogenesis of AID». Annu. Rev. Med. 2009, 60, 471-84]. The human immunodeficiency virus was independently discovered in 1983 at two laboratories: one by a research team led by Luc Montagnier at the Pasteur Institute in France and the other, led by Robert Gallo at the National Cancer Institute in the United States. The findings discussing the first isolation of a new retrovirus from tissues of patients with symptoms of AIDS were published on May 20, 1983 in the journal *Science* [Barré-Sinoussi F. at al. Isolation of a T-lymphotropic retrovirus from a patient at risk for acquired immune deficiency syndrome (AIDS). Science 1983, 220 (4599), 868-871. Gallo R. C. at al. Isolation of human T-cell leukemia virus in acquired immune deficiency syndrome (AIDS). Science 1983, 220 (4599), 865-867.]. In 2008, Luc Montagnier and Françoise Barré-Sinoussi shared the Nobel Prize for Physiology and Medicine for their "discovery of the human immunodeficiency virus."

The HIV virus infects the cells of the immune system that have CD4 receptors on their surface: T helpers, monocytes, macrophages, Langerhans cells, dendritic cells, microglial cells. This leads to immunodepression and development of Acquired Immune Deficiency Syndrome (AIDS); loss of patients' ability to protect themselves against infections and tumors; emergence of secondary opportunistic diseases that are not typical for people with normal immune status. Without treatment, average survival time after infection with HIV is estimated to be 9 to 11 years, depending on the HIV subtype [https://en.wikipedia.org/wiki/HIV].

According to the worldwide statistics [http://www.lenoblspid.ru/news24/postid/own_news/1166], in 2015, 36.7 million people globally were living with HIV, 2.1 million people were newly infected with HIV, and 1.1 million people died of AIDS-related illnesses. Since the start of the epidemic, 78 million people have become infected with HIV, of which 35 million people have died of AIDS-related illnesses.

HIV is transmitted between people through the exchange of body fluids, such as blood, semen, rectal and vaginal fluids, and breast milk. It is not transmitted through saliva.

HIV can be suppressed by combination antiretroviral therapy (ART) involving three or more antiretroviral drugs with different mechanisms of action. ART does not cure HIV infection but suppresses viral replication within a person's body and assists in strengthening the immune system and regaining its capacity to fight off infections. Life expectancy for patients receiving ART using combinations of single-component and/or two-component drugs may be extended to 70-80 years [http://www.who. int/mediacentre/factsheets/ fs360/ru/].

Antiretroviral drugs (ARDs) are divided into nucleoside reverse transcriptase inhibitors (NRTIs), non-nucleoside reverse transcriptase inhibitors (NNRTIs), fusion inhibitors, capsid inhibitors, protease inhibitors (PIs), and integrase inhibitors (INIs).

Examples of single-component ARDs can be Elsulfavirine, VM-1500A [WO 2005/102989, RU 2389719, WO 2010/028968], Rilpivirine [https://pubchem.ncbi.nlm. nih.gov/compound/Rilpivirine#section=Top; https://pubchem.ncbi.nlm.nih.gov/compound/ 5270790#section=Top], and Efavirenz [https://pubchem.ncbi.nlm.nih.gov/compound/efavirenz] are NNRTIs; Lamivudine [https://pubchem.ncbi.nlm.nih.gov/compound/ lamivudine# section=2D-Structure], Emtricitabine [https://pubchem.ncbi.nlm.nih.gov/ compound/ Emtricitabine#section=2D-Structure] snd their proinhibitors [RU 2659388] are NRTIs; Tenofovir Disoproxil Fumarate (Viread®) [https://pubchem.ncbi.nlm.nih.gov/ compound/Tenofovir_Disoproxil_Fumarate], tenofovir alafenamide hemifumarate (TAF, GS-7340, Vemlidy) [https://pubchem.ncbi.nlm.nih.gov/compound/71492247], Tenofovir cyclobutylan афенамид Tenofovir cyclobutyl alafenamide (TCBA) and TCBA fumarate [RU 2647576] are NtRTIs (не было расшифровки); Elvitegravir [https://pubchem. ncbi.nlm.nih.gov/compound/Elvitegravir] is INI; GS-CA1 (GS-6207) [WO2018035359; http://www.natap.org/2018/IDWeek/IDWeek 03.htm] is a capsid inhibitor; and Cobicistat [https://pubchem.ncbi. nlm.nih.gov/compound/Cobicistat], which does not exhibit antiviral activity but is a pharmacokinetic enhancer, an inhibitor of cytochrome P450 3A (CYP3A).

In recent years, considerable attention has been paid to INIs as drugs for combined ART that involvies simultaneous use of several ARGs with different mechanisms of action, in particular, for long-term HIV suppression (Antiretroviral Therapy as Long Acting Suppression, ATLAS).

In 2007, the U.S. Food and Drug Administration (FDA) approved the use of INIs HIV as ART drugs [https://www.healthline. com/health/hiv-aids/integrase-inhibitors#hiv].

The first FDA-registered (October, 2007) INI drug for ART was Raltegravir, developed by Merk [https://aidsinfo.nih. gov/news/803/fda-approves-the-first-integrase-inhibitorraltegravir---october-12-2007], which was patented by the Shionogi company in Europe [EP1422218 (2004). Shionogi later patented Cabotegravir and Dolutegravir [WO 2006/116764 US 8129385 (2012), US 8778943 (2015), EP 3260457 (2017)].

| | |
|---|---|
| 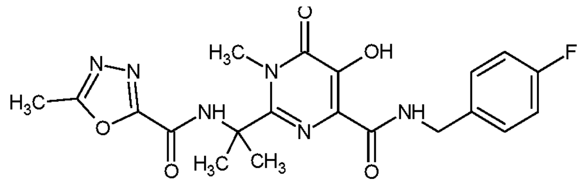 Raltegravir | 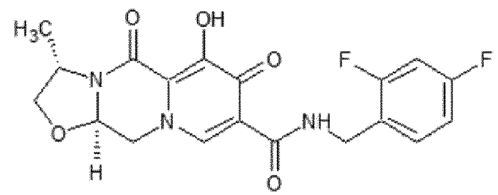 Cabotegravir (CAB) |
| 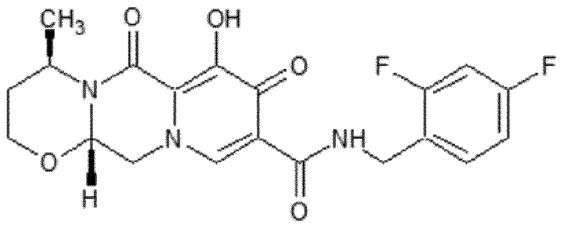 Dolutegravir (DTG) | 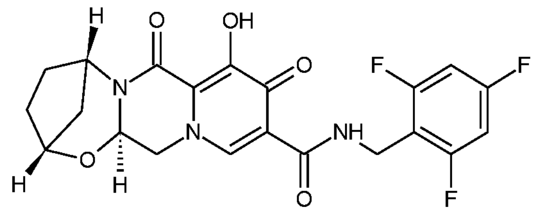 Bictegravir (BIC) |

Later, Bictegravir [WO 2014/100323], INIs A1, A2 [EP 3196201], and INIs A3 [WO 2016161382] were patented.

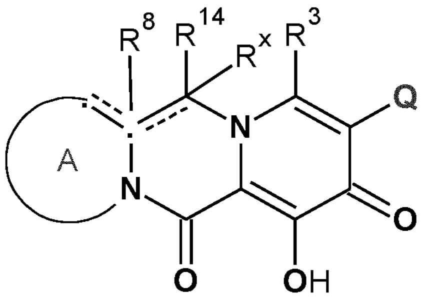

**A1**

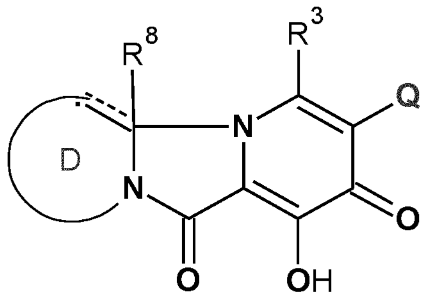

**A2**

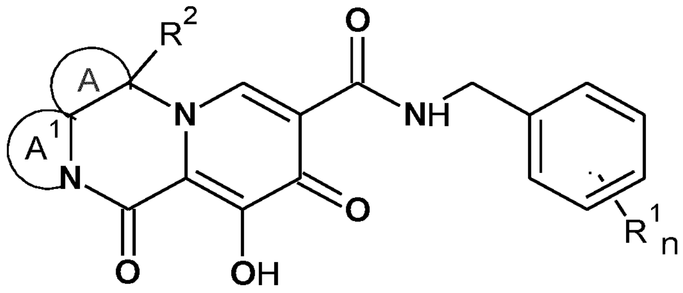

**A3**

,

wherein A1, A2: Q s an optionally substituted carbon- or heterocycle; A and D are optionally substituted heterocycles; $R^3$ and $R^8$ are independently hydrogen. halogen, hydroxy, optionally substituted lower alkyl and other substituents; $R^{14}$ and $R^x$ are independently hydrogen, optionally substituted lower alkyl, and other substituents.

A3: A is an optionally substituted 3-7 membered cycloalkyl or partially unsaturated heterocycle; $A^1$ is a 5-7 membered saturated or optionally substituted 4-7 membered monocyclic heterocycle; $R^1_n$ is an optionally identical halogen or C1-C3 alkyl; n = 1-3; $R^2$ is an optionally identical H or C1-C4 alkyl.

Currently, CAB, DTG, and BIC are the most advanced HIV INIs. Depending on the method of analysis, the inhibitory activity for CAB is $EC_{50}$ = 0.25-3.0 nM[a,b]; for DTG, $EC_{50}$= 1.1-7.4 nM[a-c]; and for BIC, $EC_{50}$ = 1.0-7.5 nM[b,c] [[a]Hassounah S.A. et al. AAC 2017, 61(12), pii: e01695-17. [b]Yoshinaga T. et al. AAC 2015, 59(1), 397-406; https://aac.asm.org/content/aac/59/1/397full.pdf; https://aac. asm.org/content/ 61/12/e01695-17.long. [c]Tsiang M. et al. AAC 2016, 60(12), 7086-7097; https://aac.asm.org/content/60/ 12/7086].

Dolutegravir (brand name: Tivicay) was approved by the FDA in August 2013 rhttps://www.drugs.com/history/tivicay.html, and in November 2017, the FDA approved the drug Juluca [https://www.drugs.com/history/juluca.html], which is a fixed combination of 52.6 mg of Dolutogravir sodium salt and o 27.5 mg of Rilpivirine hydrochloride [https://www.gsksource.com/pharma/ content/dam/Glaxo

SmithKline/US/en/Prescribing_Information/Juluca/pdf/JULUCA-PI-PIL.PDF].

Bictegravir is part of the drug Bictarvi, which is a fixed combination (50 mg of Bictegravir, 200 mg of Emtricitabine, and 25 mg of tenofovir alafenamidee) [http://www.gilead.com/~/media/files/pdfs/medicines/hiv/ biktarvy/biktarvy_pi.pdf]. Bictarvi was approved by the FDA in 2018 [https://www.google.com/ search?q=biktarvy+fda+approval+date&rlz=1C1GGRV_enUS751US751&oq=biktarvy+FD A&aqs=chrome.2.0j69i57j0l2.20836j0j4&sourceid=chrome&ie=UTF-8].

Cabotegravir is beingactively studied in the clinic in the form of an oral tablet and in a long-acting injectable nanosuspension to be injected into the muscle (known as Cabotegravir LA or CAB LA (LA means "long-acting") [https://aidsinfo.nih.gov/drugs/513/cabotegravir/0/patient. Trezza C. et al. Formulation and pharmacology of long-acting cabotegravir. Curr. Opin. HIV AIDS. 2015, 10(4), 239-245. T.D. McPherson et al. Cabotegravir in the treatment and prevention of Human Immunodeficiency Virus-1. Expert Opin Investig Drugs. 2018, 27(4), 413-420. C.D. Andrews et al. Cabotegravir long acting injection protects macaques against intravenous challenge with SIVmac251. AIDS 2017, 31(4), 461-467.].

Cabotegravir is currently being actively researched as a drug in clinical phases III ATLAS (NCT02951052), FLAIR (NCT02938520), ATLAS-2M (NCT03299049), and ACTG A5359 (NCT03635788) [https://aidsinfo.nih.gov/drugs/513/ cabotegravir/0/patient].

Cabotegravir and A1-A3 inhibitors have not yet been approved by the FDA.

Despite the results achieved in recent years in the development of INIs drugs for combined therapy, the search for new drugs of this type with improved characteristics remains relevant.

Listed below are definitions of various terms used to describe this invention. These definitions apply to the terms as they are used throughout this specification and claims, unless otherwise limited in specific instances, either individually or as part of a larger group.

The term "HIV infection" is a disease caused by a retrovirus, leading to progressive immunodeficiency (AIDS) and characterized by the addition of opportunistic diseases in the terminal phase.

The term "AIDS-associated complex (AAC) means the early symptomatic stage of HIV. This stage is associated with a risk of opportunistic infections. The clinical manifestation of AAC is accompanied by the appearance of constitutional symptoms: fever, profuse night sweats, weight loss of 10% or more, progressive weakness. The characteristic features include the appearance of dermatological symptoms, damage to the oral mucosa, recurrent herpetic infection, and recurrent cutaneous-mucous candidiasis. Often, diseases of the upper respiratory tract (sinusitis, bronchitis, pneumonia), inflammatory diseases of the pelvic organs, cervical dysplasia, peripheral neuropathy join in.

The term "AIDS" means acquired immunodeficiency syndrome or late symptomatic stage. The infectious process lasts for 7-10 years. In some cases, the disease develops faster and after 2-3 years passes into the terminal stage. This stage is characterized by severe life-threatening infections and malignant neoplasms, which have a generalized form. Lesions of organs and systems in patients are irreversible.

The term "halogen," or "halo," means fluorine, bromine, chlorine, and iodine.

The term "optionally" means that a subsequently described event or circumstance may or may not occur, and that the description includes cases where the specified event or circumstance occurs and cases in which it does not occur.

The term "crystalline form" means a substance structure characterized by packing constituent molecules into some type of crystal lattice.

The term "polycrystalline form" means a polycrystalline structure of a substance, i.e. a structure consisting of multiple small single crystals, or crystallites of a certain crystalline form.

The term "solvate" refers to the products of the addition of a solvent to dissolved substances. A special case of solvates is hydrates (with water acting as the solvent). Solvates are usually formed in a solution, but often (upon cooling the solution, evaporation of the solvent, etc.) solvates can be obtained in the form of crystalline phases named crystalline solvates.

The term "stereoisomers" (spatial isomers) are chemical compounds having the same structure but differing in the spatial arrangement of atoms. Stereoisomers, which are mirror images of each other that are not compatible in space, are called enantiomers or optical isomers. Optical isomerism is characteristic of compounds whose molecules have chiral elements, for example, an asymmetric (chiral) carbon atom bonded to four different substituents. It was first discovered by L. Pasteur in 1848 using tartaric acid as an example and explained by J.X. Van Hoff and J.A. Le Belem in 1874 based on the concept of tetrahedral configurations of carbon atoms in saturated compounds. Molecules containing an asymmetric carbon atom can be represented in the form of two optical isomers that cannot be superposed in space (i.e., they relate to each other as an object to its mirror image). Such mirror isomers differing only in the opposite arrangement of the same substituents at the chiral center are called enantiomers. Generally, enantiomers have a different biological actitivy and are also characterized by optical activity-the ability to affect plane-polarized light (to rotate the plane of polarization). Enantiomers rotate the plane of polarization at the same angle but in the opposite direction and are therefore called optical antipodes. For compounds having nn chiral centers in the molecule, the number of possible stereoisomers is 2n2n. However, for n≥2n≥2, there exist stereoisomers that are distinguished by part of inherent chirality elements. Stereomers other than enantiomers are called diastereomers.

The term "pharmaceutically acceptable salt" of a given compound refers to salts that retain the biological effectiveness and properties of the compound and are not biologically or otherwise undesirable. Pharmaceutically acceptable base addition salts may be prepared from inorganic or organic bases. Salts derived from inorganic bases include, but are not limited to, sodium, potassium, lithium, ammonium, calcium, and magnesium salts. Salts derived from organic bases include, but are not limited to, salts of primary, secondary, and tertiary amines such as alkyl amines, dialkyl amines, trialkyl amines, substituted alkyl amines, di(substituted alkyl)amines, tri(substituted alkyl)amines, alkenyl amines, dialkenyl mines, trialkenylamines, substituted alkenylamines, and the like. Also included here are amines where two or three substituents, together with the nitrogen atom of the amino group, form a heterocyclic or heteroaryl group.

Pharmaceutically acceptable acid addition salts may be prepared from inorganic acids. In the case in point, salts derived from inorganic acids include hydrochloric acid, hydrobromic acid, sulfuric acid, nitric acid, phosphoric acid, and the like.

The term "pharmaceutically acceptable carrier" includes any and all solvents, dispersion media, coatings, antibacterial and antifungal agents, isotonic and absorption delaying agents, and the like. The use of such media and agents for pharmaceutically active substances is well known in the art. They are used in therapeutic compositions unless

any conventional medium or agent is incompatible with the active ingredient. The composition may also contain additional active ingredients.

The term "active ingredient" (drug substance) refers to a physiologically active substance of a synthetic or other (biotechnological, plant, animal, bactericidal, etc.) origin having pharmacological activity, which is an active ingredient in a pharmaceutical composition.

The term "drug" means a substance (or a mixture of substances in a pharmaceutical composition) in the form of tablets, capsules, injections, ointments, and other finished dosage forms intended to restore, correct or alter physiological functions in humans and animals, as well as for the treatment and prevention of diseases, diagnosis, anesthesia, contraception, cosmetology and so on.

The term "nanosuspension" means, in particular, a solid-liquid system with a particle size of less than one micrometer. In recent years, pure medicinal products have been used as nanoparticles. Water is often used as a dispersion medium wherein the substance is largely insoluble and is administered to the subject in the form of a suspension of nanoparticles. In medicine, nanosuspensions are of great importance for substances that are poorly soluble in water (<5 mg /l) but improve their biopharmaceutical properties (e.g. absorption, bioavailability) in the form of suspension.

The term "pharmaceutical composition" refers to a composition comprising a compound of general formula 1 or 2 and at least one of the components selected from the group consisting of pharmaceutically acceptable and pharmacologically compatible fillers, solvents, diluents, carriers, excipients, distributing and delivery agents such as preservatives, stabilizers, fillers, disintegrators, moisteners, emulsifiers, suspending agents, thickeners, sweeteners, flavoring and antibacterial agents, fungicides, lubricants, and prolonged delivery controllers, the choice and proportions of which depend on the nature and route of administration and dosage. Examples of suitable suspending agents are ethoxylated isostearyl alcohol, polyoxyethylene, sorbitol and sorbitol ether, microcrystalline cellulose, aluminum metahydroxide, bentonite, agar-agar and tragacant, and mixtures thereof. Protection against microorganisms can be provided using various antibacterial and antifungal agents such as parabens, chlorobutanol, sorbic acid, and the like. Said composition may also comprise isotonic agents such as sugar, sodium chloride, and the like. The sustained action of the composition can be achieved using agents that decelerate the absorption of the active ingredient, for example, aluminum monostearate and gelatin. Examples of suitable carriers, solvents, diluents and delivery agents include water, ethanol, polyalcohols and mixtures thereof, natural oils (such as olive oil), and organic esters (such as ethyl oleate) for injections. Examples of fillers are lactose, milk sugar, sodium citrate, calcium carbonate, calcium phosphate, and the like. Examples of disintegrators and distributors are starch, alginic acid and salts thereof, and silicates. Examples of lubricants are magnesium stearate, sodium lauryl sulfate, talc, and polyethylene glycol of high molecular weight. The pharmaceutical composition for oral, sublingual, transdermal, intramuscular, intravenous, subcutaneous, local or rectal administration of the active ingredient, alone or in combination with another active component, can be administered to animals and humans in a standard administration form, as a mixture with traditional pharmaceutical carriers. Suitable unit dosage forms include oral forms such as tablets, gelatin capsules, pills, powders, granules, chewing gums and oral solutions or suspensions; sublingual and buccal administration forms; aerosols; implants; local, transdermal, subcutaneous, intramuscular, intravenous, intranasal, or intraocular administration forms; and rectal administration forms.

The term "prodrug" means a compound that, when administered *in vivo*, is metabolized through one or more steps or processes, or is otherwise converted into a biologically, pharmaceutically, or therapeutically active form of the compound. A prodrug means, for example, a compound that is chemically designed to effectively release the parent drug after overcoming biological barriers to oral delivery. To obtain a prodrug, the pharmaceutically active compound is modified so that the active compound is regenerated as a result of metabolic processes. A prodrug may be intended to alter a drug's metabolic stability or delivery characteristics in order to mask side effects or toxicity, to improve the taste of the drug, or to change other characteristics or properties of the drug. When a pharmaceutically active compound is known, those skilled in the art can develop prodrugs of such compound based on the knowledge of pharmacodynamic processes and drug metabolism in vivo (see, for example, Nogrady's (1985) Medicinal Chemistry: A Biochemical Approach, Oxford University Press, New York, pp. 388-392).

The term "inert filler," as used herein, refers to a compound that is used for producing a pharmaceutical composition and is generally safe, non-toxic and neither biologically nor otherwise undesirable, and includes excipients that are acceptable for veterinary use and pharmacologically acceptable for human use. The compounds of this invention may be administered alone, but will usually be administered in a mixture with one or more pharmaceutically acceptable excipients, diluents, or carriers selected according to the intended route of administration and standard pharmaceutical practice.

The term "therapeutically effective amount" as used herein means the amount of a substance, prodrug, or drug necessary to alleviate the symptoms of a disease in a subject. The dose of a substance, prodrug or drug will meet the individual requirements in each particular case. Said dose can vary widely depending on numerous factors such as the severity of the disease to be treated, the age and general condition of the patient, other drugs the patient is being treated with, the mode and route of administration, and the experience of the attending physician. For oral administration, the daily dose varies from about 0.01 to 10 g, including all values therebetween, in monotherapy and/or in combination

therapy. A preferred daily dose is from about 0.1 to 7 g. Typically, treatment is started with a large initial "loading dose" to quickly alleviate or eliminate the virus and continued with decreasing the dose to a level sufficient to prevent an outbreak of infection.

The term "subject" means a mammal that includes, but is not limited to, cattle, pigs, sheep, chickens, turkeys, buffalos, llamas, ostriches, dogs, cats, and humans; preferably the subject is a human.

**Disclose of the invention**

The subject of the present invention is a new annelated 9-hydroxy-1,8-dioxo-1,3,4,8-tetrahydro-2H-pyrido [1,2-*a*] pyrazine-7-carboxamide of general formula 1 or 2, or any stereoisomer, any pharmaceutically acceptable salt, any solvate, or any crystalline or polycrystalline form thereof:

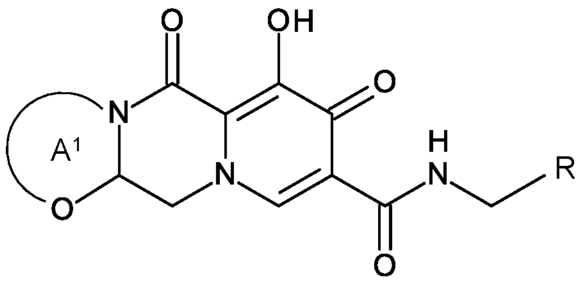

**1**

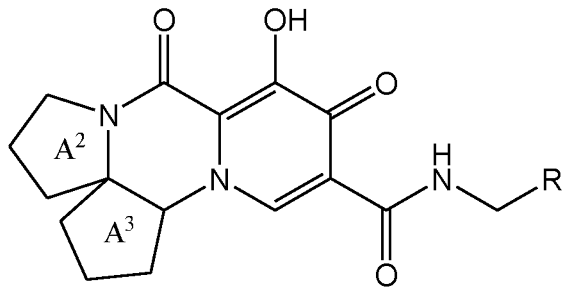

**2**

wherein:

ring $A^1$ is an optionally methyl-substituted 5-7 membered saturated heterocycle or heterobicycle;

ring $A^2$ is a 5-6 membered optionally methyl-substituted saturated or partially saturated monocyclic heterocycle;

ring $A^3$ is a 5-6 membered monocyclic saturated cycloalkane and tetrahydro-2H-pyran; R is a 5-7 membered optionally substituted with one, two or three optionally identical substituents, a monocyclic or bicyclic heterocyclic radical comprising 1-4 heteroatoms selected from the series O, S and N except (2S, 5R, 13aS)-8-hydroxy-7,9-dioxo-N-{[3-(trifluoromethyl)pyridin-2-yl]methyl}-2,3,4,5,7,9,13,13a-octahydro-2,5-methanpyrido[1', 2': 4,5]pyrazino[2,1-*b*] [1,3]oxazepine-10-carboxamide (formula A4) and (1R, 4S, 12aR) -N - [(3,5-difluoropyridin-2-yl) methyl] -7-hydroxy-6,8-dioxo-1,2,3,4,6,8,12,12a -octahydro-1,4-methanodipyrido [1,2-a: 1 ', 2'-d] pyrazine-9-carboxamide (formula A5)

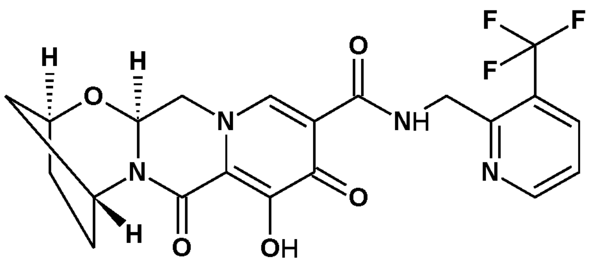

**A4**

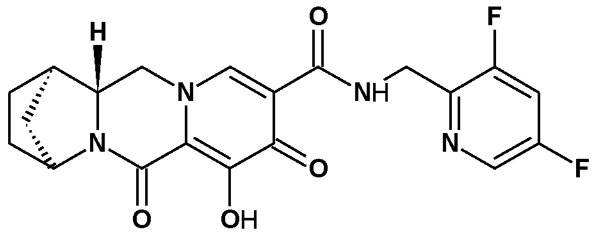

**A5**

.

The optionally substituted monocyclic heteroaryl radical R is selected from the series consisting of thienyl, furyl, pyrazolyl, isoxazolyl, thiazolyl, oxazolyl, imidazolyl, thiadiazolyl, [1,2,5]oxadiazolyl, [1,2,4]oxadiazolyl, [1,2, 4]triazolyl, tetrazolyl, pyridinyl, pyridazinyl, pyrimidinyl, pyrazinyl, 1,2,3-triazinyl, 1,2,4-triazinyl and 1,3,5-triazinyl, imidazo[2,1-b]thiazolyl, imidazo[ 2,1-b] [1,3,4]thiadiazolyl, benzothiophenyl, benzofuranyl, indolyl, 1,3-benzodioxol-5-yl, 2,3-dihydro-1,4-benzodioxin-6-yl, 1,3-benzothiazolyl, 1,3-benzoxazolyl, benzimidazolyl, 1,3-dihydro-2-oxobenzimidazolyl, 2,1,3-benzothiadiazolyl, 2,1,3-benzoxadiazolyl, quinolinyl, isoquinolinyl, imidazo[1,2-a]pyridinyl, 1,2,4-triazolo[4,3-a] pyridinyl, imidazo[1,2-a] pyrimidinyl, imidazo[1,2-a]pyrazinyl, 1,2,4-triazolo[4,3-b]pyridazinyl, 4,5,6,7-tetrahydrobenzothiophenyl,

5,6,7, 8-tetrahydro-[1,2,4]triazolo[4,3-a]pyridinyl, 1,4,5,6,7,8-hexahydrocyclohepta[c]pyrazolyl, 5,6,7,8-tetrahydro-4H-cyclohepta[*d*]thiazolyl, 5,6,7,8-tetrahydro-4H-cyclohepta[d]isothiazol-3-yl, 5,6,7,8-tetrahydro-4H-cyclohepta[d]isothiazol-3-yl, [1,2,4]triazolo[4,3-6]pyridazin-3-yl.

Preferably, the optionally substituted monocyclic heteroaryl radical R is selected from the series consisting of 2-thienyl, 2-furyl, 1H-pyrazol-3-yl, 1H-pyrazol-4-yl, 1H-pyrazol-5-yl, isoxazol-4-yl, thiazol-2-yl, 1,3-oxazol-2-yl, imidazol-2-yl, 1,2,3-thiadiazol-5-yl, 1,2,5-oxadiazol-3-yl, 1,2, 4-oxadiazol-5-yl, 1H-1,2,4-triazol-3-yl, 1H-1,2,3,4-tetrazol-5-yl, 2-pyridyl, 3-pyridyl, 4-pyridyl, pyridazin-4-yl, pyrimidin-4-yl, pyrazin-2-yl, imidazo[2,1-b]thiazol-6-yl, imidazo[2,1-b][1,3,4]thiadiazol-6-yl, benzothiophen-5-yl, benzofuran-2-yl, 1H-indol-5-yl, 1,3-benzodioxol-5-yl, 2,3-dihydro-1,4-benzodioxin-6-yl, 1,3-benzothiazol-2-yl, 1,3-benzoxazol-2-yl, 1H-benzimidazol-2-yl, 1,3-dihydro-2-oxobenzimidazol-5-yl, 2, 1,3-benzothiadiazol-5-yl, 2,1,3-benzoxadiazol-5-yl, quinolin-2-yl, quinolin-3-yl, quinolin-4-yl, quinolin-5-yl, quinolin-6-yl, quinolin-7-yl, quinolin-8-yl, 1-isoquinolinyl, imidazo[1,2-a]pyridin-3-yl, 1,2,4-triazolo[4,3-a]pyridin-3-yl, imidazo[1,2-a]pyrimidin-2-yl, imidazo[1,2-a]pyrazin-3-yl, 1,2,4-triazolo[4,3-b]pyridazin-3-yl, 4,5,6,7-tetrahydrobenzothiophen-2-yl, 5,6,7,8-tetrahydro[1,2,4] triazolo[4,3-a]pyridin-3-yl, 1,4,5 6,7,8-hexahydrocyclohepta[c]pyrazol-3-yl; 5,6,7,8-tetrahydro-4H-cyclohepta[d][1,3]thiazol-2-yl, 5,6,7,8-tetrahydro-4H-cyclohepta[*d*]isothiazol-3-yl, 5,6,7,8-tetrahydro-4H-cyclohepta[*d*]isothiazol-3-yl, [1,2,4]triazolo[4,3-*b*]pyridazin-3-yl.

Preferred substituents of the heteroaryl radical R are one, two, or three independent substituents selected from lower C1-C3 alkyls and halogen atoms, preferably F, Cl, and Br.

The subject of this invention is a compound of general formula 1.1, 1.2, or 1.3, wherein R is as defined above, ring $A^1$ is 4-methyl-1,3-oxazolidine, 4-methyl-1,3-oxazinane, and (1*R*,5*S*)-2-oxa-4-azabicyclo [3.2.1] octane, respectively, and a pharmaceutically acceptable salt or solvate thereof

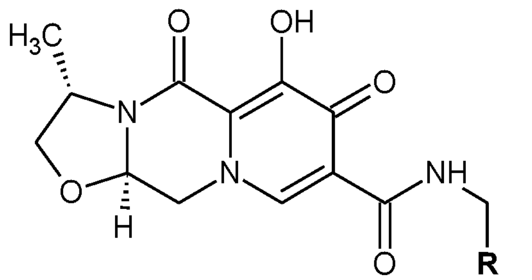

**1.1**

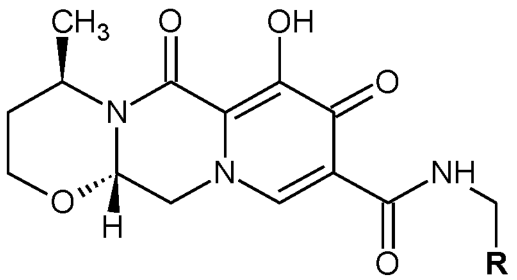

**1.2**

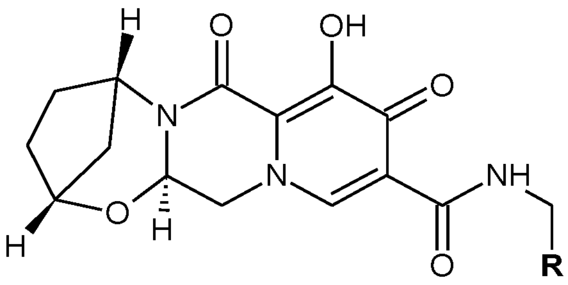
,

**1.3**

wherein R is as given above.

The subject of this invention is annelated 9-hydroxy-1,8-dioxo-N-(pyridinylmethyl)-1,3,4,8-tetrahydro-2H-pyrido[1,2-*a*] pyrazine-7-carboxamide of general formula 2.1 or 2.2, wherein R is as given above, or their stereoisomer, their pharmaceutically acceptable salt, their solvate, their crystalline or polycrystalline form, or their nanocrystalline form

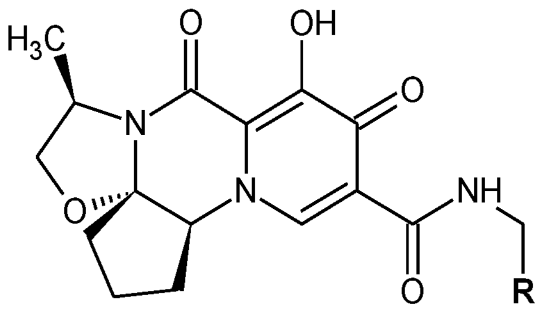
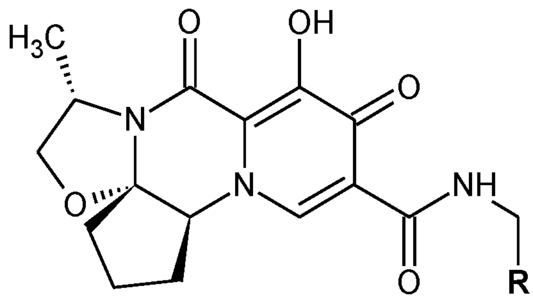

2.1 2.2 ,

wherein R is as given above.

The subject of this invention is annelated 9-hydroxy-1,8-dioxo-1,3,4,8-tetrahydro-2H-pyrido[1,2-*a*]pyrazine-7-carboxamide of general formula 1 or general formula 2 selected from the series including:

| |
|---|
| (3*S*,11*aR*)-6-hydroxy-3-methyl-5,7-dioxo-*N*-(2-thienylmethyl)-2,3,5,7,11,11*a*-hexahydro[1,3]oxazolo[3,2-*a*]pyrido[1,2-*d*]pyrazine-8-carboxamide (1.1.1), |
| (3*S*,11*aR*)-*N*-[(5-bromo-2-thienyl)methyl]-6-hydroxy-3-methyl-5,7-dioxo-2,3,5,7,11,11*a*-hexahydro[1,3]oxazolo[3,2-*a*]pyrido[1,2-*d*]pyrazine-8-carboxamide (1.1.2), |
| (3*S*,11*aR*)-6-hydroxy-3-methyl-*N*-[(5-methyl-2-furyl)methyl]-5,7-dioxo-2,3,5,7,11,11*a*-hexahydro[1,3]oxazolo[3,2-*a*]pyrido[1,2-*d*]pyrazine-8-carboxamide (1.1.3), |
| (3*S*,11*aR*)-6-hydroxy-*N*-[(3,5-dimethyl-1H-pyrazol-4-yl)methyl]-3-methyl-5,7-dioxo-2,3,5,7,11,11*a*-hexahydro[1,3]oxazolo[3,2-*a*]pyrido[1,2-*d*]pyrazine-8-carboxamide (1.1.4), |
| (3*S*,11*aR*)-6-hydroxy-3-methyl-*N*-[(1,3,5-trimethyl-1H-pyrazol-4-yl)methyl]-5,7-dioxo-2,3,5,7,11,11*a*-hexahydro[1,3]oxazolo[3,2-*a*]pyrido[1,2-*d*]pyrazine-8-carboxamide (1.1.5), |
| (3*S*,11*aR*)-6-hydroxy-*N*-[(1-ethyl-3,5-dimethyl-1H-pyrazol-4-yl)methyl]3-methyl-5,7-dioxo-2,3,5,7,11,11*a*-hexahydro[1,3]oxazolo[3,2-*a*]pyrido[1,2-*d*]pyrazine-8-carboxamide (1.1.6), |
| (3*S*,11*aR*)-6-hydroxy-*N*-[(4,5-dichloro-1-methyl-1H-pyrazol-3-yl)methyl]3-methyl-5,7-dioxo-2,3,5,7,11,11*a*-hexahydro[1,3]oxazolo[3,2-*a*]pyrido[1,2-*d*]pyrazine-8-carboxamide (1.1.7), |
| (3*S*,11*aR*)-6-hydroxy-3-methyl-*N*-(1,3-thiazol-2-ylmethyl)-5,7-dioxo-2,3,5,7,11,11*a*-hexahydro[1,3]oxazolo[3,2-*a*]pyrido[1,2-*d*]pyrazine-8-carboxamide (1.1.8), |
| (3*S*,11*aR*)-6-hydroxy-3-methyl-*N*-[(4-methyl-1,3-oxazol-2-yl)methyl]-5,7-dioxo-2,3,5,7,11,11*a*-hexahydro[1,3]oxazolo[3,2-*a*]pyrido[1,2-*d*]pyrazine-8-carboxamide (1.1.9), |
| (3*S*,11*aR*)-6-hydroxy-3-methyl-*N*-[(1,4,5-trimethyl-1H-imidazol-2-yl)methyl]-5,7-dioxo-2,3,5,7,11, 11*a*-hexahydro[1,3]oxazolo[3,2-*a*]pyrido[1,2-*d*]pyrazine-8-carboxamide (1.1.10), |
| (3*S*,11*aR*)-6-hydroxy-3-methyl-*N*-[(4-methyl-1,2,3-thiadiazol-5-yl)methyl]-5,7-dioxo-2,3,5,7,11,11*a*-hexahydro[1,3]oxazolo[3,2-*a*]pyrido[1,2-*d*]pyrazine-8-carboxamide (1.1.11), |
| (3*S*,11*aR*)-6-hydroxy-3-methyl-*N*-[(4-methyl-1,2,5-oxadiazol-3-yl)methyl]-5,7-dioxo-2,3,5,7,11,11*a*-hexahydro[1,3]oxazolo[3,2-*a*]pyrido[1,2-*d*]pyrazine-8-carboxamide (1.1.12), |
| (3*S*,11*aR*)-6-hydroxy-3-methyl-*N*-[(3-methyl-1,2,4-oxadiazol-5-yl)methyl]-5,7-dioxo-2,3,5,7,11,11*a*-hexahydro[1,3]oxazolo[3,2-*a*]pyrido[1,2-*d*]pyrazine-8-carboxamide (1.1.13), |
| (3*S*,11*aR*)-6-hydroxy-3-methyl-*N*-[(4-methyl-4H-1,2,4-triazol-3-yl)methyl]-5,7-dioxo-2,3,5,7,11,11*a*-hexahydro[1,3]oxazolo[3,2-*a*]pyrido[1,2-*d*]pyrazine-8-carboxamide (1.1.14), |
| (3*S*,11*aR*)-6-hydroxy-3-methyl-*N*-(pyridyl-2-ylmethyl)-5,7-dioxo-2,3,5,7,11,11a-hexahydro[1,3]oxazolo[3,2-*a*]pyrido[1,2-*d*]pyrazine-8-carboxamide (1.1.15), |
| (3*S*,11*aR*)-6-hydroxy-3-methyl-*N*-[(3-fluoropyridin-2-yl)methyl]-5,7-dioxo-2,3,5,7,11,11*a*-hexahydro[1,3]oxazolo[3,2-*a*]pyrido[1,2-*d*]pyrazine-8-carboxamide (1.1.16), |

(continued)

| |
|---|
| (3*S*,11*aR*)-6-hydroxy-*N*-[(3,5-difluoropyridin-2-yl)methyl]-3-methyl-5,7-dioxo-2,3,5,7,11,11*a*-hexahydro[1,3]oxazolo [3,2-*a*]pyrido[1,2-*d*]pyrazine-8-carboxamide (1.1.17), |
| (3*S*,11*aR*)-6-hydroxy-*N*-[(6-chloropyridin-3-yl)methyl]-3-methyl-5,7-dioxo-2,3,5,7,11,11*a*-hexahydro[1,3]oxazolo [3,2-*a*]pyrido[1,2-*d*]pyrazine-8-carboxamide (1.1.18), |
| (3*S*,11*aR*)-6-hydroxy-*N*-[(2,6-difluoropyridin-3-yl)methyl]-3-methyl-5,7-dioxo-2,3,5,7,11,11*a*-hexahydro[1,3]oxazolo [3,2-*a*]pyrido[1,2-*d*]pyrazine-8-carboxamide (1.1.19), |
| Sodium (3S, 11aR)-8-({[(2,6-difluoropyridin-3-yl)methyl]amino}carbonyl)-3-methyl-5,7-dioxo-2,3,5,7,11, 11a-hexahydro[1,3]oxazolo[3,2-a]pyrido[1,2-d]pyrazin-6-olate (1.1.20), |
| (3*S*,11*aR*)-6-hydroxy-N-[(2,4-difluoropyridin-3-yl)methyl]-3-methyl-5,7-dioxo-2,3,5,7,11,11*a*-hexahydro[1,3]oxazolo [3,2-*a*]pyrido[1,2-*d*]pyrazine-8-carboxamide (1.1.21), |
| (3*S*,11*aR*)-6-hydroxy-3-methyl-*N*-[(2,4,6-trifluoropyridin-3-yl)methyl]-5,7-dioxo-2,3,5,7,11,11*a*-hexahydro[1,3] oxazolo[3,2-*a*]pyrido[1,2-*d*]pyrazine-8-carboxamide (1.1.22), |
| (3*S*,11*aR*)-6-hydroxy-3-methyl-*N*-[(3-fluoropyridin-4-yl)methyl]-5,7-dioxo-2,3,5,7,11,11*a*-hexahydro[1,3]oxazolo [3,2-*a*]pyrido[1,2-*d*]pyrazine-8-carboxamide (1.1.23), |
| (3*S*,11*aR*)-6-hydroxy-*N*-(3,5-difluoropyridin-4-yl)methyl]-3-methyl-5,7-dioxo-2,3,5,7,11,11*a*-hexahydro[1,3]oxazolo [3,2-*a*]pyrido[1,2-*d*]pyrazine-8-carboxamide (1.1.24), |
| (3*S*,11*aR*)-6-hydroxy-3-methyl-*N*-(pyridazin-4-ylmethyl)-5,7-dioxo-2,3,5,7,11,11*a*-hexahydro[1,3]oxazolo[3,2-*a*] pyrido[1,2-*d*]pyrazine-8-carboxamide (1.1.25), |
| (3*S*,11*aR*)-6-hydroxy-3-methyl-*N*-[(2-methylpyrimidin-4-yl)methyl]-5,7-dioxo-2,3,5,7,11,11*a*-hexahydro[1,3]oxazolo [3,2-*a*]pyrido[1,2-*d*]pyrazine-8-carboxamide (1.1.26), |
| (3*S*,11*aR*)-6-hydroxy-3-methyl-*N*-(pyrazin-2-ylmethyl)-5,7-dioxo-2,3,5,7,11,11*a*-hexahydro[1,3]oxazolo[3,2-*a*] pyrido[1,2-*d*]pyrazine-8-carboxamide (1.1.27), |
| (3*S*,11*aR*)-6-hydroxy-3-methyl-*N*-[(2-chloroimidazo[2,1-b][1,3]thiazol-6-yl)methyl]-5,7-dioxo-2,3,5,7,11,11*a*-hexahydro[1,3]oxazolo[3,2-*a*] pyrido[1,2-*d*]pyrazine-8-carboxamide (1.1.28), |
| (3*S*,11*aR*)-6-hydroxy-*N*-(imidazo[2,1-b][1,3,4]thiadiazol-6-ylmethyl)-3-methyl-5,7-dioxo-2,3,5,7,11,11*a*-hexahydro[1,3]oxazolo[3,2-*a*]pyrido [1,2-*d*]pyrazine-8-carboxamide (1.1.29), |
| (3*S*,11*aR*)-6-hydroxy-3-methyl-*N*-[(2-methylimidazo[2,1-b][1,3,4] thiadiazol-6-yl)methyl]-5,7-dioxo-2,3,5,7,11,11*a*-hexahydro[1,3] oxazolo[3,2-*a*]pyrido[1,2-*d*]pyrazine-8-carboxamide (1.1.30), |
| (3*S*,11*aR*)-*N*-(1-benzothien-5-ylmethyl)-6-hydroxy-3-methyl-5,7-dioxo-2,3,5,7,11,11*a*-hexahydro[1,3]oxazolo[3,2-*a*]pyrido[1,2-*d*]pyrazine-8-carboxamide (1.1.31), |
| (3*S*,11*aR*)-*N*-(1-benzofuran-2-ylmethyl)-6-hydroxy-3-methyl-5,7-dioxo-2,3,5,7,11,11*a*-hexahydro[1,3]oxazolo[3,2-*a*]pyrido[1,2-*d*]pyrazine-8-carboxamide (1.1.32), |
| (3*S*,11*aR*)-6-hydroxy-3-methyl-*N*-[(1-methyl-1H-indol-5-yl)methyl]-5,7-dioxo-2,3,5,7,11,11*a*-hexahydro[1,3]oxazolo [3,2-*a*]pyrido[1,2-*d*]pyrazine-8-carboxamide (1.1.33), |
| (3*S*,11*aR*)-*N*-(1,3-benzodioxol-5-ylmethyl)-6-hydroxy-3-methyl-5,7-dioxo-2,3,5,7,11,11*a*-hexahydro[1,3]oxazolo [3,2-*a*]pyrido[1,2-*d*]pyrazine-8-carboxamide (1.1.34), |
| (3*S*,11*aR*)-*N*-[(6-bromo-1,3-benzodioxol-5-yl)methyl]-6-hydroxy-3-methyl-5, 7-dioxo-2,3,5,7,11,11*a*-hexahydro[1,3]oxazolo[3,2-*a*]pyrido [1,2-*d*]pyrazine-8-carboxamide (1.1.35), |
| (3*S*,11*aR*)-6-hydroxy-3-methyl-*N*-[(7-methyl-2,3-dihydro-1,4-benzodioxin-6-yl)methyl]-5,7-dioxo-2,3,5,7,11,11*a*-hexahydro[1,3] oxazolo[3,2-*a*]pyrido[1,2-*d*]pyrazine-8-carboxamide (1.1.36), |

(continued)

| | |
|---|---|
| | (3*S*,11*aR*)-*N*-(1,3-benzothiazol-2-ylmethyl)-6-hydroxy-3-methyl-5,7-dioxo-2,3,5,7,11,11*a*-hexahydro[1,3]oxazolo[3,2-*a*]pyrido[1,2-*d*]pyrazine-8-carboxamide (1.1.37), |
| | (3*S*,11*aR*)-6-hydroxy-3-methyl-*N*-[(5-chloro-1,3-benzoxazol-2-yl)methyl]-5,7-dioxo-2,3,5,7,11,11*a*-hexahydro[1,3]oxazolo[3,2-*a*]pyrido[1,2-*d*]pyrazine-8-carboxamide (1.1.38), |
| | (3*S*,11*aR*)-6-hydroxy-3-methyl-*N*-[(6-chloro-1,3-benzoxazol-2-yl)methyl]-5,7-dioxo-2,3,5,7,11,11*a*-hexahydro[1,3]oxazolo[3,2-*a*]pyrido[1,2-*d*]pyrazine-8-carboxamide (1.1.39), |
| (3*S*,11*aR*)-6-hydroxy-3-methyl-*N*-[(5-methyl-1H-benzimidazol-2-yl)methyl]-5,7-dioxo-2,3,5,7,11,11*a*-hexahydro[1,3]oxazolo[3,2-*a*]pyrido[1,2-*d*]pyrazine-8-carboxamide (1.1.40), | |
| (3*S*,11*aR*)-6-hydroxy-3-methyl-*N*-[(5-фторо-1H-benzimidazol-2-yl)methyl]-5,7-dioxo-2,3,5,7,11,11*a*-hexahydro[1,3]oxazolo[3,2-*a*]pyrido[1,2-*d*]pyrazine-8-carboxamide (1.1.41), | |
| (3*S*,11*aR*)-6-hydroxy-3-methyl-*N*-[(1-methyl-benzimidazol-2-yl)methyl]-5,7-dioxo-2,3,5,7,11,11*a*-hexahydro[1,3]oxazolo[3,2-*a*]pyrido[1,2-*d*]pyrazine-8-carboxamide (1.1.42), | |
| (3*S*,11*aR*)-6-hydroxy-3-methyl-*N*-[(1-methyl-6-chlorobenzimidazol-2-yl)methyl]-5,7-dioxo-2,3,5,7,11,11*a*-hexahydro[1,3]oxazolo[3,2-*a*]pyrido[1,2-*d*]pyrazine-8-carboxamide (1.1.43), | |
| (3S,11aR)-6-hydroxy-3-methyl-N-{[1-isopropyl-1H-benzimidazol-2-yl]methyl}-5,7-dioxo-2,3,5,7,11,11a-hexahydro[1,3]oxazolo[3,2-*a*]pyrido[1,2-*d*]pyrazine-8-carboxamide_(1.1.44), | |
| | (3*S*,11*aR*)-6-hydroxy-3-methyl-*N*-[(6-chloro-1-isopropyl-1H-benzimidazol-2-yl)methyl]-5,7-dioxo-2,3,5,7,11,11*a*-hexahydro[1,3]oxazolo[3,2-*a*]pyrido[1,2-*d*]pyrazine-8-carboxamide (1.1.45), |
| | (3*S*,11*aR*)-6-hydroxy-*N*-[(2-oxo-2,3-dihydro-1H--benzimidazol-5-yl)methyl]-3-methyl-5,7-dioxo-2,3,5,7,11,11*a*-hexahydro[1,3]oxazolo[3,2-*a*]pyrido[1,2-*d*]pyrazine-8-carboxamide (1.1.46), |
| | (3*S*,11*aR*)-*N*-(2,1,3-benzothiadiazol-5-ylmethyl)-6-hydroxy-3-methyl-5,7-dioxo-2,3,5,7,11,11*a*-hexahydro[1,3]oxazolo[3,2-*a*]pyrido[1,2-*d*]pyrazine-8-carboxamide (1.1.47), |
| | (3*S*,11*aR*)-*N*-(2,1,3-benzoxadiazol-5-ylmethyl)-6-hydroxy-3-methyl-5,7-dioxo-2,3,5,7,11,11*a*-hexahydro[1,3]oxazolo[3,2-*a*]pyrido[1,2-*d*]pyrazine-8-carboxamide (1.1.48), |
| | (3*S*,11*aR*)-6-hydroxy-3-methyl-*N*-(quinolin-2-ylmethyl)-5,7-dioxo-2,3,5,7,11,11*a*-hexahydro[1,3]oxazolo[3,2-*a*]pyrido[1,2-*d*]pyrazine-8-carboxamide (1.1.49), |
| | (3*S*,11*aR*)-6-hydroxy-3-methyl-*N*-(quinolin-3-ylmethyl)-5,7-dioxo-2,3,5,7,11,11*a*-hexahydro[1,3]oxazolo[3,2-*a*]pyrido[1,2-*d*]pyrazine-8-carboxamide (1.1.50), |
| | (3*S*,11*aR*)-6-hydroxy-3-methyl-*N*-(quinolin-4-ylmethyl)-5,7-dioxo-2,3,5,7,11,11*a*-hexahydro[1,3]oxazolo[3,2-*a*]pyrido[1,2-*d*]pyrazine-8-carboxamide (1.1.51), |
| | (3*S*,11*aR*)-6-hydroxy-3-methyl-*N*-(quinolin-5-ylmethyl)-5,7-dioxo-2,3,5,7,11,11*a*-hexahydro[1,3]oxazolo[3,2-*a*]pyrido[1,2-*d*]pyrazine-8-carboxamide (1.1.52), |
| | (3*S*,11*aR*)-6-hydroxy-3-methyl-*N*-(quinolin-6-ylmethyl)-5,7-dioxo-2,3,5,7,11,11*a*-hexahydro[1,3]oxazolo[3,2-*a*]pyrido[1,2-*d*]pyrazine-8-carboxamide (1.1.53), |

(continued)

| | |
|---|---|
| | (3*S*,11*aR*)-6-hydroxy-3-methyl-*N*-(quinolin-8-ylmethyl)-5,7-dioxo-2,3,5,7,11,11*a*-hexahydro[1,3]oxazolo[3,2-*a*]pyrido[1,2-*d*]pyrazine-8-carboxamide (1.1.54), |
| | (3*S*,11*aR*)-6-hydroxy-*N*-(isoquinolin-1-ylmethyl)-3-methyl-5,7-dioxo-2,3,5,7,11,11*a*-hexahydro[1,3]oxazolo[3,2-*a*]pyrido[1,2-*d*]pyrazine-8-carboxamide (1.1.55), |
| | (3*S*,11*aR*)-6-hydroxy-*N*-(imidazo[1,2-a]pyridin-3-ylmethyl)-3-methyl-5, 7 -dioxo-2,3,5,7,11,11*a*-hexahydro[1,3]oxazolo[3,2-*a*]pyrido[1,2-*d*] pyrazine-8-carboxamide (1.1.56), |
| | (3*S*,11*aR*)-6-hydroxy-3-methyl-*N*-(1,2,4-triazolo[4,3-a]pyridin-3-ylmethyl)-5,7-dioxo-2,3,5,7,11,11*a*-hexahydro[1,3]oxazolo[3,2-*a*]pyrido [1,2-*d*]pyrazine-8-carboxamide (1.1.57), |
| | (3*S*,11*aR*)-6-hydroxy-*N*-(imidazo[1,2-a]pyrimidin-2-ylmethyl)-3-methyl-5, 7 -dioxo-2,3,5,7,11,11*a*-hexahydro[1,3]oxazolo[3,2-*a*]pyrido[1,2-*d*] pyrazine-8-carboxamide (1.1.58), |
| | (3*S*,11*aR*)-6-hydroxy-*N*-(imidazo[1,2-a]pyrazin-3-ylmethyl)-3-methyl-5, 7 -dioxo-2,3,5,7,11,11*a*-hexahydro[1,3]oxazolo[3,2-*a*]pyrido[1,2-*d*] pyrazine-8-carboxamide (1.1.59), |
| | (3*S*,11*aR*)-6-hydroxy-3-methyl-*N*-[(6-chloro[1,2,4]triazolo[4,3-b] pyridazin-3-yl)methyl]-5, 7-dioxo-2,3,5,7,11,11*a*-hexahydro[1,3] oxazolo[3,2-*a*]pyrido[1,2-*d*]pyrazine-8-carboxamide (1.1.60), |
| | (3*S*,11*aR*)-6-hydroxy-3-methyl-*N*-(5,6,7,8-tetrahydro[1,2,4]triazolo[4,3-a]pyridin-3-ylmethyl)-5,7-dioxo-2,3,5,7,11,11*a*-hexahydro[1,3]oxazolo [3,2-*a*]pyrido[1,2-*d*]pyrazine-8-carboxamide (1.1.61), |
| | (3*S*,11*aR*)-6-hydroxy-*N*-(1,4,5,6,7,8-hexahydrocyclohepta[c]pyrazol-3-ylmethyl)-3-methyl-5,7-dioxo-2,3,5,7,11,11*a*-hexahydro[1,3]oxazolo [3,2-*a*]pyrido[1,2-*d*]pyrazine-8-carboxamide (1.1.62), |
| | (3*S*,11*aR*)-6-hydroxy-3-methyl-5,7-dioxo-*N*-(5,6,7,8-tetrahydro-4*H*-cyclohepta[*d*][1,3]thiazol-2-ylmethyl)-2,3,5,7,11,11*a*-hexahydro[1,3] oxazolo[3,2-*a*]pyrido[1,2-*d*]pyrazine-8-carboxamide (1.1.63), |
| | (3*S*,11*aR*)-6-hydroxy-3-methyl-*N*-[(1,2-dimethyl-1H-benzimidazol-5-yl) methyl]-5,7-dioxo-2,3,5,7,11,11*a*-hexahydro[1,3]oxazolo[3,2-*a*]pyrido [1,2-*d*]pyrazine-8-carboxamide (1.1.64), |
| | (3*S*,11*aR*)-6-hydroxy-3-methyl-*N*-(quinolin-4-ylmethyl)-5,7-dioxo-2,3,5,7,11,11*a*-hexahydro[1,3]oxazolo[3,2-*a*]pyrido[1,2-*d*]pyrazine-8-carboxamide (1.1.65), |
| | (4*R*,12a*S*)-7-hydroxy-*N*-[(2,6-difluoropyridin-3-yl)methyl]-4-methyl-6,8-dioxo-3,4,6,8,12,12a-hexahydro-2*H*-pyrido[1',2':4,5]pyrazino[2,1-*b*] [1,3]oxazine-9-carboxamide (1.2.1), |
| | (4*R*,12a*S*)-7-hydroxy-*N*-[(2,4-difluoropyridin-3-yl)methyl]-4-methyl-6,8-dioxo-3,4,6,8,12,12a-hexahydro-2*H*-pyrido[1',2':4,5]pyrazino[2,1-*b*] [1,3]oxazine-9-carboxamide (1.2.2), |
| | (4*R*,12a*S*)-7-hydroxy-4-methyl-*N*-[(2,4.6-trifluoropyridin-3-yl)methyl]-6,8-dioxo-3,4,6,8,12,12a-hexahydro-2*H*-pyrido[1',2':4,5]pyrazino[2,1-*b*][1,3]oxazine-9-carboxamide (1.2.3), |
| | (4*R*,12a*S*)-7-hydroxy-*N*-[(3,5-difluoropyridin-4-yl)methyl]-4-methyl-6,8-dioxo-3,4,6,8,12,12a-hexahydro-2*H*-pyrido[1',2':4,5]pyrazino[2,1-*b*] [1,3]oxazine-9-carboxamide (1.2.4), |

(continued)

| | |
|---|---|
| | (2*R*,5*S*,13a*R*)-8-hydroxy-*N*-[(2,6-difluoropyridin-3-yl)methyl]-7,9-dioxo-2,3,4,5,7,9,13,13a-octahydro-2,5-methanopyrido[1',2':4,5]pyrazino[2,1-*b*][1,3]oxazepine-10-carboxamide (1.3.1), |
| | (2*R*,5*S*,13a*R*)-8-hydroxy-*N*-[(2,4,6-trifluoropyridin-3-yl)methyl]-7,9-dioxo-2,3,4,5,7,9,13,13a-octahydro-2,5-methanopyrido[1',2':4,5]pyrazino[2,1-*b*][1,3]oxazepine-10-carboxamide (1.3.2), |
| | (2*R*,5*S*,13a*R*)-8-hydroxy-N-[(3,5-difluoropyridin-4-yl)methyl]-7,9-dioxo-2,3,4,5,7,9,13,13a-octahydro-2,5-methanopyrido[1',2':4,5]pyrazino[2,1-*b*][1,3]oxazepine-10-carboxamide (1.3.3), |
| | (3a*S*,6*R*,13a*S*)-9-hydroxy-*N*-[(2,6-difluoropyridin-3-yl)methyl]-6-methyl-8,10-dioxo-1,2,3,5,6,8,10,13a-octahydrocyclopenta[*b*][1,3]oxazolo[3,2-*a*]pyrido[1,2-*d*]pyrazine-11-carboxamide (2.1.1), |
| | (3a*S*,6*R*,13a*S*)-9-hydroxy-*N*-[(3,5-difluoropyridin-4-yl)methyl]-6-methyl-8,10-dioxo-1,2,3,5,6,8,10,13a-octahydrocyclopenta[*b*][1,3]oxazolo[3,2-*a*]pyrido[1,2-*d*]pyrazine-11-carboxamide (2.1.2) |
| | (3a*S*,6*R*,13a*S*)-9-hydroxy-6-methyl-*N*-[(2,4,6-trifluoropyridin-3-yl)methyl]-8,10-dioxo-1,2,3,5,6,8,10,13a-octahydrocyclopenta[*b*][1,3]oxazolo[3,2-*a*]pyrido[1,2-*d*]pyrazine-11-carboxamide (2.1.3), |
| | (3a*S*,6*S*,13a*S*)-9-hydroxy-*N*-[(2,6-difluoropyridin-3-yl)methyl]-6-methyl-8,10-dioxo-1,2,3,5,6,8,10,13a-octahydrocyclopenta[*b*][1,3]oxazolo[3,2-*a*]pyrido[1,2-*d*]pyrazine-11-carboxamide (2.2.1), |
| | (3a*S*,6*S*,13a*S*)-9-hydroxy-*N*-[(3,5-difluoropyridin-4-yl)methyl]-6-methyl-8,10-dioxo-1,2,3,5,6,8,10,13a-octahydrocyclopenta[*b*][1,3]oxazolo[3,2-*a*]pyrido[1,2-*d*]pyrazine-11-carboxamide (2.2.2), |
| (3a*S*,6*S*,13a*S*)-9-hydroxy-6-methyl-*N*-[(2,4,6-trifuoropyridin-3-yl)methyl]-8,10-dioxo-1,2,3,5,6,8,10,13a-octahydrocyclopenta[*b*][1,3]oxazolo[3,2-*a*]pyrido[1,2-*d*]pyrazine-11-carboxamide (2.2.3), their stereoisomers, their pharmaceutically acceptable salts, and their solvates. | |

| | | | |
|---|---|---|---|
| 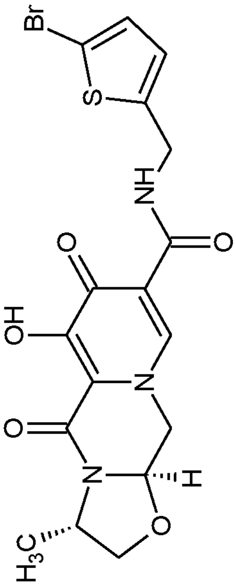 1.1.2 | 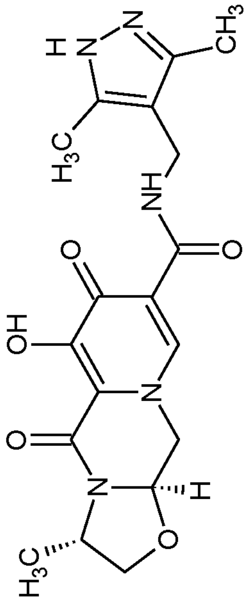 1.1.4 | 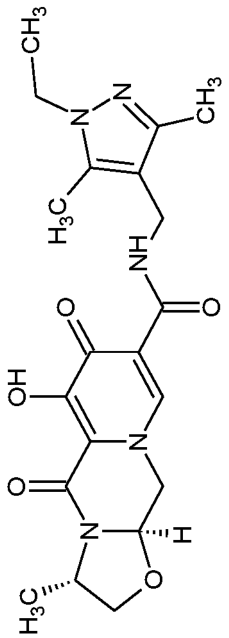 1.1.6 | 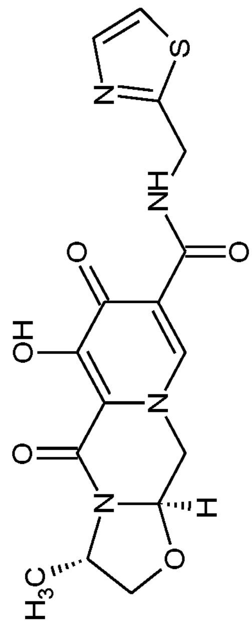 1.1.8 |
| 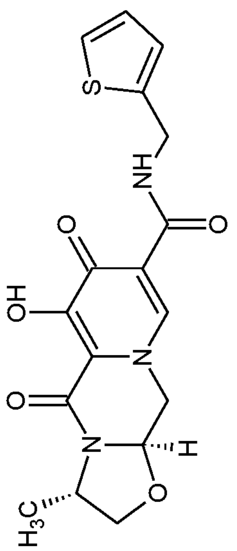 1.1.1 | 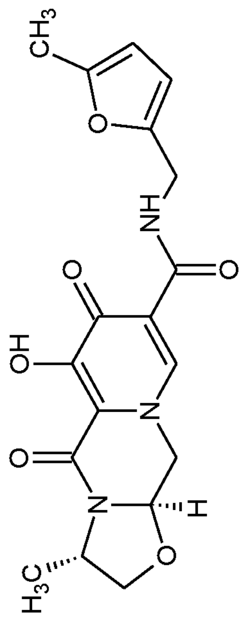 1.1.3 | 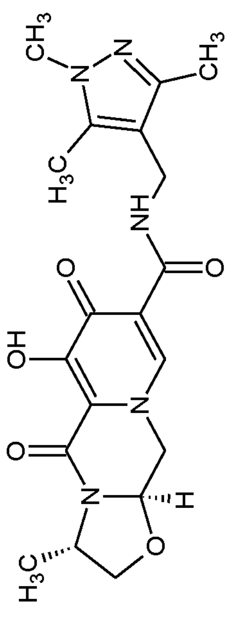 1.1.5 | 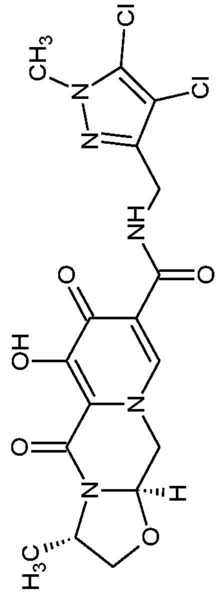 1.1.7 |

(continued)

| | |
|---|---|
| 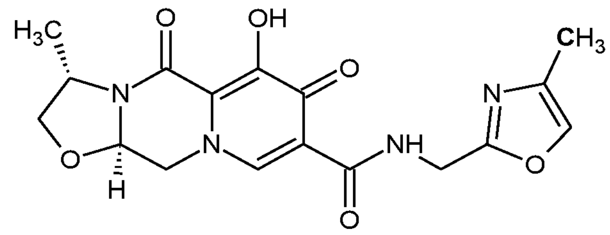 1.1.9 | 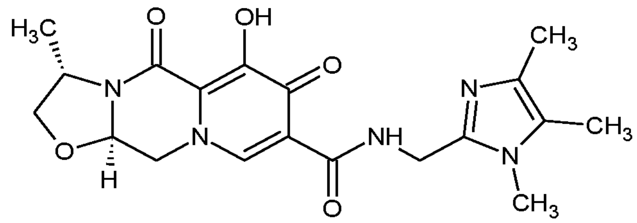 1.1.10 |
| 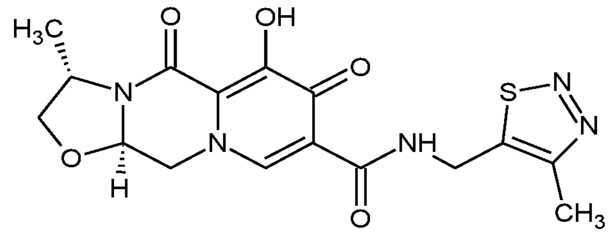 1.1.11 | 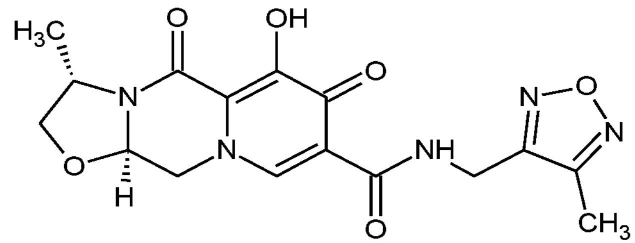 1.1.12 |
| 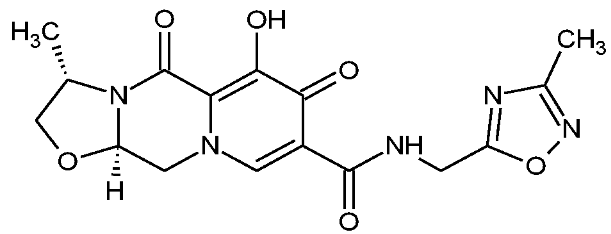 1.1.13 | 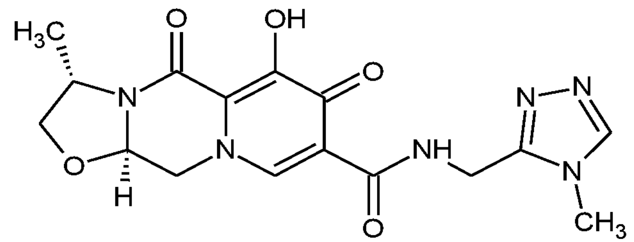 1.1.14 |
| 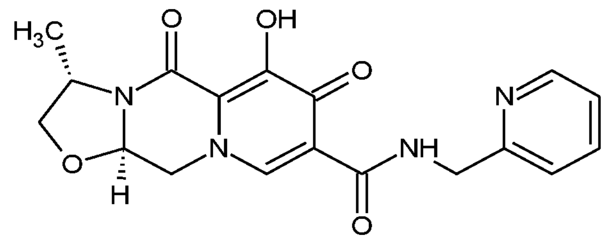 1.1.15 | 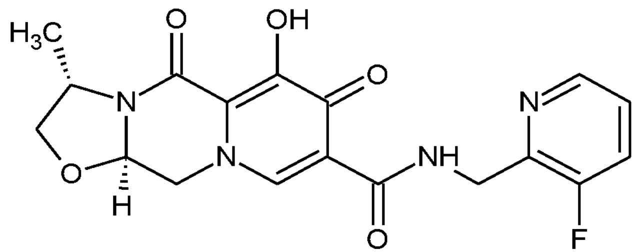 1.1.16 |

(continued)

| | |
|---|---|
| 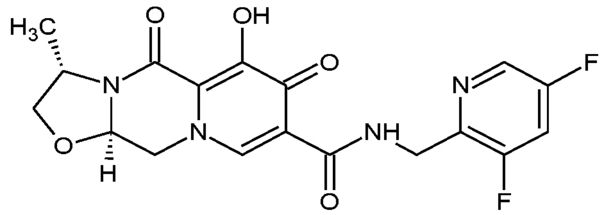 | 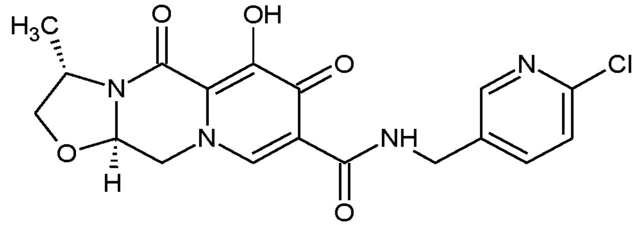 |
| **1.1.17** | **1.1.18** |
| 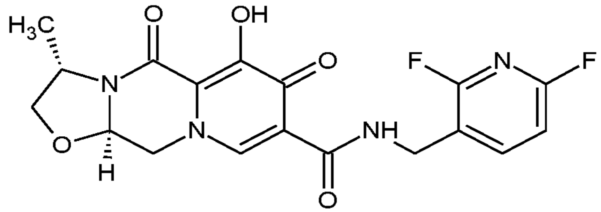 | 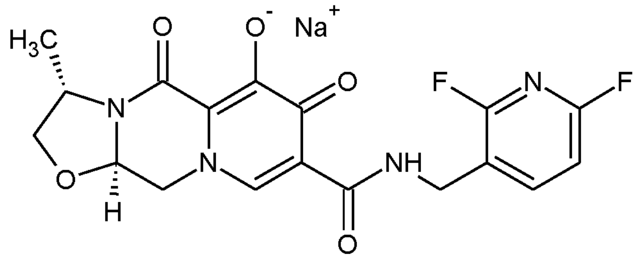 |
| **1.1.19** | **1.1.20** |
| 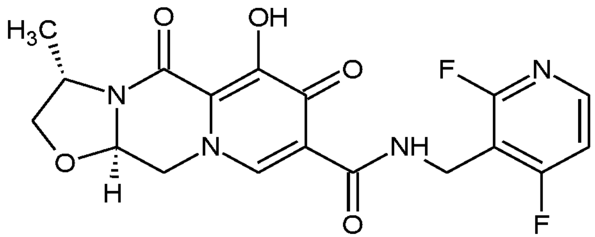 | 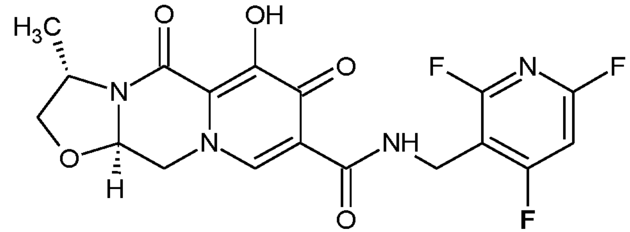 |
| **1.1.21** | **1.1.22** |
| 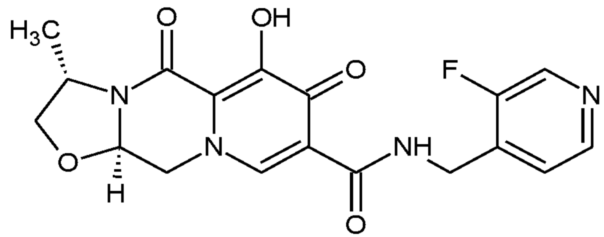 | 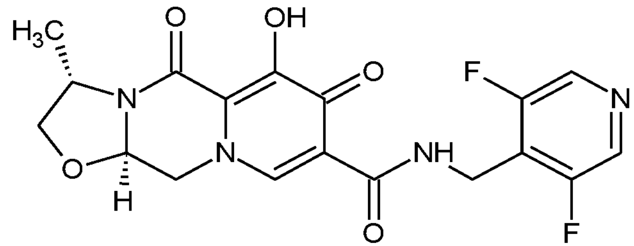 |

(continued)

| 1.1.23 | 1.1.24 |
|---|---|
| 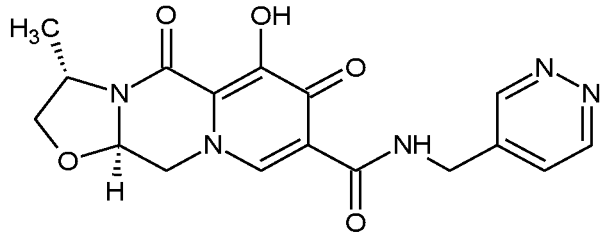<br>1.1.25 | 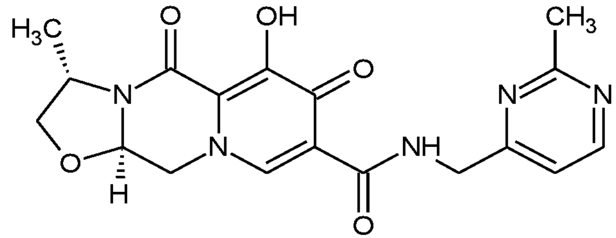<br>1.1.26 |
| 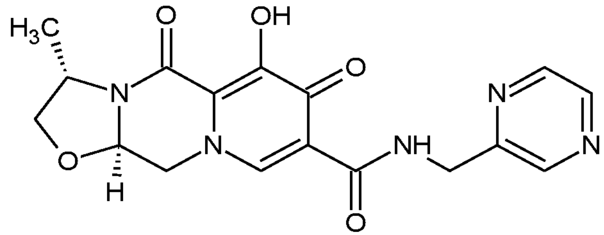<br>1.1.27 | 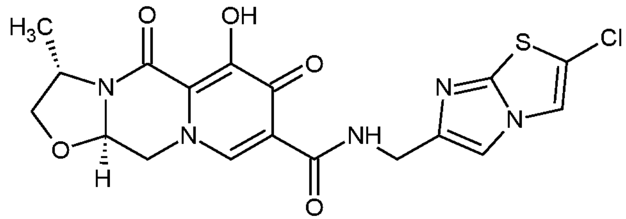<br>1.1.28 |
| 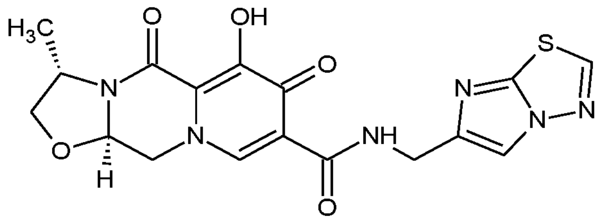<br>1.1.29 | 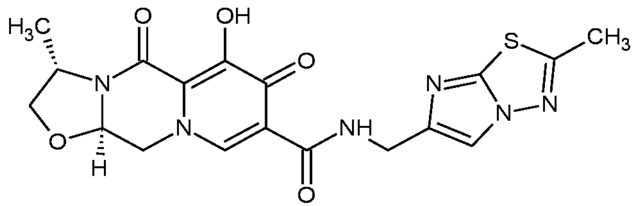<br>1.1.30 |
| 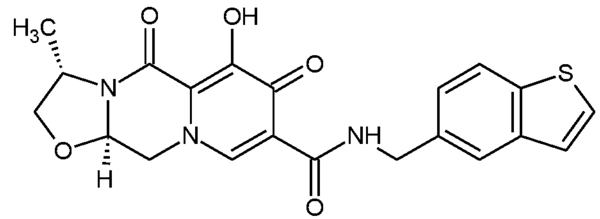<br>1.1.31 | 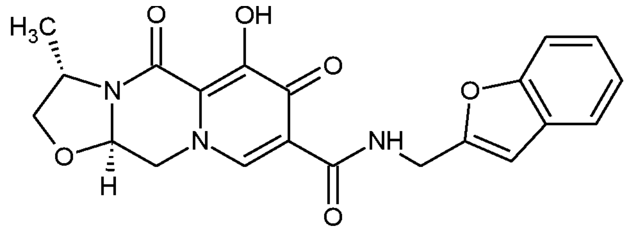<br>1.1.32 |

(continued)

| | |
|---|---|
| 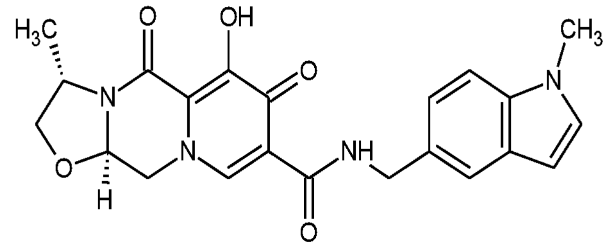 1.1.33 | 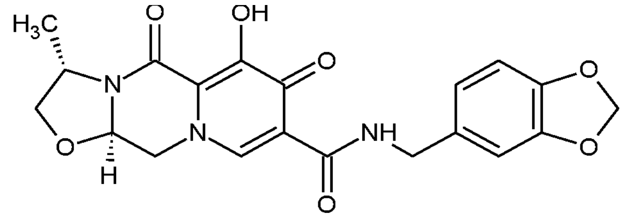 1.1.34 |
| 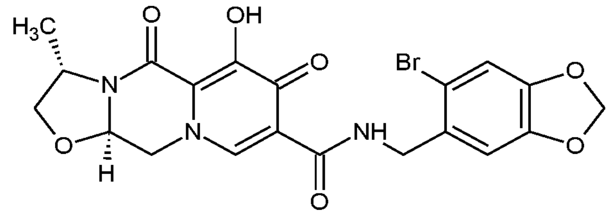 1.1.35 | 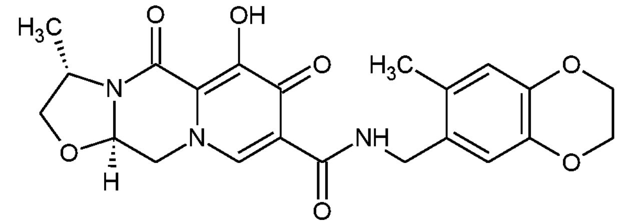 1.1.36 |
| 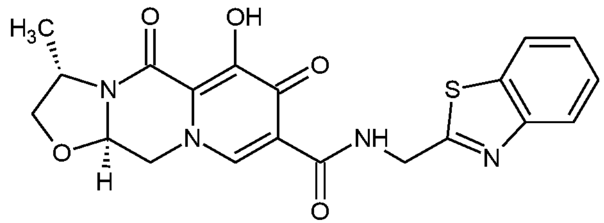 1.1.37 | 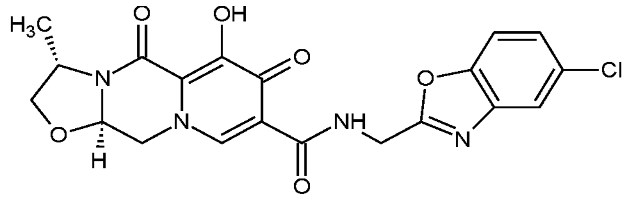 1.1.38 |
| 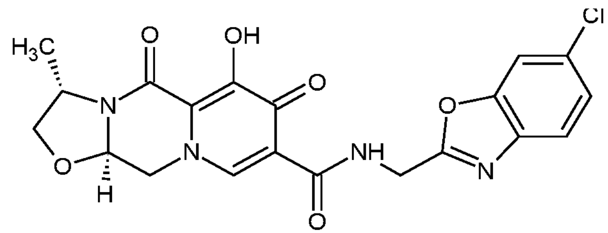 1.1.39 | 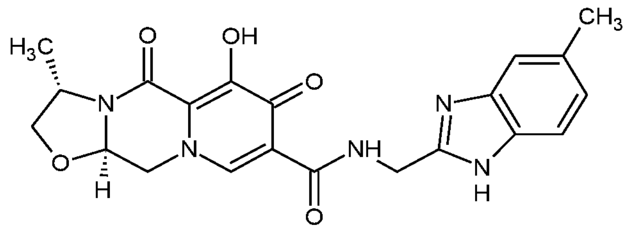 1.1.40 |

(continued)

| | |
|---|---|
| 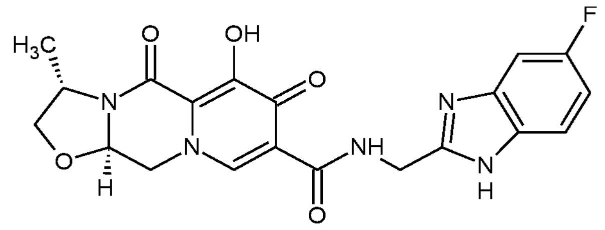 1.1.41 | 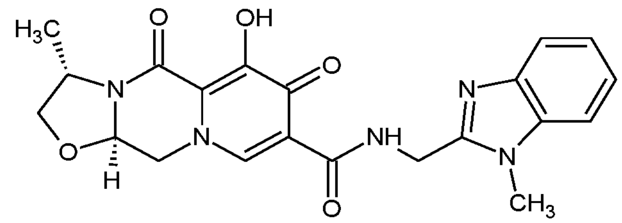 1.1.42 |
| 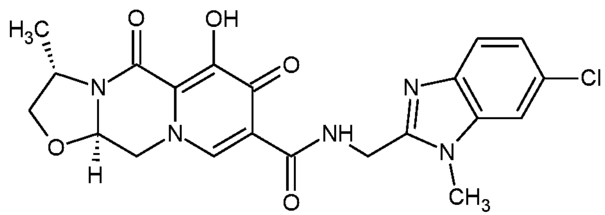 1.1.43 | 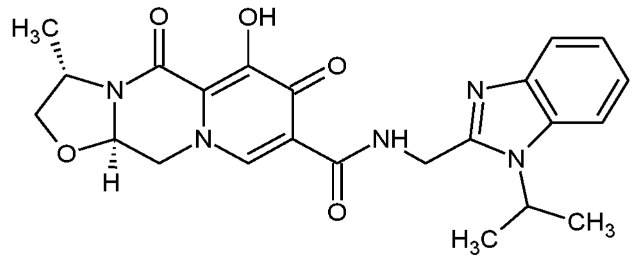 |
| | 1.1.44 |
| 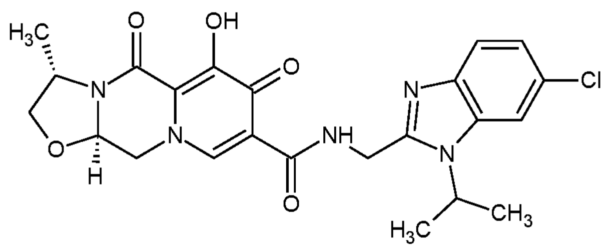 1.1.45 | 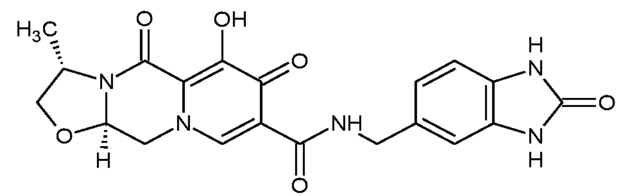 1.1.46 |
| 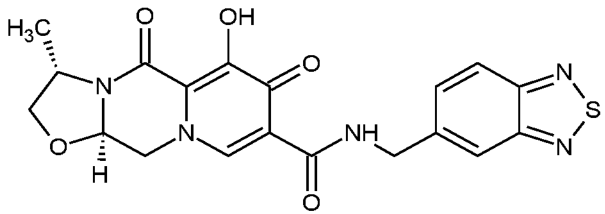 | 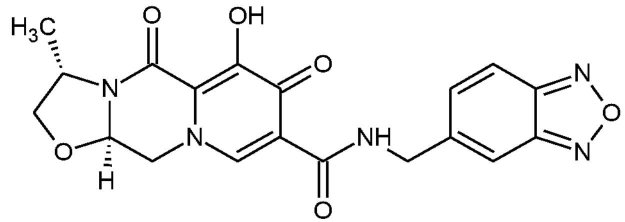 |

(continued)

| | |
|---|---|
| **1.1.47** | **1.1.48** |
| 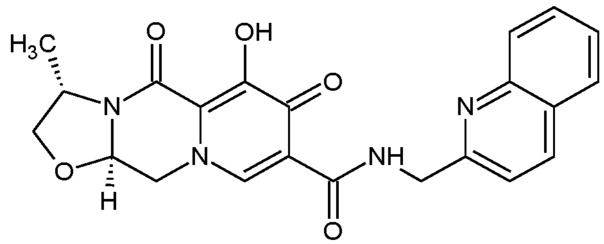 **1.1.49** | 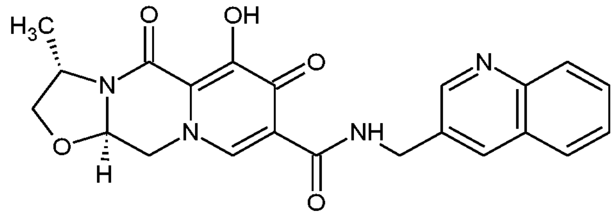 **1.1.50** |
| 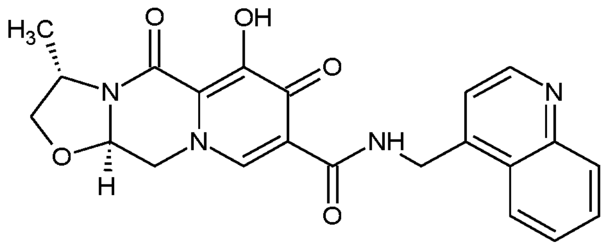 **1.1.51** | 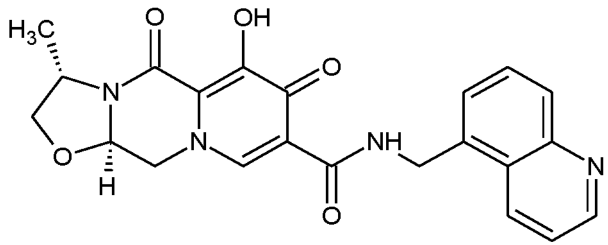 **1.1.52** |
| 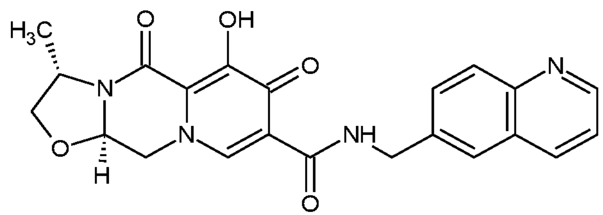 **1.1.53** | 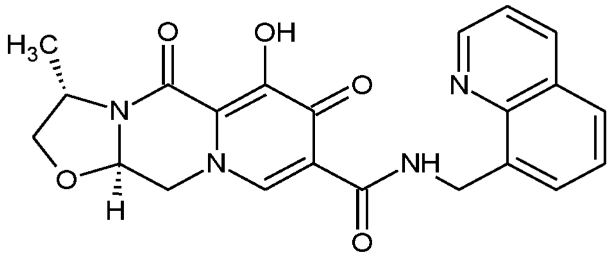 **1.1.54** |

(continued)

| | |
|---|---|
| 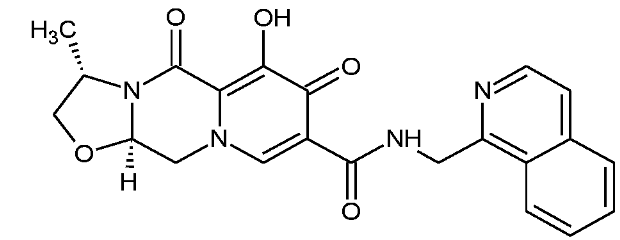 1.1.55 | 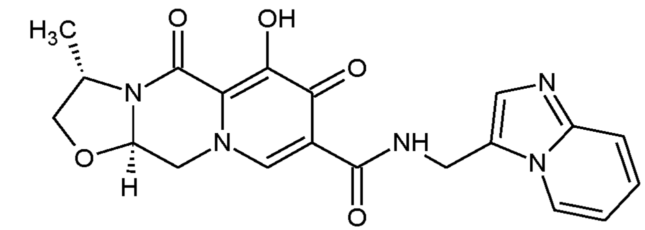 1.1.56 |
| 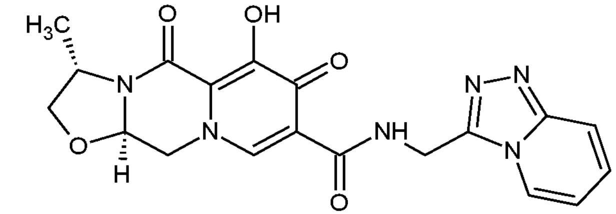 1.1.57 | 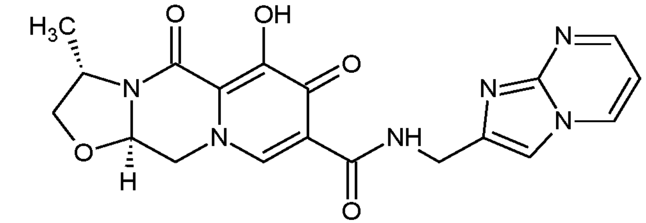 1.1.58 |
| 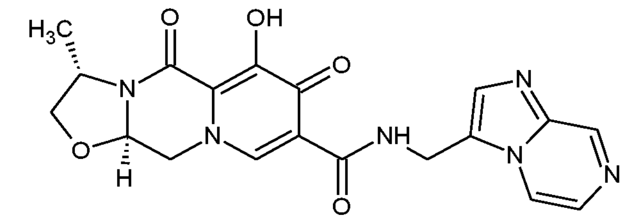 1.1.59 | 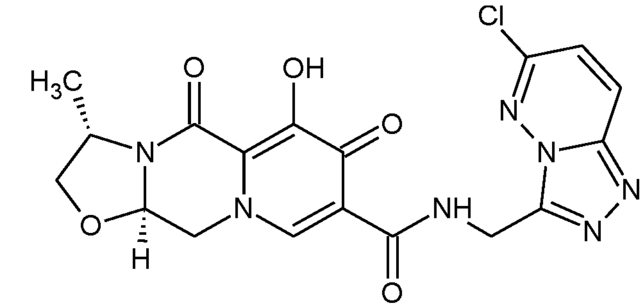 1.1.60 |
| 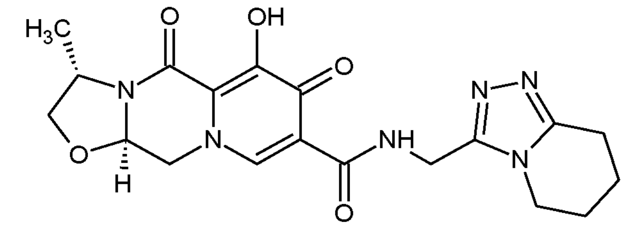 | 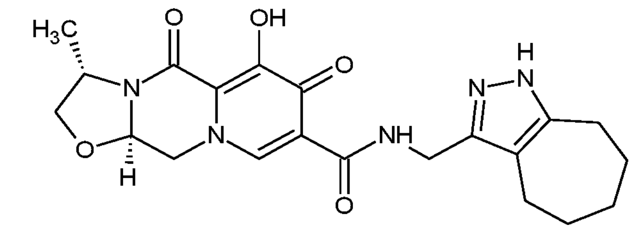 |

(continued)

| 1.1.61 | 1.1.62 |
|---|---|
| 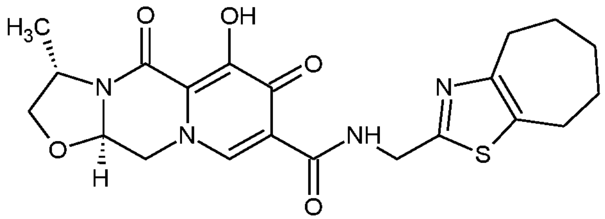 1.1.63 | 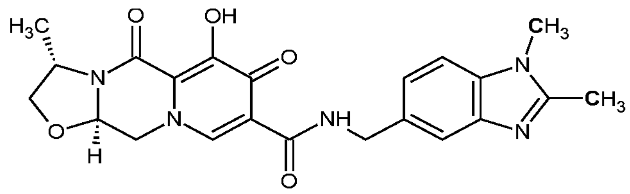 1.1.64 |
| 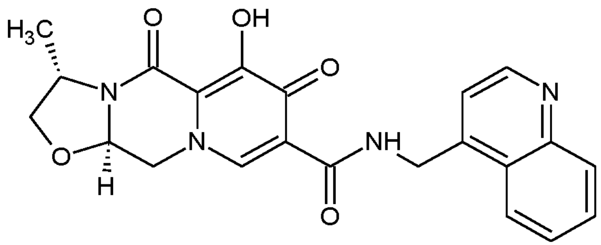 1.1.65 | 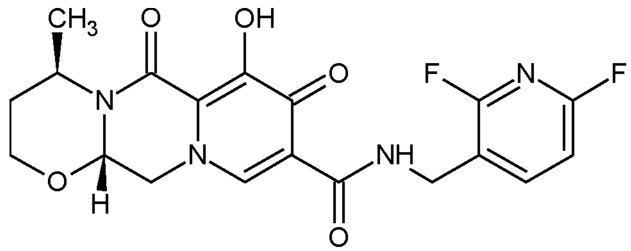 1.2.1 |
| 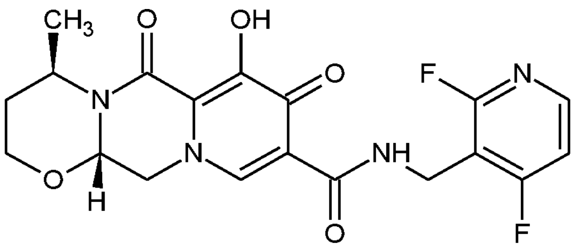 1.2.2 | 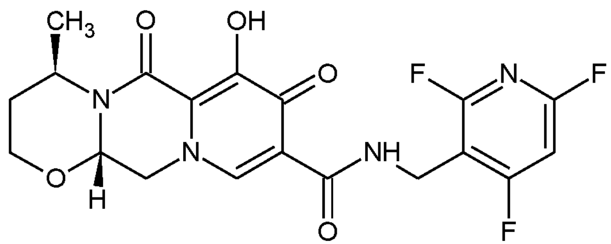 1.2.3 |

(continued)

| | |
|---|---|
| 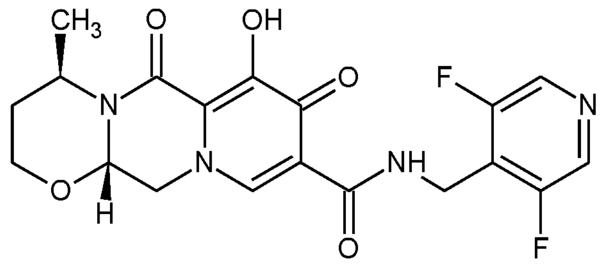 | 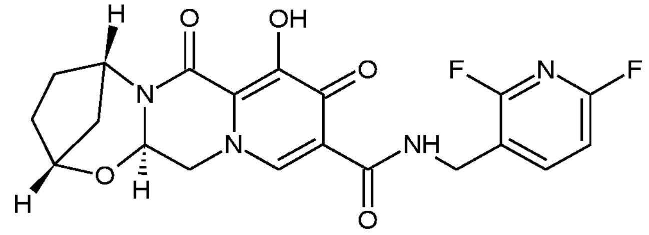 |
| **1.2.4** | **1.3.1** |
| 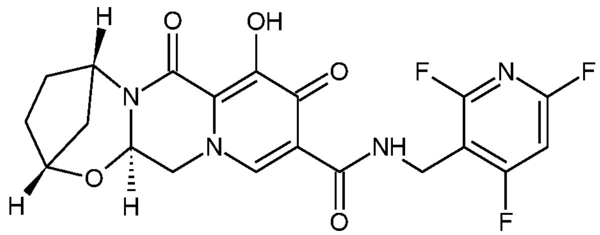 | 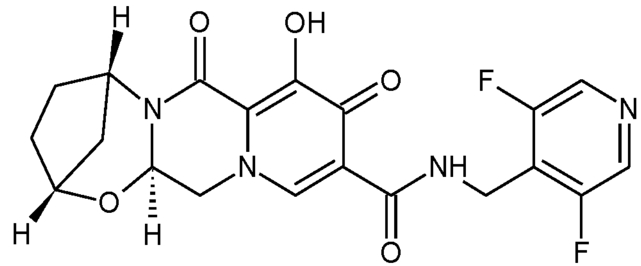 |
| **1.3.2** | **1.3.3** |
| 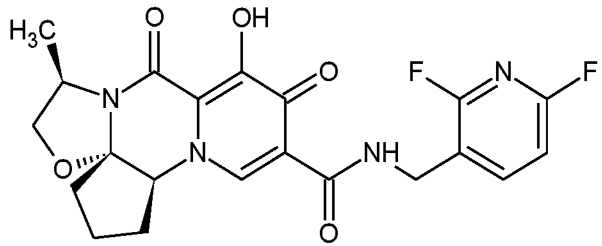 | 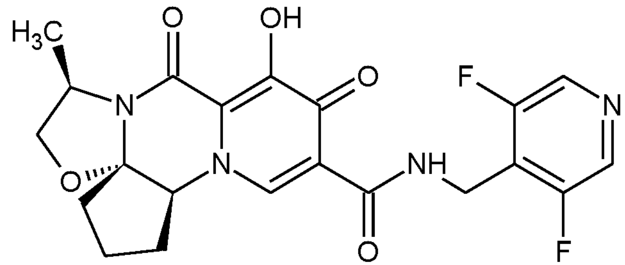 |
| **2.1.1** | **2.1.2** |

(continued)

| | |
|---|---|
| 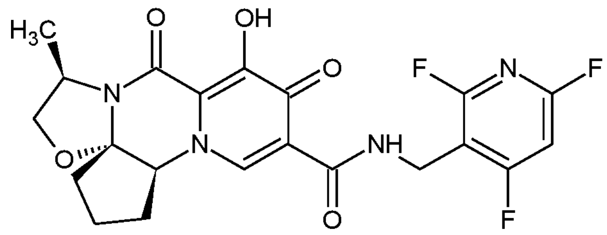 2.1.3 | 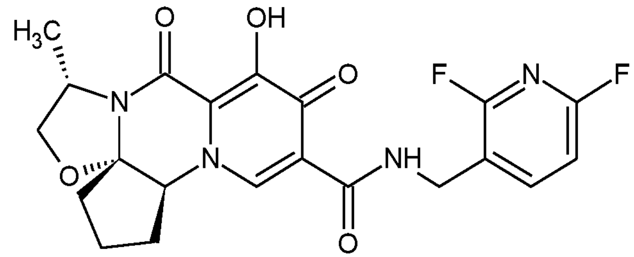 2.2.1 |
| 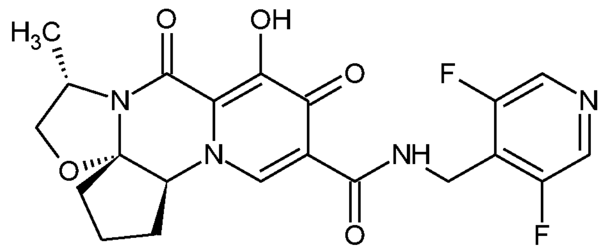 2.2.2 | 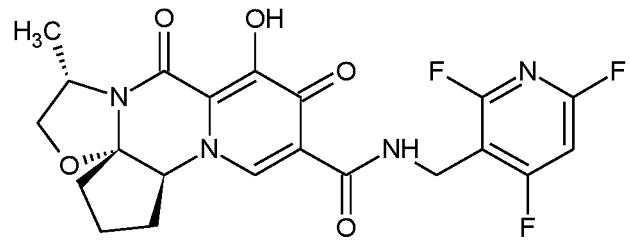 2.2.3 |

The subject of this invention is methods for producing (Scheme 1) annelated 9-hydroxy-1,8-dioxo-N- (pyridinylmethyl) -1,3,4,8-tetrahydro-2H-pyrido[1,2-*a*]pyrazine-7-carboxamides of general formula 1 or 2 and stereoisomers thereof by the interaction of corresponding bromides (3, 4) and heterocyclylmethylamines in dimethyl sulfoxide in the presence of CO and $Pd(PPh_3)_4$ at elevated temperature followed by debenzylation of the resulting compounds (5, 6) to yield target products (1, 2)

Scheme 1

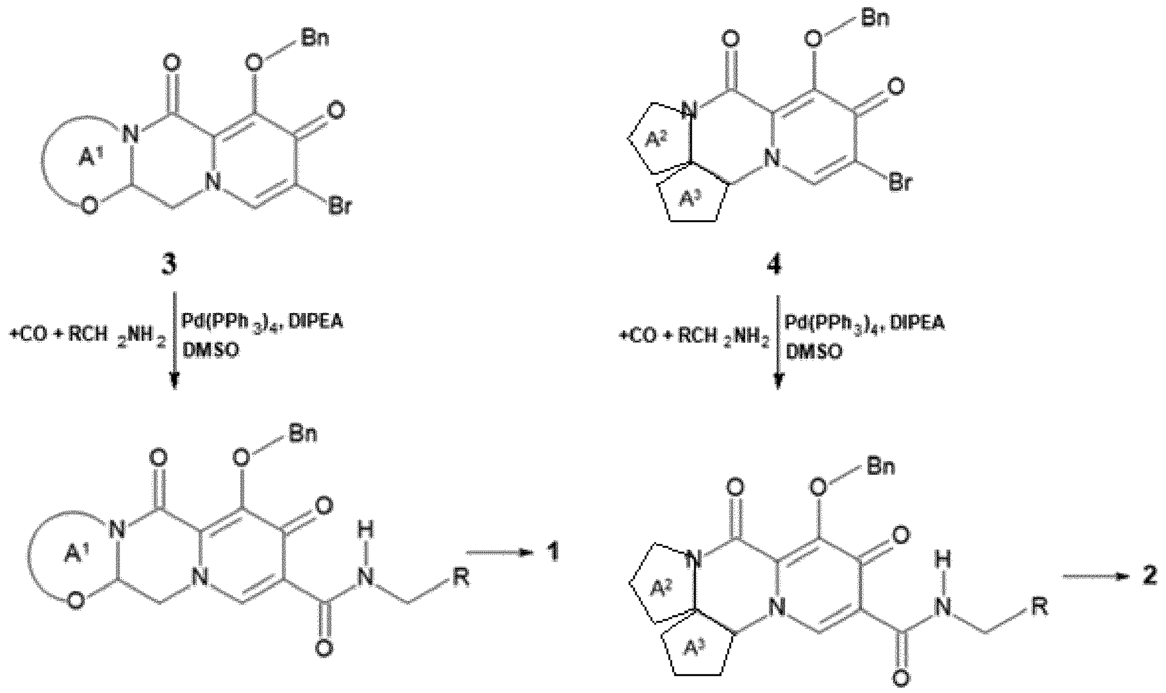


5 6

wherein Bn, R, $A^1$, and $A^2$ are as given above.

Another dubject of this invention is a method to produce (Scheme 2) annelated 9-hydroxy-1,8-dioxo-*N*-(pyridinylmethyl)-1,3,4,8-tetrahydro-2H-pyrido[1,2-*a*]pyrazine-7-carboxamides of general formula (1 or 2) and stereoisomers thereof by by acylation with corresponding acids (7 or 8) of heterocyclylmethylamines and subsequent debenzylation of the resulting products (5, 6) to yield (1, 2)

Scheme 2

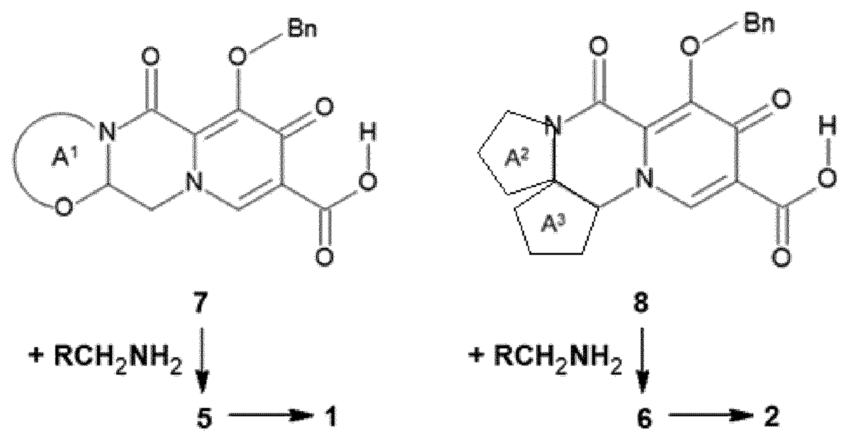


wherein Bn is as given above.

The nanomolar inhibitory activity of the novel compounds of general formula 1 or 2 (Table 1) is comparable to the activity of the most advanced integrase inhibitors CAB, DLG, and BIC. Thus, compounds 1.1.7, 1.1.17, 1.1.19, and 1.1.48 have $EC_{50}$ values of 0.59 nM, 0.24 nM, 0.24 nM, and 0.43 nM, respectively. This result was unexpected, since it was known that the substitution of benzyl moieties with a heterocyclylmethyl moiety in BIC and analogs thereof leads to a drastic decrease in inhibitory activities. Specifically, only two inhibitors-A4 and A5-are known to comprise a pyridin-2-ylmethyl moiety instead of a benzyl moiety [WO 2014/100323]. Indeed, substitution in the BIC (compound 42, $EC_{50}$ = 2.5 nM in WO 2014/100323) of the benzyl moiety with a pyridin-2-ylmethyl moiety leads to an A4 inhibitor (compound 49, $EC_{50}$ = 33.3 nM in WO 2014 /100323), whose activity is 13.3 times lower than that of BIC. A similar pattern is observed for a pair of inhibitors A5 (compound 49, $EC_{50}$ = 17.8 nM in WO 2014/100323) and A6 (compound 66, $EC_{50}$ = 9.4 nM in WO 2014/100323). The substitution of the benzyl moiety in A6 with a pyridin-2-yl moiety leading to A5 is accompanied by an almost twofold decrease in activity.

The novel compounds of general formula 1 or 2 possess ADME properties required for drug candidates.

Depending on their structure, they dissolve differently in aqueous solutions (Table 2). For example, the solubility in water (0.00285 μg / ml) of compound 1.1.48 is almost three times lower than the solubility of CAB (0.008 μg / ml), which can be a significant advantage when using 1.1.48 as an INI in long-term injection HIV therapy At the same time, for example, the solubility of the sodium salt 1.1.20 (2.679 μg/ml) of compound 1.1.19, as well as the solubility of INI 1.1.19 (0.269 μg/ml), exceeds the solubility of CAB by orders of magnitude, which makes them more promising when using as INI in oral HIV therapy. This is confirmed, in particular, by comparing the pharmacokinetic parameters obtained from oral and intravenous administration of compound 1.1.19 and the prototype CAB to rats under identical conditions (Table 3). As can be seen from Table 3, in the case of intravenous injection, the pharmacokinetic parameters $AUC_{INF}$, $AUC_{last}$, $C_{max}$, and $T_{1/2}$ of compound 1.1.4 are considerably higher than those for CAB. An even greater difference in these parameters (two or more times) is observed when these compounds are aministered to rats orally (Table 3). In addition, the time to reach peak plasma concentration ($T_{1/2}$ = 16.5 h) is noticeably higher than that for CAB ($T_{1/2}$ = 18.3 h), and the bioavailability of compound 1.1.19 (17.5 %) calculated by the formula: F =

($AUC_{last}$ $^{PO}$/5·$AUC_{last}$ $^{IV}$) ·100 is twice as high as the bioavailability of CAB (8.8%). S9 фракции печени человека

The novel compounds of general formula 1 or 2 are stable in human and murine plasma (Figure 2) and human and murine liver S9 fraction (Figure 3) and have a high degree of binding to plasma proteins of humans and rats (Table 4).

The subject of this invention is a pharmaceutical composition containing a compound of general formula (1) or a compound of general formula (2) or a stereoisomer thereof, or a pharmaceutically acceptable salt thereof, or a solvate thereof, or a crystalline or polycrystalline form thereof.

The subject of this invention is a pharmaceutical composition containing a compound of general formula 1.1, 1.2, 1.3, 2.1, or 2.2 or a stereoisomer thereof, or a pharmaceutically acceptable salt thereof, or a solvate thereof, or a crystalline or polycrystalline form thereof and a pharmaceutically acceptable excipient.

The subject of this invention is a pharmaceutical composition containing a compound selected from the range 1.1.1-1.1.65, 1.2.1-1.2.4, 1.3.1-1.3.3, 2.1.1-2.1.3, 2.2.1-2.2.3 or a stereoisomer thereof, or a pharmaceutically acceptable salt thereof, or a solvate thereof, or a crystalline or polycrystalline form thereof and a pharmaceutically acceptable excipient.

The subject of this invention is a pharmaceutical composition containing a compound of general formula (1) or (2) or a stereoisomer thereof, or a pharmaceutically acceptable salt thereof, or a solvate thereof, or a crystalline or polycrystalline form thereof as well as one or more additional therapeutic agents.

The subject of this invention is a pharmaceutical composition containing a compound of general formula (1) or (2) or a stereoisomer thereof, or a pharmaceutically acceptable salt thereof, or a solvate thereof, or a crystalline or polycrystalline form thereof as well as one or more anti-HIV agents.

The subject of this invention is a pharmaceutical composition containing a compound of general formula (1) or (2), or a stereoisomer thereof, or a pharmaceutically acceptable salt thereof, or a solvate thereof, or a crystalline or polycrystalline form thereof as well as one or more additional therapeutic agents selected from a group consisting of HIV protease inhibitors, non-nucleoside HIV reverse transcriptase inhibitors, nucleoside or nucleotide HIV reverse transcriptase inhibitors, allosteric HIV integrase inhibitors, HIV capsid assembly inhibitors, and combinations thereof.

The subject of this invention is a pharmaceutical composition containing a compound of general formula (1) or (2) or a stereoisomer thereof, or a pharmaceutically acceptable salt thereof, or a solvate thereof, or a crystalline or polycrystalline form thereof as well as a first additional therapeutic agent selected from the group consisting of abacavir sulfate, tenofovir, tenofovir disoproxil, tenofovir disoproxil fumarate, tenofovir alafenamidee, tenofovir alafenamidee hemifumarate, tenofovir cyclobutylalafenamide, tenofovir cyclobutylalafenamide fumarate, tenofovir cyclobutylalafenamide hemifumarate, elsulfavirin, VM-1500A, GS-CA1, and a second additional therapeutic agent selected from the group consisting of emtricitabine and lamivudine.

The subject of this invention is a pharmaceutical composition containing a compound of general formula (1) or

(2) or a stereoisomer thereof, or a pharmaceutically acceptable salt thereof, or a solvate thereof, or a crystalline or polycrystalline form thereof as well as a first additional therapeutic agent selected from the group consisting of tenofovir cyclobutylalafenamide tenofovir cyclobutylalafenamide or tenofovir cyclobutylalafenamide tenofovir cyclobutylalafenamide and tenofovir cyclobutylalafenamide hemifumarate, tenofovir cyclobutylalafenamide hemifumarate, and emtricitabine.

The subject of this invention is a pharmaceutical composition containing a compound of general formula (1) or (2) or a stereoisomer thereof, or a pharmaceutically acceptable salt thereof, or a solvate thereof, or a crystalline or polycrystalline form thereof as well as one or more anti-hepatitis C agents (HCV) and/or hepatitis B (HBV).

Anti-HCV agents may include:

- prodrugs of HCV NS5B nucleoside inhibitors, for instance, Sovaldi® (Sofosbuvir, PSI-7977, GS-7977) [https://www.accessdata.fda.gov/drugsatfda_docs/label/ 2015/204671s004lbl.pdf] or cyclobutyl (S)-2-{(S)-[(2R,3R,4R,5R)-5-(3,4-dihydro-2,4-dioxo-2*H*-pyrimidin-1-yl)-3-hydroxy-4-methyl-4-fluoro-tetrahydrofuran-2-ylmethoxy]-phenoxy-phosphorylamino}-propanoate [PatentRU 2644256 (2018)];

  - HCV NS5A inhibitors, for example, Daklinza (Daclatasvir, BMS790052) [https://www.accessdata.fda.gov/drugsatfda_docs/label/2017/206843s006lbl.pdf]; Hepavivir (AV-4025) [Ivachtchenko, A. V. et al. Discovery of Novel Highly Potent Hepatitis C Virus NS5A Inhibitor (AV-4025). J. Med. Chem. 2014, 57, 7716-7730. Patent WO 2012/074437 (2012), US 9428491, 2016.]; Ombitasvir (ABT-267) [https://www.drugbank.ca/drugs/DB09296]; Elbasvir (MK-8742), [https://www.drugbank.ca/drugs/DB11574]; Velpatasvir, (VEL, GS-5816), [https://www.drugbank.ca/drugs/DB11613]; Pibrentasvir (ABT-530; ABT530; ABT 530) [https://www.drugbank.ca/drugs/DB13878]; Grazoprevir (MK-5172) [https://www.drugbank.ca/drugs/DB1157];

  - HCV NS3 inhibitors, for example, Narlaprevir (SCH 900518) [https://newdrugapprovals.org/tag/narlaprevir/)/] ;

  - HCV NS3/NS4 inhibitots, for example, **Olysio** (Simeprevir) [https://www.accessdata.fda.gov/drugsatfda_docs/label/2013/205123s001lbl.pdf]; Voxilaprevir (GS-9857; GS 9857; GS9857) [https://www.drugbank.ca/drugs/ DB12026] ;

- combinations of the above-mentioned HCV inhibitors [V. Soriano et al. Treatment of hepatitis C with new fixed dose combinations. Expert Opin Pharmacother. 2017 Aug;18(12):1235-1242.], including fixed combinations, , for example, EPCLUSA® (sofosbuvir and velpatasvir) [https://www.accessdata.fda.gov/drugsatfda-docs/label/2016/208341s000lbl.p df];

Zepatier (Elbasvir/grazoprevir) [https://www.merck.com/product/usa/pi_circulars/z/zepatier/ zepatier_pi.pdf]; Viekira XR™ (dasabuvir, ombitasvir, paritaprevir, and ritonavir) [https://www.rxabbvie.com/pdf/viekiraxr_pi.pdf]; Mavyret (glecaprevir/pibrentasvir) [https://www.rxabbvie.com/pdf/mavyret_pi.pdf], and others. Anti-HCV agents may include Baraclude (entecavir) [https://packageinserts.bms.com/pi/pi_baraclude.pdf]; Epivir (lamivudine) [https://www.ema.europa.eu/documents/product-information/epivir-epar-product-information_en.pdf]; Hepsera (Adefovir, adefovir dipivoxil) [https://www.ema.europa.eu/documents/product-information/hepsera-epar-product-information_en.pdf]; Tyzeka (telbivudine) [https://www.accessdata.fda.gov/drugsatfda_ docs/label/2006/022011lbl.pdf]; Viread (tenofovir) [https://www.ema.europa.eu/ documents/variation-report/viread-h-c-419-ii-0120-epar-assessment-report-variation_en.pdfl; Vemlidy (tenofovir alafenamidee fumarate, TAF) [https://www.gilead.eom/~/ media/files/pdfs/medicines/liver-disease/vemlidy/vemlidy_pi.pdf?la=en]; Cyclobutyl (S)-2-[[[(R)-2-(6-aminopurin-9-yl)-1-methyl-ethoxy]methyl-phenoxy-phosphoryl]amino]-propanoate fumarate [Patent RU 2674576 (2018)], and others.

A further subject of this invention is a pharmaceutical composition in the form of lyophilizate obtained by freeze drying a nanosuspension of a compound of general formula 1 or 2, with a particle size of 200 nm to 900 nm, preferably 200 nm, containing pharmaceutically acceptable excipients.

The method for preparing a lyophilized pharmaceutical composition consists in grinding wet granules of the compound of general formula 1 or 2 with excipients and water to obtain a particle size of 200-900 nm, preferably 200 nm, followed by lyophilization of the resulting suspension.

Excipients for said lyophilized pharmaceutical composition are selected from mannite, polysorbate, polyethylene glycol, poloxamer, mannitol, and sucrose.

The subject of this invention is also an injectable drug for long-term supportive therapy of HIV infection, said drug comprising a pharmaceutical composition containing a lyophilized compound of general formula 1 or 2, a phosphate buffered saline solution, and water for injection.

The method for preparing a novel injectable drug consists in mixing a pharmaceutical composition containing a lyophilized compound of general formula 1 or 2, a phosphate buffered saline (PBS) solution of pH = 6.8, and water

for injection.

The subject of this invention is a method to treat HIV infection in an HIV-infected or HIV-exposed individual by administering to said individual a therapeutically effective amount of the compound of general formula (1) or (2) or a stereoisomer thereof, or a pharmaceutically acceptable salt thereof, or a solvate, or a crystalline or polycrystalline form thereof, or a pharmaceutical composition disclosed herein, or an injectable drug.

The subject of this invention is a method for the prevention and treatment of HIV infection in a HIV-infected or HIV-exposed individual by administering to said individual a therapeutically effective amount of the compound of general formula (1) or (2), or a stereoisomer thereof, or a pharmaceutically acceptable salt thereof, or a solvate thereof, or a crystalline or polycrystalline form thereof, or a pharmaceutical composition or injectable drug disclosed herein with further administering to said person a therapeutically effective amount of one or more additional therapeutic agents.

The subject of this invention is a method for the prevention and treatment of HIV infection in a HIV-infected or HIV-exposed person by administering to said person a therapeutically effective amount of the compound of general formula (1) or (2), or a stereoisomer thereof, or a pharmaceutically acceptable salt thereof, or a solvate thereof, or a crystalline or polycrystalline form thereof, or a pharmaceutical composition or injectable drug disclosed herein with further administering to said person a therapeutically effective amount of one or more additional therapeutic agents selected from the group consisting of HIV protease inhibitors, non-nucleoside HIV reverse transcriptase inhibitors, nucleoside or nucleotide HIV reverse transcriptase inhibitors, allosteric HIV integrase inhibitors, HIV capsid assembly inhibitors, and combinations thereof.

In a particular embodiment, the method used for preventing and treating HIV infecttion in a HIV-infected or HIV-exposed person involves administering to said person a therapeutically effective amount of the compound of general formula (1) or (2), or a stereoisomer thereof, or a pharmaceutically acceptable salt thereof, or a solvate thereof, or a crystalline or polycrystalline form thereof, or a pharmaceutical composition or injectable drug disclosed herein with further administering to said person a therapeutically effective amount of non-nucleoside HIV reverse transcriptase inhibitors.

The subject of this invention is a method for the prevention and treatment of HIV infection in a HIV-infected or HIV-exposed person by administering to said person a therapeutically effective amount of the compound of general formula (1) or (2), or a stereoisomer thereof, or a pharmaceutically acceptable salt thereof, or a solvate thereof, or a crystalline or polycrystalline form thereof, or a pharmaceutical composition or injectable drug disclosed herein with further administering to said person a therapeutically effective amount of a first additional therapeutic agent selected from the group consisting of abacavir sulfate, tenofovir, tenofovir disoproxil, tenofovir disoproxil fumarate, tenofovir alafenamidee, tenofovir alafenamidee hemifumarate, tenofovir cyclobutylalafenamide, tenofovir cyclobutylalafenamide fumarate and tenofovir cyclobutylalafenamide hemifumarate, elsulfavirine, VM-1500A, GS-CA and a second additional therapeutic agent selected from the group consisting of emtricitabine and lamivudine.

The subject of this invention is a method for the prevention and treatment of HIV infection in a HIV-infected or HIV-exposed individual by administering to said individual a single dosage form for a one-time administration, for example, in solid dosage form for oral administration of a compound of general formula (1) or (2), or a stereoisomer thereof, or a pharmaceutically acceptable salt thereof, or a solvate thereof, or a crystalline or polycrystalline form thereof in combination with a first additional therapeutic agent selected from the group consisting of abacavir sulfate, tenofovir, tenofovir disoproxil, tenofovir disoproxil fumarate, tenofovir alafenamide, tenofovir alafenamide hemifumarate, tenofovir cyclobutylalafenamide, tenofovir cyclobutylalafenamide fumarate and tenofovir cyclobutylalafenamide hemifumarate, elsulfavirine, VM-1500A, GS-CA, and a second additional therapeutic agent selected from the group consisting of emtricitabine and lamivudine.

The subject of this invention is the use of a compound of general formula (1) or (2) or a stereoisomer thereof, or a pharmaceutically acceptable salt thereof, or a solvate thereof, or a crystalline or polycrystalline form thereof in antiretroviral therapy (ART), combination antiretroviral therapy cART, and Antiretroviral Therapy as Long Acting Suppression (ATLAS) for treating HIV infection in a HIV-infected or HIV-exposed person.

The subject of this invention is the use of a pharmaceutical composition or injectable drug disclosed herein in antiretroviral therapy (APT), combination antiretroviral therapy (cAPT), and antiretroviral therapy as long acting suppression (ATLAS) for treating HIV infection in a HIV-infected or HIV-exposed person.

The subject of this invention is the use of a compound of general formula (1) or (2) or a stereoisomer thereof, or a pharmaceutically acceptable salt thereof, or a solvate thereof, or a pharmaceutical composition or an injectable drug disclosed herein in pre-exposure prophylaxis before an individual comes into contact with HIV to prevent transmission of HIV infection in case an individual comes into contact with the virus, and/or to prevent persistent viral infection, and/or to prevent the onset of symptoms, and/or to prevent the appearance of detectable levels of virus in the blood.

The subject of this invention is a method of inhibiting HIV replication using a therapeutically effective amount of a compound of general formula (1) or (2) or a stereoisomer thereof, or a pharmaceutically acceptable salt thereof, or a solvate thereof, or a crystalline or polycrystalline form thereof, or a pharmaceutical composition or injectable drug disclosed herein to affect HIV under conditions that promote HIV replication inhibition.

The subject of this invention is the use of a compound of general formula (1) or (2), or a stereoisomer thereof,

or a pharmaceutically acceptable salt thereof, or a solvate thereof or a pharmaceutical composition or injectable drug disclosed herein for inhibiting the activity of an HIV integrase enzyme.

The subject of this invention is the use of a compound of general formula (1) or (2), or a stereoisomer thereof, or a pharmaceutically acceptable salt thereof, or a solvate thereof or a pharmaceutical composition or injectable drug disclosed herein as an agent for HIV inhibition.

It should be understood that in any embodiment of this invention set forth above, the compound of general formula (1) or (2) or a stereoisomer thereof, or a pharmaceutically acceptable salt thereof, or a solvate thereof, or a crystalline or polycrystalline form thereof, or any particular substituent described herein as a group of $A^{1,}$ $A^2$, $A^3$, or R in the compounds of formulas (1) or (2) or in a stereoisomer thereof, or in a pharmaceutically acceptable salt thereof, or in a solvate thereof, or in a crystalline or polycrystalline form thereof, as set forth herein above, can independently be combined with other embodiments and/or substituents of a compound of any of formulas (1) or (2) or a stereoisomer thereof, or a pharmaceutically acceptable salt thereof, or a solvate thereof, or a crystalline or polycrystalline form thereof to obtain embodiments that are not specifically described above. In addition, if the list of substituents is given for any specific $A^{1,}$ $A^2$, $A^3$, or R in a particular embodiment and/or claim, it should be understood that each individual substituent may be excluded from a particular embodiment and / or a claim and that the remaining list of substituents will be within the scope of the embodiments disclosed herein.

The administration to a subject of a compound of general formula (1) or (2) or a stereoisomer thereof, or a pharmaceutically acceptable salt thereof, or a solvate thereof, or a crystalline or polycrystalline form thereof, according to embodiments as disclosed herein, in pure form or as part of a corresponding pharmaceutical composition or an injectable drug disclosed herein can be performed by any customary way of administering agents of similar purpose.

The pharmaceutical compositions disclosed herein, according to embodiments as disclosed herein, can be prepared by combining the compound according to embodiments as disclosed herein with a suitable pharmaceutically acceptable excipient and can be formulated in solid, semi-solid, or liquid gaseous forms such as tablets, capsules, powders, granules, ointments, nanosuspensions, solutions, suppositories, injections, inhalants, gels, microspheres, and aerosols. Typical routes of administration of said pharmaceutical compositions include, but are not limited to, oral, topical, transdermal, pulmonary, parenteral, sublingual, buccal, rectal, vaginal, and intranasal.

Pharmaceutical compositions according to embodiments as disclosed herein are formulated so as to provide bioavailability of the active ingredients within the composition to be administered to a subject. Said compositions are administered to a subject or a patient in dosage forms containing one or more dosage units, where, for example, a tablet may be a dosage form containing one dosage unit and a container with a compound according to embodiments as disclosed herein, in the form of an aerosol may contain many dosage units.

Actual methods for preparing said dosage forms are known or will be apparent to those skilled in the art; see, for example, Remington. The Science and Practice of Pharmacy, 20th Edition (Philadelphia College of Pharmacy and Science, 2000). The composition to be administered will in any case comprise a therapeutically effective amount of a compound according to embodiments as disclosed herein, or a pharmaceutically acceptable salt thereof for treating a disease or condition of interest in accordance with the recommendations of the description herein.

In one embodiment of this invention, the pharmaceutical composition is in oral unit dosage form.

In another embodiment, the pharmaceutical composition is a solid oral unit dosage form.

In another embodiment, the pharmaceutical composition is a tablet.

In another embodiment, the pharmaceutical composition is a capsule.

In another embodiment, the pharmaceutical composition is a lyophilized nanosuspension placed in a pre-sterilized glass bottle, covered with a pre-sterilized stopper and tightly sealed.

The pharmaceutical compositions disclosed herein can be prepared by methods well known in the pharmaceutical field. For example, a pharmaceutical composition intended for administration by injection may be prepared by combining the compound of the embodiments disclosed herein with sterile distilled water to form a solution or nanosuspension. To ensure homogeneity of the solution or nanosuspension, a surfactant may be added.

Surfactants are compounds that non-covalently interact with a compound according to embodiments as disclosed herein and facilitate dissolution thereof or homogeneity of the nanosuspension of the compound in an aqueous delivery system.

Compounds of general formula (1) or (2) or a stereoisomer thereof, or a pharmaceutically acceptable salt thereof, or a solvate thereof, or a crystalline or polycrystalline form thereof according to embodiments as disclosed herein, as is or within a corresponding pharmaceutical composition, is administered to a subject or patient in a therapeutically effective amount, which will vary depending on a variety of factors like the activity of the particular compound used; metabolic stability and duration of action of the compound; the patient's age, body weight, general health condition, gender, and diet; mode and time of administration; excretion rate; a combination of drugs; the severity of the particular disorder or condition, and the subject being treated.

In some embodiments of the invention, for the treatment or prevention of HIV infection in a HIV-infected or HIV-exposed person, a method is provided comprising administering to a person a therapeutically effective amount of a

compound disclosed herein, or a stereoisomer thereof, or a pharmaceutically acceptable salt thereof, or a solvate thereof, disclosed herein, or a pharmaceutical composition disclosed herein in combination with a therapeutically effective amount of one or more (for example, one, two, three, one or two, or from one to three) additional therapeutic agents (up to three).

In some embodiments of this invention, a method is proposed for treating an HIV infection, as provided herein, involving administering to the patient, if necessary, a therapeutically effective amount of the compound or a stereoisomer thereof, or a pharmaceutically acceptable salt or solvate thereof, or a crystalline or polycrystalline form thereof, as disclosed herein, in combination with a therapeutically effective amount of one or more (e.g., one, two, three, one or two, or one to three) additional therapeutic agents suitable for the treatment of HIV infection.

The compound disclosed herein (for example, any compound of formula (1) or (2) or a stereoisomer thereof, or a pharmaceutically acceptable salt thereof, or a solvate thereof, or a crystalline or polycrystalline form thereof) may be combined with one or more additional therapeutic agents at any dose of the compound of formula (1) or (2) or a stereomer thereof, or a pharmaceutically acceptable salt thereof, or a solvate thereof, or a crystalline or polycrystalline form thereof (for example, from 50 mg to 1000 mg of the compound).

In one embodiment of the invention disclosed herein, a compound of formula (1) or (2) or a stereoisomer thereof, or a pharmaceutically acceptable salt thereof, or a solvate thereof, or a crystalline or polycrystalline form thereof are used in combination with one or more (e.g. one, two, three, one or two, or from one to three) additional therapeutic agents and a pharmaceutically acceptable excipient.

In the above embodiments, the additional therapeutic agent may be an anti-HIV agent. For example, in some embodiments of this invention, the additional therapeutic agent is selected from the group consisting of HIV protease inhibitors, non-nucleoside or non-nucleotide HIV reverse transcriptase inhibitors, nucleoside or nucleotide HIV reverse transcriptase inhibitors, HIV integrase inhibitors, non-catalytic (or allosteric) site HIV integrase inhibitors, HIV nucleoside or nucleotide reverse transcriptase inhibitors, HIV capsid assembly inhibitors, HIV penetration inhibitors (e.g., CCR5 inhibitors, gp41 inhibitors (t + e + fusion inhibitors) and CD4 attachment inhibitors, CXCR4 inhibitors, gpl20 inhibitors, G6PD and NADH oxidase inhibitors, anti-HIV vaccines, HIV maturation inhibitors, latency reversing agents (e.g., histone deacetylase inhibitors, proteasome inhibitors, protein kinase C (PKC) activators and BRD inhibitors), HIV capsid compounds ("capsid inhibitors," for example capsid polymerization inhibitors or capsid disrupting compounds, HIV p7 nucleocapsid protein inhibitors (NCp7), and HIV p24 capsid protein inhibitors), pharmacokinetic enhancers, immunological treatments (e.g., Pd-1 modulators, Pd-LI modulators, toll-like receptor modulators, IL-15 agonists), HIV antibodies, bispecific antibodies and "antibody-like" therapeutic proteins (e.g., DARTs®, Duobodies®, Bites®, XmAbs®, TandAbs®, Fab derivatives), including drugs acting on HIV gpl20 or gp41, anti-HIV combination drugs, HIV p17 matrix protein inhibitors, IL-13 antagonists, peptidyl-prolyl *cis*/*trans* isomerase A modulators, protein disulfide isomerase inhibitors, C5a complement receptor antagonists, DNA methyl transferase inhibitors, HIV vif gene modulators, HIV-1 viral infectivity factor inhibitors, TAT protein inhibitors, HIV-1 Nef modulators, Nek tyrosine kinase modulators, mixed-lineage kinase 3 (MLK-3) inhibitors, HIV-1 splicing inhibitors, Rev protein inhibitors, integrin antagonists, nucleoprotein inhibitors, splicing factor modulators, COMM domain-containing protein 1 modulators, HIV ribonuclease H inhibitors, retrocyclin modulators, CDK-9 inhibitors, dendritic cell-specific ICAM-3-grabbing nonintegrin protein-1 inhibitors, HIV GAG protein inhibitors, HIV POL protein inhibitors, complement factor H modulators, ubiquitin ligase inhibitors, deoxycytidine kinase inhibitors, cyclin-dependent kinase inhibitors, proprotein convertase PC9 stimulators, ATP-dependent RNA helicase DDX3X inhibitors, priming reverse transcriptase complex inhibitors, HIV gene therapy, PI3K inhibitors, compounds similar to those disclosed in WO 2013/006738, US 2013/0165489, WO 2013/091096A1, WO 2009/062285, US 20140221380, US 20140221378, WO 2010/130034, WO 2013/159064, WO 2012/145728, WO 2012/003497, WO 2014/100323, WO2012/145728, WO2013/159064, WO 2012/003498, and WO 2013/006792 and other drugs for HIV therapy and combinations thereof.

In some embodiments, a compound of formula (1) or (2) or a stereoisomer thereof, or a pharmaceutically acceptable salt thereof, or a solvate thereof, or a crystalline or polycrystalline form thereof, is prepared in the form of a tablet, which may optionally contain one or more other compounds used for HIV treatment. In some embodiments, the tablet may contain another active ingredient for treating HIV, such as HIV protease inhibitors, non-nucleoside or non-nucleotide HIV reverse transcriptase inhibitors, nucleoside or nucleotide HIV reverse transcriptase inhibitors, HIV integrase inhibitors, a non-catalytic (or allosteric) HIV integrase site inhibitor, HIV capsid assembly inhibitors, pharmacokinetic enhancers, and combinations thereof.

In some embodiments of the invention, said tablets are suitable for once daily administration.

In some embodiments, the additional therapeutic agent is:

- one or more combination drugs selected from the group comprising ATRIPLA® (efavirenz + tenofovir disoproxil fumarate + emtricitabine), COMPLERA® (EVIPLERA®, rilpivirine + tenofovir disoproxil fumarate + emtricitabine), STRffilLD® (elvitegravir + cobicistat + tenofovir disoproxil fumarate + emtricitabine), dolutegravir + abacavir sulfate + lamivudine, TRJUMEQ® (dolutegravir + abacavir + lamivudine), lamivudine + nevirapine + zidovudine, dolutegravir + rilpivirin, atazanavir sulfate + cobicistat, atazanavir + cobicistat, darunavir + cobicistat, efavirenz + lamivudine +

tenofovir disoproxil fumarate, tenofovir alafenamide hemifumarate + Emtricitabine + Cobicistat + Elvitegravir, tenofovir alafenamide hemifumarate + Emtricitabine, tenofovir alafenamide + emtricitabine, tenofovir alafenamide hemifumarate + emtricitabine + rilpivirine, tenofovir alafenamide + emtricitabine + rilpivirine, Vacc-4x + romidepsin, darunavir + tenofovir alafenamide hemifumarate + emtricitabine + cobicistat, APH-0812, raltegravir + lamivudine, KALETRA® (ALUVIA®, lopinavir + ritonavir), atazanavir sulfate + ritonavir, COMBIVIR® (zidovudine + lamivudine, AZT+3TC), EPZICOM® (Livexa®, abacavir sulfate + lamivudine, ABC+3TC), TRIZIVIR® (abacavir sulfate + zidovudine + lamivudine, ABC+AZT+3TC), TRUVADA® (tenofovir disoproxil fumarate + emtricitabine, TDF+FTC), tenofovir + lamivudine, lamivudine + tenofovir disoproxil fumarate, and lamivudine + tenofovir cyclobutylalafenamide or fumarate or hemifumarate thereof;

- an HIV protease inhibitor selected from the group consisting of amprenavir, atazanavir, fosamprenavir, fosamprenavir calcium, indinavir, indinavir sulfate, lopinavir, ritonavir, nelfinavir, nelfinavir mesylate, saquinavir, saquinavir mesylate, tipranavir, brecanavir, darunavir, DG-17, TMB-657 (PPL-100), and TMC-310911;

- a non-nucleoside or non-nucleotide HIV reverse transcriptase inhibitor selected from the group consisting of delavirdine, delavirdine mesylate, nevirapine, etravirine, dapivirin, doravirine, rilpivirin, efavirenz, KM-023, VM-1500, lentinan, and AIC-292;

- a nucleoside or nucleotide HIV reverse transcriptase inhibitor selected from the group consisting of VIDEX® and VIDEX® EC (didanosine, ddl), zidovudine, emtricitabine, didanosine, stavudine, zalcitabine, lamivudine, censavudin, abacavir, abacavir sulfate, amdoxovir, elvucitabin, alovudine, phosphazide, fozivudine tidoxil, apricitabine, amdoxovir, KP-1461, fosalvudin tidoxil, tenofovir, tenofovir disoproxil, tenofovir disoproxil fumarate, tenofovir disoproxil hemifumarate, tenofovir alafenamide, tenofovir alafenamide hemifumarate, tenofovir alafenamide fumarate, tenofovir cyclobutylalafenamide, tenofovir cyclobutylalafenamide hemifumarate, tenofovir cyclobutylalafenamide fumarate, elsulfavirine, VM-1500A, adefovir, adefovir dipivoxil and festinavir;

- an HIV integrase inhibitor selected from the group consisting of curcumin, curcumin derivatives, chicoric acid, chicoric acid derivatives, 3,5-dicaffeoylquinic acid, 3,5-dicaffeoylquinic acid derivatives, aurintricarboxylic acid, aurintricarboxylic acid derivatives, caffeic acid phenethyl ester, caffeic acid phenethyl ester derivatives, tyrfostin, tyrfostin derivatives, quercetin, quercetin derivatives, raltegravir, elvitegravir, dolutegravir, and cabotegravir;

- an HIV non-catalytic (or allosteric) site integrase inhibitor (NCINI) selected from the group consisting of CX-05168, CX-05045 and CX-14442;

- HIV capsid assembly inhibitors selected from the group consisting of GS-CA1;

- an HIV gp41 inhibitor selected from the group consisting of enfuvirtide, sifuvirtide and albuvirtide;

- an HIV penetration inhibitor selected from the group consisting of cenicriviroc;

- an HIV gpl20 inhibitor selected from the group consisting of Radha-108 (receptol) and BMS -663068;

- a CCR5 inhibitor selected from the group consisting of aplaviroc, vicriviroc, maraviroc, cenicriviroc, PRO-140, adaptavir (RAP-101), TBR-220 (TAK-220), nifeviroc (TD-0232), TD-0680, and vMIP (Haimipu);

- CD4 attachment inhibitors selected from the group consisting of ibalizumab;

- a CXCR4 inhibitor selected from the group consisting of plerixafor, ALT- 1188, vMIP and Haimipu;

- a pharmacokinetic enhancer selected from the group consisting of cobicistat and ritonavir;

- an immunological drug selected from the group consisting of dermaVir, interleukin-7, plaquenil (hydroxychloroquine), proleukin (aldesleukin, IL-2), interferon alpha, interferon alpha-2b, interferon alpha-3b, pegylated interferon alpha, interferon gamma, hydroxyurea, mycophenolic acid (MPA) and its ether derivastive mycophenolate mofetil (MMF), WF-10, ribavirin, IL-2, IL-12, polyethyleneimine polymer (PEI), hepone, VGV-1, MOR-22, BMS-936559, modulators of toll-like receptors (tlrl, tlr2, tlr3, tlr4, tlr5, tlr6, tlr7, tlr8, tlr9, tlrlO, tlrll, tlrl2, and tlrl3), rintatolimod, and IR-103;

- anti-HIV vaccines selected from the goup consisting of peptide vaccines, vaccines based on recombinant subunits,

live vector vaccines, DNA vaccines, vaccines based on virus-like particles (pseudovirion-based vaccines), peptide vaccines based on CD4 derivatives, vaccine combinations, rgpl20 (AIDSVAX), ALVAC HIV (vCP1521)/AIDSVAX IN/E (gpl20) (RV144), Remune, ITV-1, Contre Vir, Ad5-ENVA-48, DCVax-001 (CDX-2401), PEP-6409, Vacc-4x, Vacc-C5, VAC-3 S, a multi-type vaccine based on recombinant DNA adenovirus serotype 5 (rAd5), Pennvax-G, VRC-HIV MAB060-00-AB, AVX-101, vaccines Tat Oyi, AVX-201, HIV-LAMP-vax, Ad35, Ad35- GRIN, and NAcGM3/VSSP ISA-51, poly-ICLC adjuvant vaccines, Tatlmmune, GTU- multiHIV (FIT-06), AGS-004, gpl40[delta]V2.TVI+ MF-59, rVSVIN HIV-1 gag vaccines, SeV-Gag vaccines, AT-20, DNK-4, Ad35-GRIN/ENV, TBC-M4, HIVAX, HIV AX-2, NYVAC-HIV-PT1, NYVAC-HIV-PT4, DNA-HIV-PT123, rAAVI-PG9DP, GOVX-B11, GOVX-B21, ThV-01, TUTI-16, VGX-3300, TVI-HIV-1, Ad-4 (Ad4-env Clade C + Ad4-mGag), EN41-UGR7C, EN41-FPA2, PreVaxTat, TL-01, SAV-001, AE- H, MYM-V101, CombiHIVvac, ADVAX, MYM-V201, MVA-CMDR, ETV-01 and DNA- Ad5 gag/pol/nef/nev (HVTN505);

- an anti-HIV antibody, a bispecific antibody, and "antibody-like" therapeutic proteins (such as DARTs®, Duobodies®, Bites®, XmAbs®, TandAbs®, Fabderivatives) including BMS-936559, TMB-360, and a drug acting on gpl20 or gp41 HIV selected from the group consisting of bavituximabб UB-421, C2F5, C2G12, C4E10, C2F5+C2G12+C4E10, 3-BNC-117, PGT145, PGT121, MDX010 (ipilimumab), VRC01, A32, 7B2, 10E8, and VRC07;

- latency reversing agents selected from the group consisting of histone diacetylase inhibitors, such as romidepsin, vorinostat, panobinostat; proteasome inhibitors such as velcade; protein kinase C (PKC) activators, such as indolactam, prostratin, ingenol B, and DAT lactones, ionomycin, GSK-343, PMA, SAHA, inhibitors BRD4, IL-15, JQ1, disulfiram and amphotericin B;

- an HIV p7 nucleocapsid protein inhibitor (NCp7) selected from the group consisting of azodicarbonamide;

- an HIV maturation inhibitor selected from the group consisting of BMS-955176 and GSK-2838232;

- a PI3K inhibitor selected from the group consisting of idelalisib, AZD-8186, buparlisib, CLR-457, pictilisib, neratinib, rigosertib, sodium rigosertib, EN-3342, TGR-1202, alpelisib, duvelisib, UCB-58, UCB-58, UCB-58 - 765, gedatolisib, VS-5584, copanlisib, CAT orotate, perifosin, RG-7666, GSK-2636771, DS-7423, panulisib, GSK-2269557, GSK-2126458, CUDC-907, PQR-309, INCB-040093, pilaralisib, BAY-1082439, puquitinib mesylate, SAR-245409, AMG-319, RP-6530, ZSTK-474, MLN-1117, SF-1126, RV-1729, sonolisib, LY-3023414, SAR-260301 HCLR-1401;

- compounds disclosed in WO 2004/096286, WO 2006/110157, WO 2006/015261, WO 2013/006738, US 2013/0165489, US 20140221380, US 20140221378, WO 2013/006792, WO 2009/062285, WO 2010/130034, WO 2013/091096A1, WO 2013/159064, WO 2012/145728, WO2012/003497, WO2014/100323, WO2012/145728, WO2013/159064, and WO 2012/003498;

- other anti-HIV drugs selected from the group consisting of BanLec, MK-8507, AG-1105, TR-452, MK-8591, REP 9, CYT-107, alisporivir, NOV-205, IND-02, met-enkephalin, PGN-007, acemannan, gamimune, prolastin, 1,5-dicaffeoylquinic acid, BIT-225, RPI-MN, VSSP, HiViral, SB-728-T, RPI-MN, VIR-576, HGTV-43, MK-1376, rHIV7-shl-TAR-CCR5RZ, MazF-gene therapy, BlockAide, ABX-464, SCY-635, naltrexone, AAV-eCD4-Ig-gene therapy, and PA-1050040 (PA-040).

In some embodiments of the invention, a compound of formula (1) or (2) disclosed herein, or a stereoisomer thereof, or a pharmaceutically acceptable salt thereof, or a solvate thereof, or a crystalline or polycrystalline form thereof, is combined with one, two, three, four, or more additional therapeutic agents.

Said one, two, three, four, or more additional therapeutic agents may be different therapeutic agents selected from one class of therapeutic agents or from different classes of therapeutic agents.

In a specific embodiment of the invention, the compound of formula (1) or (2) disclosed herein, or a stereoisomer thereof, or a pharmaceutically acceptable salt thereof, or a solvate thereof, or a crystalline or polycrystalline form thereof, are combined with an HIV non-nucleoside reverse transcriptase inhibitor.

In a specific embodiment of the invention, the compound of formula (1) or (2) disclosed herein, or a stereoisomer thereof, or a pharmaceutically acceptable salt or solvate thereof is combined with an HIV nucleoside or nucleotide reverse transcriptase inhibitor and an HIV non-nucleoside reverse transcriptase inhibitor.

In another specific embodiment of the invention, the compound of formula (1) or (2) as described herein, or a stereoisomer thereof, or a pharmaceutically acceptable salt thereof, or a solvate thereof, or a crystalline or polycrystalline form thereof, is combined with an HIV nucleoside or nucleotide reverse transcriptase inhibitor and an HIV protease

inhibiting compound.

In another embodiment of the invention, the compound of formula (1) or (2) disclosed herein, or a stereoisomer thereof, or a pharmaceutically acceptable salt thereof, or a solvate thereof, or a crystalline or polycrystalline form thereof is combined with an HIV nucleoside or nucleotide reverse transcriptase inhibitor, an HIV non-nucleoside reverse transcriptase inhibitor, and an HIV protease inhibiting compound.

In another embodiment of the invention, the compound of formula (1) or (2) disclosed herein, or a stereoisomer thereof, or a pharmaceutically acceptable salt thereof, or a solvate thereof, or a crystalline or polycrystalline form thereof, is combined with an HIV nucleoside or nucleotide reverse transcriptase inhibitor, an HIV non-nucleoside reverse transcriptase inhibitor, HIV capsid assembly inhibitors, and a pharmacokinetic enhancer.

In some embodiments of the invention, the compound of formula (1) or (2) disclosed herein, or a stereoisomer thereof, or a pharmaceutically acceptable salt thereof, or a solvate thereof, or a crystalline or polycrystalline form thereof is combined with at least one HIV nucleoside reverse transcriptase inhibitor, an integrase inhibitor, and a pharmacokinetic enhancer.

In another specific embodiment of the invention, the compound of formula (1) or (2) disclosed herein, or a stereoisomer thereof, or a pharmaceutically acceptable salt thereof, or a solvate thereof, or a crystalline or polycrystalline form thereof is combined with two HIV nucleoside or nucleotide reverse transcriptase inhibitors.

In a specific embodiment of the invention, the compound of formula (1) or (2) disclosed herein, or a stereoisomer thereof, or a pharmaceutically acceptable salt thereof, or a solvate thereof, or a crystalline or polycrystalline form thereof is combined with abacavir sulfate, tenofovir, tenofovir disoproxyl, tenofovir disoproxil fumarate, tenofovir disoproxil hemifumarate, tenofovir alafenamide, tenofovir alafenamide hemifumarate, tenofovir alafenamide fumarate, tenofovir cyclobutylalafenamide, tenofovir cyclobutylalafenamide hemifumarate, tenofovir cyclobutylalafenamide fumarate, elsulfavirine, VM-1500A or GS-CA1.

In a specific embodiment of the invention, the compound of formula (1) or (2) as described herein, or a stereoisomer thereof, or a pharmaceutically acceptable salt thereof, or a solvate thereof, or a crystalline or polycrystalline form thereof is combined with a first additional therapeutic agent selected from the group consisting of abacavir sulfate, tenofovir, tenofovir disoproxil, tenofovir disoproxil fumarate, tenofovir disoproxil hemifumarate, tenofovir alafenamide, tenofovir alafenamide hemifumarate, tenofovir alafenamide fumarate, tenofovir cyclobutylalafenamide, tenofovir cyclobutylalafenamide hemifumarate, tenofovir cyclobutylalafenamide fumarate, elsulfavirine, VM-1500A or GS-CA1, and a second additional therapeutic agent selected from the group consisting of emtricidabine and lamivudine.

In a specific embodiment of the invention, the compound of formula (1) or (2) disclosed herein, or a stereoisomer thereof, or a pharmaceutically acceptable salt thereof, or a solvate thereof, or a crystalline or polycrystalline form thereof is combined with a first additional therapeutic agent selected from the group consisting of tenofovir, tenofovir disoproxil, tenofovir disoproxil fumarate, tenofovir disoproxil hemifumarate, tenofovir alafenamide, tenofovir alafenamide hemifumarate, tenofovir alafenamide fumarate, tenofovir cyclobutylalafenamide, tenofovir cyclobutylalafenamide hemifumarate or tenofovir cyclobutylalafenamide fumarate, elsulfavirine, VM-1500A or GS-CA1, and a second additional therapeutic agent, emtricitabine.

In a specific embodiment of the invention, the compound of formula (1) or (2) disclosed herein, or a stereoisomer thereof, or a pharmaceutically acceptable salt thereof, or a solvate thereof, or a crystalline or polycrystalline form thereof, is combined with 5-30 mg of tenofovir, tenofovir disoproxyl , tenofovir disoproxil fumarate, tenofovir disoproxil hemifumarate, tenofovir alafenamide, tenofovir alafenamide hemifumarate, tenofovir alafenamide fumarate, tenofovir cyclobutylalafenamide, tenofovir cyclobutylalafenamide hemifumarate, tenofovir cyclobutylalafenamide fumarate, elsulfavirine, VM-1500A or GS-CA1, and 200 mg of emtricitabine.

In some embodiments of the invention disclosed herein, the compound of formula (1) or (2) or a stereoisomer thereof, or a pharmaceutically acceptable salt thereof, or a solvate thereof, or a crystalline or polycrystalline form thereof is combined with 5-10 mg; 5-15 mg; 5-20 mg; 5-25 mg; 25-30 mg; 20-30 mg; 15-30 mg or 10-30 mg of tenofovir, tenofovir disoproxil, tenofovir disoproxil fumarate, tenofovir disoproxil hemifumarate, tenofovir alafenamide, tenofovir alafenamide hemifumarate, tenofovir alafenamide fumarate, tenofovir cyclobutylalafenamide, tenofovir cyclobutylalafenamide hemifumarate, tenofovir cyclobutylalafenamide fumarate, elsulfavirine, VM-1500A or GS-CA1, and 200 mg of emtricitabine.

In a specific embodiment of the invention disclosed herein, the compound of formula (1) or (2) or a stereoisomer thereof, or a pharmaceutically acceptable salt thereof, or a solvate thereof, or a crystalline or polycrystalline form thereof is combined with 10 mg of tenofovir, tenofovir disoproxil, tenofovir disoproxil fumarate, tenofovir disoproxil hemifumarate, tenofovir alafenamide, tenofovir alafenamide hemifumarate, tenofovir alafenamide fumarate, tenofovir cyclobutylalafenamide, tenofovir cyclobutylalafenamide hemifumarate, tenofovir cyclobutylalafenamide fumarate, elsulfavirine, VM-1500A or GS-CA1, and 200 mg of emtricitabine.

In a specific embodiment of the invention, disclosed herein, the compound of formula (1) or (2) or a stereoisomer thereof, or a pharmaceutically acceptable salt thereof, or a solvate thereof, or a crystalline or polycrystalline form thereof is combined with 25 mg of tenofovir, tenofovir disoproxil, tenofovir disoproxil fumarate, tenofovir disoproxil hemifumarate, tenofovir alafenamide, tenofovir alafenamide hemifumarate, tenofovir alafenamide fumarate, tenofovir cyclobutylalafenamide, tenofovir cyclobutylalafenamide hemifumarate, tenofovir cyclobutylalafenamide fumarate, elsulfavirine, VM-1500A or GS-CA1, and 200 mg of emtricitabine.

In the above embodiments, the additional therapeutic agent may be the above anti-HBV and/or anti-HCV agent.

In some embodiments of the invention disclosed herein, the compound of formula (1) or (2) or a stereoisomer thereof, or a pharmaceutically acceptable salt thereof, or a solvate thereof, or a crystalline or polycrystalline form thereof, or a pharmaceutical composition is combined with one or more additional therapeutic agents as described above, said composition components being adminuistered simultaneously or sequentially. Sequential administration of said combination may involve two or more administrations.

In some embodiments of the invention disclosed herein, the compound of formula (1) or (2) or a stereoisomer thereof, or a pharmaceutically acceptable salt thereof, or a solvate thereof, or a crystalline or polycrystalline form thereof is combined with one or more additional therapeutic agents in a single dosage form to be simultaneously administered to a patient in, for example, a solid dosage form for oral administration of fixed-dose components.

In some embodiments of the invention disclosed herein, the compound of formula (1) or (2) or a stereoisomer thereof, or a pharmaceutically acceptable salt thereof, or a solvate thereof, or a crystalline or polycrystalline form thereof is administered with one or more additional therapeutic agents.

Coadministration of the compound disclosed herein and one or more additional therapeutic agents generally refers to a simultaneous or sequential administration of the compound disclosed herein and one or more additional therapeutic agents to ensure the presence of therapeutically effective amounts of the compound disclosed herein and therapeutically effective amounts of one or more additional therapeutic agents in the patient's body.

Coadministration involves administering unit doses of the compounds disclosed herein before or after administering unit doses of one or more additional therapeutic agents, for example, the administration of the compound disclosed herein within seconds, minutes, or hours before or after administration of one or more additional therapeutic agents. For example, in some embodiments, a unit dose of the compound disclosed herein is administered first followed by a unit dose of one or more additional therapeutic agents within seconds or minutes.

Alternatively, other embodiments involve administering first a unit dose of one or more additional therapeutic agents followed by a unit dose of the compound disclosed herein within seconds or minutes. In some embodiments of the invention, a unit dose of the compound disclosed herein is administered first followed by administering a unit dose of one or more additional therapeutic agents after a few hours (for example, 1-12 hours later).

In other embodiments, a unit dose of one or more additional therapeutic agents is administered first, followed by administering a unit dose of the compound disclosed herein after a few hours (for example, 1-12 hours later).

In another embodiment of the invention, a compound of formula (1) or (2) or a stereoisomer thereof, or a pharmaceutically acceptable salt thereof, or a solvate thereof, or a crystalline or polycrystalline form thereof, or a pharmaceutical composition disclosed herein are proposed for use in HIV therapy (as a method of treatment) in an HIV-infected or HIV-exposed individual.

In another embodiment of the invention, a compound of formula (1) or (2) or a stereoisomer thereof, or a pharmaceutically acceptable salt thereof, or a solvate thereof, or a crystalline or polycrystalline form thereof or a pharmaceutical composition disclosed herein are provided for use in the treatment of infection HIV in a person having a specified infection or having a risk of acquiring a specified infection, said therapy further involving the administration to said individual of one or more additional therapeutic agents.

In another embodiment of the invention, the compound of formula (1) or (2) or a stereoisomer thereof, or a new pharmaceutically acceptable salt thereof, or a solvate thereof, or a crystalline or polycrystalline form thereof, or a pharmaceutical composition disclosed herein is proposed for using in the treatment of HIV infection in an HIV-infected or HIV-exposed individual, wherein said treatment further comprises administering to said individual one or more additional therapeutic agents selected from the group consisting of HIV protease inhibitors, HIV non-nucleoside reverse transcriptase inhibitors, HIV nucleoside or nucleotide reverse transcriptase inhibitors, allosteric HIV integrase inhibitors, HIV capsid assembly inhibitors, and combinations thereof.

In another embodiment of the invention, the compound of formula (1) or (2) or a stereoisomer thereof, or a pharmaceutically acceptable salt thereof, or a solvate thereof, or a crystalline or polycrystalline form thereof, or a pharmaceutical composition disclosed herein is proposed for using in the treatment of HIV infection in an HIV-infected or HIV-exposed individual, wherein said treatment further comprises administering to said individual a single dosage form for simultaneous administration to a patient for example, in the form of a solid dosage form for oral administration, a compound of formula (1) or (2) or their stereoisomer, or their pharmaceutically acceptable salt, or their solvate, or their crystalline or polycrystalline form, or the pharmaceutical composition disclosed herein in combination with a first additional therapeutic agent selected from ther group consisting of abacavir sulfate, tenofovir, tenofovir disoproxil, tenofovir disoproxil fumarate, tenofovir disoproxil hemifumarate, tenofovir alafenamide, tenofovir alafenamide hemifumarate, tenofovir cyclobutylalafenamide, tenofovir cyclobutylalafenamide hemifumarate, tenofovir cyclobutylalafenamide fumarate, elsulfavirine, VM-1500A or GS-CA1 and a a second additional therapeutic agent selected from the group consisting of emtricitabine and lamivudine.

The pharmaceutical compositions of the embodiments disclosed herein can be prepared by combining a compound of formula (1) or (2) or a stereoisomer thereof, or a pharmaceutically acceptable salt thereof, or a solvate thereof with an appropriate pharmaceutically acceptable excipient to yield drugs in solid, semi-solid, liquid, or gaseous forms such as tablets, capsules, powders, granules, ointments, solutions, nanosuspensions, suppositories, injections, inhalants, gels, microspheres, and aerosols. Typical routes of administration of said pharmaceutical compositions include, but are not limited to, oral, topical, transdermal, inhalation, parenteral, sublingual, buccal, rectal, vaginal, and intranasal ones.

Pharmaceutical compositions according to embodiments as disclosed herein are formulated so as to provide bioavailability of the active ingredients within the composition to be administered to a subject. Said compositions are administered to a subject or a patient in dosage forms containing one or more dosage units, where, for example, a tablet may be a dosage form containing one dosage unit and a container with a compound according to embodiments as disclosed herein, in the form of an aerosol may contain many dosage units. Actual methods for preparing said dosage forms are known or will be apparent to those skilled in the art; see, for example, Remington. The Science and Practice of Pharmacy, 20th Edition (Philadelphia College of Pharmacy and Science, 2000). The composition to be administered will in any case comprise a therapeutically effective amount of a compound according to embodiments as disclosed herein for treating a disease or condition of interest in accordance with the recommendations of the description herein.

The pharmaceutical compositions disclosed herein can be prepared by methods well known in the pharmaceutical field. For example, a pharmaceutical composition intended for administration by injection can be prepared by combining the compound of the embodiments disclosed herein with sterile distilled water to form a solution or nanosuspension. To ensure homogeneity of the solution or nanosuspension, a surfactant may be added. Surfactants are compounds that non-covalently interact with a compound according to embodiments as disclosed herein and facilitate dissolution thereof or homogeneity of the nanosuspension of the compound in an aqueous delivery system.

The subject of this invention is a method for the prevention and treatment of HIV infection in an HIV-infected or HIV-exposed individual by administering to said individual a therapeutically effective amount of a compound of general formula 1 or 2, or a pharmaceutical composition containing a compound of general formula 1 or 2.

The subject of this invention is a method for the prevention and treatment of HIV infection in an HIV-infected or HIV-exposed individual by orally administering to said individual a therapeutically effective amount of a compound of general formula 1 or 2 or a pharmaceutical composition containing a compound of general formula 1 or 2.

The subject of this invention is a method for the prevention and treatment of HIV infection in an HIV-infected or HIV-exposed individual by injecting to said individual a therapeutically effective amount of a nanosuspension containing a compound of general formula (1) or a compound of general formula (2) or a stereoisomer thereof.

The invention is illustrated by the following drawings:

Figure 1. Simulation of an X-ray diffraction pattern for a (3S,11 aR)-6-hydroxy-N-[(2,6-difluoro-3-pyridyl)methyl]-3-methyl-5,7-dioxo-2,3,5,7,11,11a-hexahydro[1,3]oxazole[3,2-a]pyridori,2-d]pyrazine-8-carboxamide (1.1.19) sample using the Pauli technique. The blue line indicates an experimental XRD pattern; the red line, a calculated one; and the gray line, a difference curve. The vertical lines denote peaks due to impuritues.

Figure 2a. Stability of compounds of general formula 1.1 in human plasma.

Figure 2b. Stability of compounds of general formula 1.1 rat plasma.

Figure 3a. Stability of compounds of general formula 1.1 in human liver S9 fraction.

Figure 3b. Stability of compounds of general formula 1.1 in rat liver S9 fraction.

**Best embodiment**

The following examples explain the present invention but are not construed as limiting.

**Example 1. Physicochemical studies of compounds of general formula 1 or 2.**

***General chemical procedures.*** All chemicals and solvents were used as **supplied** without further purification. The crude reaction mixtures were concentrated under reduced pressure by removing organic solvents on a rotary evaporator.

*Nuclear Magnetic Resonance Spectra* ***(NMR)*** were recorded using a Broker DPX-400 spectrometer at room temperature (rt) with tetramethylsilane employed as an internal standard. Chemical shifts ($\delta$) are given in parts per million (ppm), and the signals are presented as s (singlet), d (doublet), t (triplet), q (quartet), m (multiplet) or brs (broad singlet).

*High resolution mass spectra* ***(HRMS)*** **were obtained using** an Orbitrap Elite mass spectrometer (Thermo, Bremen, Germany) equipped with a HESI ion source.

*High performance liquid chromatography* ***(HPLC).*** The purity of the final compounds was determined using HPLC and amounted to more than 98%. The HPLC conditions for purity assessment were as follows: Shimadzu HPLC, XBridge C18, 4.6 mm x 250 mm (3.5 μm); a gradient of 0.1% TFA in 5% acetonitrile /water (A) and 0.1% TFA in acetonitrile (B); flow rate 0.5 ml/min; collection time 20 min; and UV wavelengths 214 and 254 nm. The preparative HPLC system included two sets of Shimadzu LC-8A pumps, a Shimadzu SCL 10Avp controller, and a Shimadzu SPD 10Avp detector. A Reprosil-Pur C18-AQ column of 10 μm, 250 mm x 20 mm was used. The mobile phase had a gradient of 0.1% TFA in water (A) and 0.1% TFA in acetonitrile (B). LC/MS **(LC/MS)** was performed on a **PE Sciex API 165 system using positive ion electrospray [M + H]+ and a Shimadzu HPLC system equipped with a Waters XBridge C18 3.5** μm **column (4.6 mm × 150 mm).**

The diastereoisomers were separated on chiral HPLC Phenomenex Lux 5u Cellulose-4, AXIA F, 250 x 30.00 mm (flow rate: 25 ml/min; UV detector at 215 nm).

*X-ray phase analysis*. X-ray diffraction patterns were obtained on a Bruker D8 Advance Vario diffractometer equipped with an X-ray tube with a copper anode and a Ge(lll) monochromator (CuK$a_1$ and a position-sensitive LynxEye detector in the transmission settings. The shooting angle range was 2-60° $2\theta$ and the shooting step, 0.02° $2\theta$. The analysis was performed using the Bruker Topas 5 software [Bruker TOPAS5 User Manual. - Karlsruhe, Germany: Bruker AXS GmbH, 2015].

**Example 2. General synthetic procedure for annelated 9-hydroxy-1,8-dioxo-1,3,4,8-tetrahydro-2H-pyrido[1,2-a]pyrazine-7-carboxamides (general formula 1 or 2) according to Scheme 1.**

Heterocyclylmethylamine or its hydrochloride (0.75 mmol), diisopropylamine (0.131 ml, 0.75 mmol; plus 0.75 mmol per each hydrochloride), and $Pd(PPh)_4$ (29 mg, 0.025 mmol) were added to a solution of corresponding annelated 9-benzyloxy-7-bromo-1,8-dioxo-1,3,4,8-tetrahydro-2H-pyrido[1,2-a]pyrazine (0.5 mmol) of formula 3 or 4 in DMSO (2 ml ). The reaction mass was stirred for 14 h at 90°C under CO. Upon completion of the reaction LC-MS control), the reaction mass was evaporated in vacuum. The residue was dissolved in dichloromethane, washed with water, dried over sodium sulfate, evaporated on a rotary evaporator and subjected to column chromatography on silica gel to yield the corresponding annelated 9-benzyloxy-1,8-dioxo-1,3,4,8-tetrahydro-2H-pyrido[1,2-a]pyrazine-7-carboxamide (formula 6 or 7).

a) The resulting compound of formula 6 or 7 (0.35 mmol) was dissolved in a mixture of THF (18 ml) and methanol (2 ml), then 10% Pd/C (0.04 g) was added and the mixture was stirred in a hydrogen atmosphere for 8 h. The reaction mass was passed through celite, and the filtrate was evaporated. The residue was treated with ether, the precipitate was filtered off and dried in vacuum to yield the corresponding annelated 9-hydroxy-1,8-dioxo-1,3,4,8-tetrahydro-2H-pyrido[1,2-a]pyrazine-7-carboxamide (general formula 1 or 2).

b) The compound of formula 6 or 7 (0.35 mmol) was dissolved in 2 ml of trifluoroacetic acid and stirred for 2 h at room temperature. The solution was evaporated in vacuum, the residue was dissolved in chloroform, washed with a saturated $NaHCO_3$ solution, dried over $Na_2SO_4$ and evaporated in vacuum.

Остаток обрабатывали эфиром, осадок отфильтровывали , dried in vacuum to yield the corresponding annelated 9-hydroxy-1,8-dioxo-1,3,4,8-tetrahydro-2H-pyrido[1,2-a]pyrazine-7-carboxamide (general formula 1 or 2).

c) The compound of formula 6 or 7 (0.35 mmol) was dissolved in 1.5 ml of *N,N*-dimethylacetamide, then 0.148 g (3.5 mmol) of LiCl was added, and the mixture was stirred for 3 h at 80°C. Upon completion of the reaction (LC-MS control), the reaction mass was evaporated in vacuum. The product was isolated by HPLC to yield the corresponding annelated 9-hydroxy-1,8-dioxo-1,3,4,8-tetrahydro-2H-pyrido[1,2-a]pyrazine-7-carboxamide (general formula 1 or 2) including

*(3S,11aR)-6-hydroxy 3-methyl-5,7-dioxo-N-(2-thienylmethyl)-2,3,5,7,* 11,*11a-hexahydro[1,3]oxazolo[3,2-a]pyrido[1,2-d]pyrazine-8-carboxamide* (1.1.1): LC-MS (M+1) = 376; $^1$H NMR (DMSO-$d_6$, 400 MHz) δ 11.47 (brs, 1H), 10.33 (t, *J* = 4.8 Hz, 1H), 8.49 (s, 1H), 7.40 (dd, $J_1$ = 8.8 Hz, $J_2$ = 4.8 Hz, 1H), 7.03 (d, *J* = 2.8 Hz, 1H), 6.96 (dd, $J_1$ = 4.8 Hz, $J_2$ = 3.6 Hz, 1H), 5.39 (dd, $J_1$ = 10.0 Hz, $J_2$ = 4.0 Hz, 1H), 4.90 (dd, $J_1$ = 12.0 Hz, $J_2$ = *4.0* Hz, 1H), 4.70 (d, *J* = 5.6 Hz, 2H), 4.40 (dd, $J_1$ = 8.4 Hz, $J_2$ = 7.2 Hz, 1H), 4.29 (m, 1H), 4.01 (t, *J* = 11.2 Hz, 1H), 3.67 (dd, $J_1$ = 8.4 Hz, $J_2$ = 7.2 Hz, 1H), 1.34 (d, *J* = 6.0 Hz, 3H);

*(3S, 11aR)-6-hydroxy-3-methyl-N-r(5-methyl-2-furyl)methyl]-5,7-dioxo-2,3,5,7,11,11a-hexahydro[1,3]oxazolo[3,2-a]pyrido[1,2-d]pyrazine-8-carboxamide (1.1.3):* LC-MS (ESI) 374 $(M+H)^+$; $^1H$ NMR (DMSO-$d_6$, 400 MHz) δ 11.46 (brs, 1H), 10.20 (t, *J* = 5.2 Hz, 1H), 8.46 (s, 1H), 6.15 (d, *J* = 2.4 Hz, 1H), 5.99 (d, *J* = 2.4 Hz, 1H), 5.39 (dd, $J_1$ = 9.6 Hz, $J_2$ = 4.0 Hz, 1H), 4.89 (dd, $J_1$ = 12.0 Hz, $J_2$ = 4.0 Hz, 1H), 4.46 (d, *J* = 5.2 Hz, 2H), 4.39 (dd, $J_1$ = 8.4 Hz, $J_2$ = 7.2 Hz, 1H), 4.29 (m, 1H), 4.00 (t, *J* = 11.0 Hz, 1H), 3.66 (dd, $J_1$ = 8.4 Hz, $J_2$ = 7.2 Hz, 1H), 2.23 (s, 3H), 1.34 (d, *J* = 6.0 Hz, 3H);

*(3S,11aR)-6-hydroxy-N-[(3,5-dimethyl-1H-pyrazol-4-yl)methyl]-3-methyl-5,7-dioxo-*2,3,5,7,*11,11a-hexahydro[1,3]oxazolo[3,2-a]pyrido[1,2-d]pyrazine-8-carboxamide (1.1.4):* LC-MS (ESI) 388 $(M+H)^+$; $^1H$ NMR (DMSO-$d_6$, 400 MHz) δ 12.04 (brs, 1H), 11.42 (brs, 1H), 9.92 (t, *J* = 4.8 Hz, 1H), 8.46 (s, 1H), 5.38 (dd, $J_1$ = 10.0 Hz, $J_2$ = 4.0 Hz, 1H), 4.88 (dd, $J_1$ = 12.0 Hz, $J_2$ = 4.0 Hz, 1H), 4.38 (dd, $J_1$ = 8.0 Hz, $J_2$ = 7.2 Hz, 1H), 4.29 (m, 1H), 4.26 (d, *J* = 5.2 Hz, 2H), 4.00 (t, *J* = 11.2 Hz, 1H), 3.66 (dd, $J_1$ = 8.0 Hz, $J_2$ = 7.2 Hz, 1H), 1.33 (d, *J*= 6.4 Hz, 3H);

*(3S,11aR)-6-hydroxy-3-methyl-N-[9(1,3,5-trimethyl-1H-pyrazol-4-yl)methyl]-5,7-dioxo-2,3,5,7,11,11a-hexahvdro[1,3]oxazolo[3,2-a]pyrido[1,2-d]pyrazine-8-carboxamide (1.1.5):* LC-MS (ESI) 402 $(M+H)^+$; $^1H$ NMR (DMSO-$d_6$, 400 MHz) δ 11.40 (brs, 1H), 9.92 (t, *J* = 4.8 Hz, 1H), 8.46 (s, 1H), 5.38 (dd, $J_1$ = 9.6 Hz, $J_2$ = 3.6 Hz, 1H), 4.88 (dd, $J_1$ = 12.0 Hz, $J_2$ = 3.6 Hz, 1H), 4.39 (t, *J* = 7.4 Hz, 1H), 4.29 (m, 1H), 4.25 (d, *J* = 4.8 Hz, 2H), 4.00 (t, *J* = 11.0 Hz, 1H), 3.66 (t, *J*= 7.4 Hz, 1H), 3.32 (s, 3H), 2.19 (s, 3H), 2.08 (s, 3H), 1.33 (d, *J* = 6.0 Hz, 3H);

*(3S,11aR)-6-hydroxy-N-[(1-ethyl-3,5-dimethyl-1H-pyrazol-4-yl)methyl]3-methyl-5, 7-dioxo-2,3,5,7,11,11a-hexahydro[1,3]oxazolo[3,2-a]pyrido[1,2-d]pyrazine-8-carboxamide (1.1.6):* LC-MS (ESI) 416 $(M+H)^+$; $^1H$ NMR (DMSO-$d_6$, 400 MHz) δ 11.40 (brs, 1H), 9.92 (t, *J* = 5.2 Hz, 1H), 8.47 (s, 1H), 5.38 (dd, $J_1$ = 10.0 Hz, $J_2$ = 4.0 Hz, 1H), 4.88 (dd, $J_1$ = 12.0 Hz, $J_2$ = 4.0 Hz, 1H), 4.39 (dd, $J_1$ = 8.4 Hz, $J_2$ = 6.8 Hz, 1H), 4.28 (m, 1H), 4.25 (d, *J* = 5.6 Hz, 2H), 4.00 (t, *J* = 11.2 Hz, 1H), 3.94 (q, *J* = 7.2 Hz, 2H), 3.66 (dd, $J_1$ = 8.4 Hz, $J_2$ = 6.4 Hz, 1H), 2.20 (s, 3H), 2.09 (s, 3H), 1.33 (d, *J* = 6.0 Hz, 3H), 1.24 (t, *J*= 7.2 Hz, 3H);

*(3S,11aR)-6-hydroxy-N-[(4,5-∂uchloro-1-methyl-1H-pyrazol-3-yl)methyl]3-methyl-5,7-dioxo-2,3,5,7,11,11a-hexahydro[1,3]oxazolo[3,2-a]pyrido[1,2-d]pyrazine-8-carboxamide* (1.1.7): LC-MS (ESI) 443 $(M+H)^+$; $^1H$ NMR (DMSO-$d_6$, 400 MHz) δ 11.44 (brs, 1H), 10.28 (t, *J*= 5.6 Hz, 1H), 8.46 (s, 1H), 5.39 (dd, $J_1$ = 10.0 Hz, $J_2$ = 3.6 Hz, 1H), 4.89 (dd, $J_1$ = 12.0 Hz, $J_2$ = 4.0 Hz, 1H), 4.50 (d, *J* = 5.6 Hz, 2H), 4.39 (dd, $J_1$ = 8.4 Hz, $J_2$ = 6.8 Hz, 1H), 4.29 (sxt, *J* = 6.4 Hz, 1H), 4.01 (t, *J* = 11.2 Hz, 1H), 3.80 (s, 3H), 3.66 (dd, $J_1$ = 8.4 Hz, $J_2$ = 6.8 Hz, 1H), 1.34 (d, *J*= 6.0 Hz, 3H);

(*3S,11aR)-6-hydroxy-3-methyl-N-(thiazol-2-ylmethyl)-5,7-dioxo-2,3,5,7,11,11a-hexahydro[1,3]oxazolo[3,2-a]pyrido[1,2-d]pyrazine-8-carboxamide (1.*1.8): LC-MS (ESI) 377 $(M+H)^+$; $^1H$ NMR (DMSO-$d_6$, 400 MHz) δ 11.50 (brs, 1H), 10.58 (t, *J* = 6.0 Hz, 1H), 8.49 (s, 1H), 7.74 (d, *J* = *3.2* Hz, 1H), 7.62 (d, *J* = 3.2 Hz, 1H), 5.40 (dd, $J_1$ = 10.0 Hz, $J_2$ = 4.0 Hz, 1H), 4.90 (dd, $J_1$ = 12.0 Hz, $J_2$ = 4.0 Hz, 1H), 4.84 (d, *J* = 6.0 Hz, 2H), 4.40 (dd, $J_1$ = 8.4 Hz, $J_2$ = 7.2 Hz, 1H), 4.30 (m, 1H), 4.01 (dd, $J_1$ = 12.0 Hz, $J_2$ = 10.0 Hz, 1H), 3.67 (dd, $J_1$ = 8.4 Hz, $J_2$ = 6.4 Hz, 1H), 1.35 (d, *J* = 6.4 Hz, 3H);

*(3S,11aR)-6-hydroxy-3-methyl-N-[(4-methyl-1,2,5-oxadiazol-3-yl)methyl]-5,7-dioxo-2,3,5,7,11,11a-hexahydro[1,3]oxazolo[3,2-a]pyrido[1,2-d]pyrazine-8-carboxamide (1.1.12):* LC-MS (ESI) 376 $(M+H)^+$; $^1H$ NMR (DMSO-$d_6$, 400 MHz) δ 11.51 (brs, 1H), 10.45 (t, *J* = 5.6 Hz, 1H), 8.47 (s, 1H), 5.39 (dd, $J_1$ = 10.0 Hz, $J_2$ = 4.0 Hz, 1H), 4.89 (dd, $J_1$ = 12.0 Hz, $J_2$ = 4.0 Hz, 1H), 4.74 (d, *J* = 5.6 Hz, 2H), 4.40 (dd, $J_1$ = 8.4 Hz, $J_2$ = 7.2 Hz, 1H), 4.29 (m, 1H), 4.00 (t, *J* = 11.0 Hz, 1H), 3.67 (dd, $J_1$ = 8.0 Hz, $J_2$ = 7.2 Hz, 1H), 2.37 (s, 3H), 1.34 (d, *J* = 6.0 Hz, 3H);

*(3S,11aR)-6-hydroxy-3-methyl-N-[(4-methyl-4H-1,2,4-triazol-3-yl)methyl]-5,7-dioxo-2,3,5,7,11,11a-hexahydro[1,3]oxazolo[3,2-a]pyrido[1,2-d]pyrazine-8-carboxamide (1.1.14):* LC-MS (ESI) 375 $(M+H)^+$; $^1H$ NMR (DMSO-$d_6$, 400 MHz) δ 11.49 (brs, 1H), 10.41 (t, *J* = 5.2 Hz, 1H), 8.48 (s, 1H), 8.40 (s, 1H), 5.39 (dd, $J_1$ = 10.0 Hz, $J_2$ = 3.6 Hz, 1H), 4.89 (dd, $J_1$ = 11.6 Hz, $J_2$ = 3.6 Hz, 1H), 4.69 (d, *J* = 5.2 Hz, 2H), 4.39 (t, *J* = 7.6 Hz, 1H), 4.29 (m, 1H), 4.01 (t, *J* = 11.2 Hz, 1H), 3.66 (m, 1H), 3.65 (s, 3H), 1.34 (d, *J* = 6.0 Hz, 3H);

*(3S,11aR)-6-hydroxy-3-methyl-N-(pyridyl-2-ylmethyl)-5,7-dioxo-2,3,5,7,11,11a-hexahydro[1,3]oxazolo[3,2-a]pyrido[1,2-d]pyrazine-8-carboxamide (1.1.15):* LC-MS (ESI) 371 $(M+H)^+$; $^1H$ NMR (DMSO-$d_6$, 400 MHz) δ 11.44 (brs, 1H), 10.52 (t, *J*= 5.6 Hz, 1H), 8.53 (d, *J*= 4.0 Hz, 1H), 8.48 (s, 1H), 7.76 (t, *J*= 7.6 Hz, 1H), 7.33 (d, *J* = 8.0 Hz, 1H), 7.28 (m, 1H), 5.39 (dd, $J_1$ = 10.0 Hz, $J_2$ = 3.6 Hz, 1H), 4.89 (dd, $J_1$ = 12.0 Hz, $J_2$ = 3.6 Hz, 1H), 4.64 (d, *J*= 5.6 Hz, 2H), 4.40 (t, *J* = 7.6 Hz, 1H), 4.30 (m, 1H), 4.01 (t, *J*= 11.2 Hz, 1H), 3.66 (dd, $J_1$ = 8.0 Hz,

$J_2$ = 6.8 Hz, 1H), 1.34 (d, $J$ = 6.0 Hz, 3H);

*(3S,11aR)-6-hydroxy-3-methyl-N-[(2-methylpyrimidin-4-yl)methyl]-5,7-dioxo-2,3,5,7,11,11a-hexahydro[1,3]oxazolo[3,2-a]pyrido[1,2-d]pyrazine-8-carboxamide (1.1.26):* LC-MS (M+1) = 386; $^1$H NMR (DMSO-$d_6$, 400 MHz) δ 11.50 (brs, 1H), 10.50 (t, $J$= 6.0 Hz, 1H), 8.61 (d, $J$= 5.0 Hz, 1H), 8.46 (s, 1H), 7.16 (d, $J$ = 5.0 Hz, 1H), 5.40 (dd, $J_1$ = 10.0 Hz, $J_2$ = 3.2 Hz, 1H), 4.89 (dd, $J_1$ = 12.0 Hz, $J_2$ = 3.2 Hz, 1H), 4.59 (d, $J$ = 6.0 Hz, 2H), 4.40 (dd, $J_1$ = 8.4 Hz, $J_2$ = 6.8 Hz, 1H), 4.30 (m, 1H), 4.01 (t, $J$ = 11.2 Hz, 1H), 3.67 (dd, $J_1$ = 8.4 Hz, $J_2$ = 6.4 Hz, 1H), 2.60 (s, 3H), 1.35 (d, $J$ = 6.0 Hz, 3H);

*(3S,11aR)-N-(1-benzothien-5-ylmethyl)-6-hydroxy-3-methyl-5,7-dioxo-2,3,5,7,11,11a-hexahydro[1,3]oxazolo[3,2-a]pyrido[1,2-d]pyrazine-8-carboxamide (1.1.31):* LC-MS (ESI) 426 (M+H)$^+$; $^1$H NMR (DMSO-$d_6$, 400 MHz) δ 11.47 (brs, 1H), 10.38 (t, $J$ = 6.0 Hz, 1H), 8.50 (s, 1H), 7.96 (d, $J$ = 8.4 Hz, 1H), 7.80 (s, 1H), 7.76 (d, $J$ = 9.4 Hz, 1H), 7.43 (d, $J$ = 9.4 Hz, 1H), 7.33 (dd, $J_1$ = 8.4 Hz, $J_2$ = 0.6 Hz, 1H), 5.39 (dd, $J_1$ = 10.0 Hz, $J_2$ = 4.0 Hz, 1H), 4.90 (dd, $J_1$ = 12.0 Hz, $J_2$ = 4.0 Hz, 1H), 4.66 (d, $J$ = 5.6 Hz, 2H), 4.40 (dd, $J_1$ = 8.4 Hz, $J_2$ = 6.8 Hz, 1H), 4.30 (m, 1H), 4.01 (t, $J$ = 11.2 Hz, 1H), 3.67 (dd, $J_1$ = 8.4 Hz, $J_2$ = 6.8 Hz, 1H), 1.34 (d, $J$ = 6.0 Hz, 3H).

*(3S,11aR)-N-(1-benzofuran-2-ylmethyl)-6-hydroxy-3-methyl-5,7-dioxo-2,3,5,7,11,11a-hexahydro[1,3]oxazolo[3,2-a]pyrido[1,2-d]pyrazine-8-carboxamide (1.1.32):* LC-MS (ESI) 410 (M+H)$^+$; $^1$H NMR (DMSO-$d_6$, 400 MHz) δ 11.50 (brs, 1H), 10.41 (t, $J$ = 5.6 Hz, 1H), 8.50 (s, 1H), 7.59 (d, $J$ = 7.6 Hz, 1H), 7.54 (d, $J$ = 7.6 Hz, 1H), 7.27 (t, $J$ = 7.6 Hz, 1H), 7.22 (t, $J$ = 7.2 Hz, 1H), 6.75 (s, 1H), 5.40 (dd, $J_1$ = 10.0 Hz, $J_2$ = 3.6 Hz, 1H), 4.91 (dd, $J_1$ = 12.0 Hz, $J_2$ = 3.6 Hz, 1H), 4.72 (d, $J$ = 5.6 Hz, 2H), 4.40 (t, $J$ = 7.6 Hz, 1H), 4.28 (sxt, $J$ = 6.0 Hz, 1H), 4.02 (t, $J$ = 11.0 Hz, 1H), 3.67 (t, $J$ = 7.6 Hz, 1H), 1.34 (d, $J$ = 6.0 Hz, 3H).

*(3S,11aR)-6-hydroxy-3-methyl-N-[(1-methyl-1H-indol-5-yl)methyl]-5,7-dioxo-2,3,5,7,11,11a-hexahydro[1,3]oxazolo[3,2-a]pyrido[1,2-d]pyrazine-8-carboxamide (1.1.33):* LC-MS (ESI) 423 (M+H)$^+$; $^1$H NMR (DMSO-$d_6$, 400 MHz) δ 11.43 (brs, 1H), 10.27 (t, $J$ = 6.0 Hz, 1H), 8.50 (s, 1H), 7.48 (s, 1H), 7.39 (d, $J$ = 8.8 Hz, 1H), 7.30 (d, $J$ = 2.8 Hz, 1H), 7.12 (dd, $J_1$ = 8.8 Hz, $J_2$ = 0.8 Hz, 1H), 6.37 (dd, $J_1$ = 2.8 Hz, $J_2$ = 0.4 Hz, 1H), 5.39 (dd, $J_1$ = 10.0 Hz, $J_2$ = 4.0 Hz, 1H), 4.90 (dd, $J_1$ = 12.0 Hz, $J_2$ = 4.0 Hz, 1H), 4.59 (d, $J$ = 5.6 Hz, 2H), 4.39 (dd, $J_1$ = 8.4 Hz, $J_2$ = 7.2 Hz, 1H), 4.29 (m, 1H), 4.01 (t, $J$ = 11.2 Hz, 1H), 3.77 (s, 3H), 3.66 (dd, $J_1$ = 8.4 Hz, $J_2$ = 6.8 Hz, 1H), 1.34 (d, $J$ = 6.0 Hz, 3H).

*(3S,11aR)-N-(1,3-benzodioxol-5-ylmethyl)-6-hydroxy-3-methyl-5,7-dioxo-2,3,5,7,11,11a-hexahydro[1,3]oxazolo[3,2-a]pyrido[1,2-d]pyrazine-8-carboxamide (1.1.34):* LC-MS (ESI) 414 (M+H)$^+$; $^1$H NMR (DMSO-$d_6$, 400 MHz) δ 11.45 (brs, 1H), 10.24 (t, $J$ = 5.8 Hz, 1H), 8.48 (s, 1H), 6.86 (m, 2H), 6.79 (m, 1H), 5.98 (s, 2H), 5.39 (dd, $J_1$ = 10.0 Hz, $J_2$ = 4.0 Hz, 1H), 4.89 (dd, $J_1$ = 12.4 Hz, $J_2$ = 4.0 Hz, 1H), 4.43 (d, $J$ = 6.0 Hz, 2H), 4.40 (dd, $J_1$ = 8.4 Hz, $J_2$ = 7.2 Hz, 1H), 4.29 (m, 1H), 4.01 (t, $J$ = 11.2 Hz, 1H), 3.67 (dd, $J_1$ = 8.4 Hz, $J_2$ = 6.0 Hz, 1H), 1.34 (d, $J$ = 6.4 Hz, 3H).

*(3S,11aR)-N-[(6-bromo-1,3-benzodioxol-5yl)methyl]-6-hydroxy-3-methyl-5,7-dioxo-*2,3,5,7,*11,11a-hexahydro[1,3]oxazolo[3,2-a]pyrido[1,2-d]pyrazine-8-carboxamide (1.1.35):* LC-MS (ESI) 493 (M+H)$^+$; $^1$H NMR (DMSO-$d_6$, 400 MHz) δ 11.44 (brs, 1H), 10.30 (m, 1H), 8.46 (d, $J$ = 2.4 Hz, 1H), 7.23 (d, $J$ = 2.4 Hz, 1H), 6.93 (d, $J$ = 2.4 Hz, 1H), 6.05 (d, $J$ = 2.4 Hz, 2H), 5.38 (m, 1H), 4.88 (m, 1H), 4.46 (m, 2H), 4.39 (m, 1H), 4.29 (m, 1H), 4.00 (m, 1H), 3.66 (m, 1H), 1.34 (dd, $J_1$ = 6.4 Hz, $J_2$ = 2.8 Hz, 3H).

*(3S,11aR)-6-hydroxy-3-methyl-N-[(7-methyl-2,3-dihydro-1,4-benzodioxin-6-yl)methyl]-5,7-dioxo-2,3,5,7,11,11a-hexahydro[1,3]oxazolo[3,2-a]pyrido[1,2-d]pyrazine-8-carboxamide (1.1.36):* LC-MS (ESI) 434 (M+H)$^+$; $^1$H NMR (DMSO-$d_6$, 400 MHz) δ 11.43 (brs, 1H), 10.14 (t, $J$ = 5.6 Hz, 1H), 8.47 (s, 1H), 6.73 (s, 1H), 6.68 (s, 1H), 5.39 (dd, $J_1$ = 10.0 Hz, $J_2$ = 4.0 Hz, 1H), 4.89 (dd, $J_1$ = 12.0 Hz, $J_2$ = 3.6 Hz, 1H), 4.39 (m, 3H), 4.29 (m, 1H), 4.18 (s, 4H), 4.00 (t, $J$ = 11.2 Hz, 1H), 3.66 (dd, $J_1$ = 8.0 Hz, $J_2$ = 7.2 Hz, 1H), 2.17 (s, 3H), 1.34 (d, $J$ = 6.0 Hz, 3H).

*(3S,11aR)-N-(1,3-benzothiazol-2-ylmethyl)-6-hydroxy-3-methyl-5,7-dioxo-2,3,5,7,11,11a-hexahydro[1,3]oxazolo[3,2-a]pyrido[1,2-d]pyrazine-8-carboxamide (1.1.37):* LC-MS (ESI) 427 (M+H)$^+$; $^1$H NMR (DMSO-$d_6$, 400 MHz) δ 11.54 (brs, 1H), 10.69 (t, $J$ = 5.6 Hz, 1H), 8.50 (s, 1H), 8.04 (d, $J$ = 8.0 Hz, 1H), 7.95 (d, $J$ = 8.0 Hz, 1H), 7.50 (t, $J$ = 7.2 Hz, 1H), 7.41 (t, $J$ = 7.2 Hz, 1H), 5.40 (dd, $J_1$ = 9.6 Hz, $J_2$ = 3.6 Hz, 1H), 4.97 (d, $J$ = 5.6 Hz, 2H), 4.90 (dd, $J_1$ = 12.0 Hz, $J_2$ = 3.6 Hz, 1H), 4.40 (t, $J$ = 7.6 Hz, 1H), 4.30 (m, 1H), 4.02 (t, $J$ = 11.2 Hz, 1H), 3.67 (t, $J$ = 7.6 Hz, 1H), 1.35 (d, $J$ = 6.0 Hz, 3H).

*(3S,11aR)-6-hydroxy-3-methyl-N-[(5-chloro-1,3-benzoxazol-2-yl)methyl]-5,7-dioxo-2,3,5,7,11,11a-hexahydro[1,3]oxazolo[3,2-alpyrido[1,2-d]pyrazine-8-carboxamide (1.1.38):* LC-MS (ESI) 445 (M+H)$^+$; $^1$H NMR (DMSO-$d_6$, 400 MHz) δ 11.52 (brs, 1H), 10.54 (t, $J$ = 5.6 Hz, 1H), 8.47 (s, 1H), 7.83 (d, $J$ = 2.0 Hz, 1H), 7.75 (d, $J$ = 8.8 Hz, 1H), 7.42 (dd, $J_1$ = 8.8 Hz, $J_2$ = 2.0 Hz, 1H), 5.39 (dd, $J_1$ = 10.0 Hz, $J_2$ = 4.0 Hz, 1H), 4.88 (m, 3H), 4.40 (dd, $J_1$ = 8.4 Hz, $J_2$ = 6.8 Hz, 1H), 4.30 (m, 1H), 4.01 (dd, $J_1$ = 11.6 Hz, $J_2$ = 6.4 Hz, 1H), 3.67 (dd, $J_1$ = 8.4 Hz, $J_2$ = 6.4 Hz, 1H), 1.35 (d, $J$ = 6.4 Hz, 3H).

*(3S,11aR)-6-hydroxy-3-methyl-N-[(6-chloro-1,3-benzoxazol-2-yl)methyl]-5,7-dioxo-2,3,5,7,11,11a-hexahydro[1,3]oxazolor[3,2-a]pyrido[1,2-d]pyrazine-8-carboxamide (1.1.39):* LC-MS (ESI) 445 (M+H)$^+$; $^1$H NMR (DMSO-$d_6$, 400 MHz) δ 11.52 (brs, 1H), 10.54 (t, $J$ = 5.4 Hz, 1H), 8.47 (s, 1H), 7.92 (d, $J$ = 1.2 Hz, 1H), 7.72 (d, $J$ = 8.4 Hz, 1H), 7.41 (dd, $J_1$ = 8.4 Hz, $J_2$ = 1.6 Hz, 1H), 5.40 (dd, $J_1$ = 10.0 Hz, $J_2$ = 4.0 Hz, 1H), 4.89 (m, 1H), 4.87 (d, $J$ = 6.0 Hz, 2H), 4.40 (dd, $J_1$ = 8.4 Hz, $J_2$ = 7.2 Hz, 1H), 4.30 (m, 1H), 4.01 (t, $J$ = 11.2 Hz, 1H), 3.67 (dd, $J_1$ = 8.0 Hz, $J_2$ = 6.8 Hz, 1H), 1.35 (d, $J$ = 6.4 Hz, 3H).

*(3S,11aR)-6-hydroxy-3-methyl-N-[(5-methyl-1H-benzimidazol-2-yl)methyl]-5,7-dioxo-2,3,5,7,11,11a-hexahydro[1,3]oxazolo[3,2-a]pyrido[1,2-d]pyrazine-8-carboxamide (1.1.40):* LC-MS (ESI) 424 (M+H)$^+$; $^1$H NMR (DMSO-$d_6$, 400 MHz) δ 12.19 (s, 1H), 11.47 (brs, 1H), 10.48 (s, 1H), 8.47 (s, 1H), 7.32 (m, 2H), 6.96 (m, 1H), 5.40 (m, 1H), 4.90 (m, 1H), 4.72 (m, 2H), 4.40 (m, 1H), 4.30 (m, 1H), 4.02 (m, 1H), 3.67 (m, 1H), 2.39 (s, 3H), 1.35 (d, $J$ = 4.4 Hz, 3H).

*(3S,11aR)-6-hydroxy-3-methyl-N-[(5-ɸmopo-1H-benzimidazol-2-yl)methyl]-5,7-dioxo-2,3,5,7,11,11a-hexahydro[1,3]oxazolo[3,2-a]pyridof[1,2-d]pyrazine-8-carboxamide (1.1.41):* LC-MS (ESI) 428 (M+H)$^+$; $^1$H NMR (DMSO-$d_6$, 400 MHz) δ 11.49 (brs, 1H), 10.51 (brs, 1H), 8.47 (s, 1H), 7.52 (m, 1H), 7.33 (d, $J$ = 8.4 Hz, 1H), 7.03 (t, $J$ = 8.4 Hz, 1H), 5.40 (m, 1H), 4.90 (m, 1H), 4.77 (brs, 2H), 4.40 (m, 1H), 4.30 (m, 1H), 4.02 (t, $J$ = 10.8 Hz, 1H), 3.67 (m, 1H), 1.35 (d, $J$ = 5.6 Hz, 3H).

*(3S,11aR)-6-hydroxy-3-methyl-N-[(1-methyl-6-chlorobenzimidazol-2-yl)methyl]-5,7-dioxo-2,3,5,7,11,11a-hexahydro[1,3]oxazolo[3,2-a]pyrido[1,2-d]pyrazine-8-carboxamide (1.1.43):* LC-MS (ESI) 458 (M+H)$^+$; $^1$H NMR (DMSO-$d_6$, 400 MHz) δ 11.47 (brs, 1H), 10.58 (t, $J$ = 5.2 Hz, 1H), 8.50 (s, 1H), 7.65 (d, $J$ = 1.2 Hz, 1H), 7.58 (d, $J$ = 8.4 Hz, 1H), 7.27 (dd, $J_1$ = 8.4 Hz, $J_2$ = 1.2 Hz, 1H), 5.40 (dd, $J_1$ = 9.6 Hz, $J_2$ = 3.2 Hz, 1H), 4.90 (m, 1H), 4.85 (d, $J$ = 5.2 Hz, 2H), 4.40 (dd, $J_1$ = 8.4 Hz, $J_2$ = 7.2 Hz, 1H), 4.30 (m, 1H), 4.02 (t, $J$ = 11.0 Hz, 1H), 3.81 (s, 3H), 3.67 (dd, $J_1$ = 8.4 Hz, $J_2$ = 7.2 Hz, 1H), 1.34 (d, $J$ = 6.0 Hz, 3H).

*(3S,11aR)-6-hydroxy-3-methyl-N-{[1-isopropyl-1H-benzimidazol-2-yl]methyl}-5,7-dioxo-2,3,5,7,11,11a-hexahydro[1,3]oxazolo[3,2-a]pyrido[1,2-d]pyrazine-8-carboxamide (1.1.44):* LC-MS (ESI) 452 (M+H)$^+$; $^1$H NMR (DMSO-$d_6$, 400 MHz) δ 11.47 (brs, 1H), 10.57 (t, $J$ = 5.2 Hz, 1H), 8.52 (s, 1H), 7.70 (d, $J$ = 8.0 Hz, 1H), 7.61 (d, $J$ = 8.0 Hz, 1H), 7.18 (m, 2H), 5.40 (dd, $J_1$ = 10.0 Hz, $J_2$ = 4.0 Hz, 1H), 4.91 (dd, $J_1$ = 12.0 Hz, $J_2$ = 4.0 Hz, 1H), 4.86 (d, $J$ = 5.2 Hz, 2H), 4.83 (m, 1H), 4.40 (dd, $J_1$ = 8.4 Hz, $J_2$ = 6.8 Hz, 1H), 4.29 (m, 1H), 4.02 (dd, $J_1$ = 12.0 Hz, $J_2$ = 10.0 Hz, 1H), 3.66 (dd, $J_1$ = 8.4 Hz, $J_2$ = 6.8 Hz, 1H), 1.56 (d, $J$ = 6.8 Hz, 6H), 1.34 (d, $J$ = 6.0 Hz, 3H).

*(3S,11aR)-6-hydroxy-N-[(2-oxo-2,3-dihydro-1H-benzimidazol-5-yl)methyl]-3-methyl-5,7-dioxo-2,3,5,7,11,11a-hexahydro[1,3]oxazolo[3,2-a]pyrido[1,2-d]pyrazine-8-carboxamide (1.1.46):* LC-MS (ESI) 426 (M+H)$^+$; $^1$H NMR (DMSO-$d_6$, 400 MHz) δ 11.46 (brs, 1H), 10.56 (s, 2H), 10.26 (t, $J$ = 5.6 Hz, 1H), 8.48 (s, 1H), 6.88 (m, 3H), 5.39 (m, 1H), 4.90 (m, 1H), 4.49 (d, $J$ = 5.6 Hz, 2H), 4.40 (dd, $J_1$ = 8.4 Hz, $J_2$ = 6.8 Hz, 1H), 4.29 (m, 1H), 4.01 (t, $J$ = 11.0 Hz, 1H), 3.66 (dd, $J_1$ = 8.4 Hz, $J_2$ = 6.8 Hz, 1H), 1.34 (d, $J$ = 6.0 Hz, 3H).

*(3S,11aR)-N-(2,1,3-benzoxadiazol-5-ylmethyl)-6-hydroxy-3-methyl-5,7-dioxo-2,3,5,7,11,11a-hexahydro[1,3]oxazolo[3,2-a]pyrido[1,2-d]pyrazine-8-carboxamide (1.1.47):* LC-MS (ESI) 412 (M+H)$^+$; $^1$H NMR (DMSO-$d_6$, 400 MHz) δ 11.51 (brs, 1H), 10.46 (t, $J$ = 6.0 Hz, 1H), 8.49 (s, 1H), 8.03 (d, $J$ = 9.4 Hz, 1H), 7.78 (s, 1H), 7.57 (d, $J$ = 9.4 Hz, 1H), 5.40 (dd, $J_1$ = 9.8 Hz, $J_2$ = 3.4 Hz, 1H), 4.90 (dd, $J_1$ = 12.0 Hz, $J_2$ = 3.4 Hz, 1H), 4.68 (d, $J$ = 5.6 Hz, 2H), 4.40 (t, $J$ = 7.6 Hz, 1H), 4.30 (m, 1H), 4.01 (t, $J$ = 11.2 Hz, 1H), 3.67 (dd, $J_1$ = 8.4 Hz, $J_2$ = 6.8 Hz, 1H), 1.35 (d, $J$ = 6.0 Hz, 3H).

*(3S,11aR)-6-hydroxy-3-methyl-N-(quinolin-2-ylmethyl)-5,7-dioxo-2,3,5,7,11,11a-hexahydro[1,3]-oxazolo[3,2-a]pyridor[1,2-d]pyrazine-8-carboxamide (1.1.49):* LC-MS (ESI) 421 (M+H)$^+$; $^1$H NMR (DMSO-$d_6$, 400 MHz) δ 11.44 (brs, 1H), 10.30 (t, $J$ = 5.6 Hz, 1H), 8.50 (s, 1H), 7.45 (s, 1H), 7.42 (d, $J$ = 8.0 Hz, 1H), 7.16 (dd, $J_1$ = 8.0 Hz, $J_2$= 0.8 Hz, 1H), 5.39 (dd, $J_1$ = 10.0 Hz, $J_2$ = 3.6 Hz, 1H), 4.90 (m, 1H), 4.61 (d, $J$ = 5.6 Hz, 2H), 4.39 (dd,

$J_1$ = 8.4 Hz, $J_2$ = 7.2 Hz, 1H), 4.29 (sxt, $J$ = 6.4 Hz, 1H), 4.01 (t, $J$ = 11.2 Hz, 1H), 3.70 (s, 3H), 3.66 (dd, $J_1$ = 8.4 Hz, $J_2$ = 6.8 Hz, 1H), 2.50 (s, 3H), 1.34 (d, $J$ = 6.4 Hz, 3H).

*(3S,11aR)-6-hydroxy-3-methyl-N-(quinolin-3-ylmethyl)-5,7-dioxo-2,3,5,7,11,11a-hexahydro[1,3]oxazolo[3,2-a]pyrido[1,2-d]pyrazine-8-carboxamide (1.1.50):* LC-MS (ESI) 421 (M+H)$^+$; $^1$H NMR (DMSO-$d_6$, 400 MHz) δ 11.50 (brs, 1H), 10.47 (t, $J$ = 6.0 Hz, 1H), 8.91 (d, $J$ = 2.0 Hz, 1H), 8.50 (s, 1H), 8.24 (s, 1H), 8.02 (d, $J$ = 8.0 Hz, 1H), 7.96 (d, $J$ = 7.6 Hz, 1H), 7.74 (dt, $J_1$ =8.0 Hz, $J_2$ =1.2 Hz, 1H), 7.61 (m, 1H), 5.39 (dd, $J_1$ = 10.0 Hz, $J_2$ = 4.0Hz, 1H), 4.89 (dd, $J_1$ = 12.0 Hz, $J_2$ = 4.0 Hz, 1H), 4.76 (d, $J$ = 6.0 Hz, 2H), 4.40 (dd, $J_1$ = 8.4 Hz, $J_2$ = 6.8 Hz, 1H), 4.30 (m, 1H), 4.01 (t, $J$ = 11.2 Hz, 1H), 3.67 (dd, $J_1$ = 8.4 Hz, $J_2$ = 6.8 Hz, 1H), 1.35 (d, $J$ = 6.0 Hz, 3H).

*(3S,11aR)-6-hydroxy-3-methyl-N-(2-methylquinolin-4-ylmethyl)-5,7-dioxo-2,3,5,7,11,11a-hexahydro[1,3]oxazolo[3,2-a]pyrido[1,2-d]pyrazine-8-carboxamide (1.1.51):* LC-MS (ESI) 435 (M+H)$^+$; $^1$H NMR (DMSO-$d_6$, 400 MHz) δ 11.49 (brs, 1H), 10.47 (t, $J$ = 5.6 Hz, 1H), 8.52 (s, 1H), 8.14 (d, $J$ = 8.4 Hz, 1H), 7.95 (d, $J$ = 8.4 Hz, 1H), 7.73 (t, $J$ = 7.6 Hz, 1H), 7.57 (t, $J$ = 7.2 Hz, 1H), 7.28 (s, 1H), 5.40 (dd, $J_1$ = 10.0 Hz, $J_2$ = 4.0 Hz, 1H), 5.03 (d, $J$ = 5.6 Hz, 2H), 4.91 (dd, $J_1$ = 12.0 Hz, $J_2$ = 4.0 Hz, 1H), 4.40 (dd, $J_1$ = 8.4 Hz, $J_2$ = 7.2 Hz, 1H), 4.30 (m, 1H), 4.02 (t, $J$ = 11.2 Hz, 1H), 3.67 (dd, $J_1$ = 8.4 Hz, $J_2$ = 6.8 Hz, 1H), 2.61 (s, 3H), 1.34 (d, $J$ = 6.4 Hz, 3H).

*(3S,11aR)-6-hydroxy-3-methyl-N-(quinolin-5-ylmethyl)-5,7-dioxo-2,3,5,7,11,11a-hexahydro[1,3]oxazolo[3,2-a]pyrido[1,2-d]pyrazine-8-carboxamide (1.1.52):* LC-MS (ESI) 421 (M+H)$^+$; $^1$H NMR (DMSO-$d_6$, 400 MHz) δ 11.44 (s, 1H), 10.42 (t, $J$ = 6.0 Hz, 1H), 8.92 (dd, $J_1$ = 8.0 Hz, $J_2$ = 1.2 Hz, 1H), 8.61 (d, $J$ = 8.0 Hz, 1H), 8.52 (s, 1H), 7.97 (d, $J$ = 8.4 Hz, 1H), 7.73 (t, $J$ = 8.2 Hz, 1H), 7.58 (m, 2H), 5.39 (dd, $J_1$ = 10.0 Hz, $J_2$ = 4.0 Hz, 1H), 5.03 (d, $J$ = 6.0 Hz, 2H), 4.91 (dd, $J_1$ = 12.0 Hz, $J_2$ = 4.0 Hz, 1H), 4.39 (dd, $J_1$ = 8.4 Hz, $J_2$ = 6.8 Hz, 1H), 4.29 (sxt, $J$ = 6.4 Hz, 1H), 4.01 (t, $J$ = 11.2 Hz, 1H), 3.66 (dd, $J_1$ = 8.4 Hz, $J_2$ = 6.8 Hz, 1H), 1.33 (d, $J$ = 6

*(3S,11aR)-6-hydroxy-3-methyl-N-(quinolin-8-ylmethyl)-5,7-dioxo-2,3,5,7,11,11a-hexahydro[1,3]oxazolo[3,2-a]pyrido[1,2-d]pyrazine-8-carboxamide (1.1.54):* LC-MS (ESI) 421 (M+H)$^+$; $^1$H NMR (DMSO-$d_6$, 400 MHz) δ 11.41 (brs, 1H), 10.49 (t, $J$ = *5.6* Hz, 1H), 8.99 (dd, $J_1$ = 4.0 Hz, $J_2$ = 1.6 Hz, 1H), 8.47 (s, 1H), 8.40 (dd, $J_1$ = 8.0 Hz, $J_2$ = 1.2 Hz, 1H), 7.91 (d, $J$ = 8.0 Hz, 1H), 7.67 (d, $J$ = 6.8 Hz, 1H), 7.58 (m, 2H), 5.39 (dd, $J_1$ = 10.0 Hz, $J_2$ = 3.6 Hz, 1H), 5.13 (d, $J$ = 5.6 Hz, 2H), 4.88 (dd, $J_1$ = 12.0 Hz, $J_2$ = 4.0 Hz, 1H), 4.39 (dd, $J_1$ = 8.0 Hz, $J_2$ = 7.6 Hz, 1H), 4.29 (m, 1H), 4.00 (t, $J$ = 11.2 Hz, 1H), 3.66 (dd, $J_1$ = 8.0 Hz, $J_2$ = 6.8 Hz, 1H), 1.34 (d, $J$ = 6.0 Hz, 3H).

*(3S,11aR)-6-hydroxy-3-methyl-N-(5,6,7,8-tetrahydro-[1,2,4]triazolo[4,3-a]pyridin-3 ylmethyl)-5,7-dioxo-2,3,5,7,11,11a-hexahydro[1,3]oxazolo[3,2-a]pyrido[1,2-d]pyrazine-8-carboxamide (1.1.61):* LC-MS (ESI) 415 (M+H)$^+$; $^1$H NMR (DMSO-$d_6$, 400 MHz) δ 11.48 (brs, 1H), 10.38 (t, $J$ = 5.6 Hz, 1H), 8.48(s, 1H), 5.39 (dd, $J_1$ = 10.0 Hz, $J_2$ = 3.6 Hz, 1H), 4.89 (dd, $J_1$ = 12.0 Hz, $J_2$ = 3.6 Hz, 1H), 4.64 (d, $J$ = 5.6 Hz, 2H), 4.39 (dd, $J_1$ = 8.0 Hz, $J_2$ = 7.2 Hz, 1H), 4.29 (sxt, $J$ = 6.0 Hz, 1H), 4.01 (t, $J$ = 11.2 Hz, 1H), 3.93 (t, $J$ = 6.0 Hz, 2H), 3.66 (dd, $J_1$ = 8.4 Hz, $J_2$ = 6.8 Hz, 1H), 2.79 (t, $J$ = 6.4 Hz, 2H), 1.88 (m, 2H), 1.79 (m, 2H), 1.34 (d, $J$ = 6.0 Hz, 3H).

*(3S,11aR)-6-hydroxy-N-(1,4,5,6,7,8-hexahydrocyclohepta[c]pyrazol-3-ylmethyl)-3-methyl-5,7-dioxo-2,3,5,7,11,11a-hexahydro[1,3]oxazolo[3,2-a]pyrido[1,2-d]pyrazine-8-carboxamide (1.1.62):* LC-MS (ESI) 428 (M+H)$^+$; $^1$H NMR (DMSO-$d_6$, 400 MHz) δ 12.13 (brs, 1H), 11.43 (brs, 1H), 10.07 (t, $J$ = 5.2 Hz, 1H), 8.47 (s, 1H), 5.40 (dd, $J_1$ = 10.0 Hz, $J_2$ = 3.6 Hz, 1H), 4.89 (dd, $J_1$ = 12.0 Hz, $J_2$ = 3.6 Hz, 1H), 4.39 (m, 3H), 4.29 (m, 1H), 4.00 (t, $J$ = 11.2 Hz, 1H), 3.66 (dd, $J_1$ = 8.4 Hz, $J_2$ = 6.8 Hz, 1H), 2.63 (m, 2H), 2.45 (m, 2H), 1.75 (m, 2H), 1.55 (m, 4H), 1.34 (d, $J$ = 6.0 Hz, 3H).

*(3S,11aR)-6-hydroxy-3-methyl-5,7-dioxo-N-(5,6,7,8-tetrahydro-4H-cyclohepta[d][1,3]thiazol-2-ylmethyl)-2,3,5,7,11,11a-hexahydro[1,3]oxazolo[3,2-a]pyrido[1,2-d]pyrazine-8-carboxamide (1.1.63):* LC-MS (ESI) 445 (M+H)$^+$; $^1$H NMR (DMSO-$d_6$, 400 MHz) δ 11.49 (brs, 1H), 10.47 (m, 1H), 8.48 (s, 1H), 5.39 (m, 1H), 4.90 (m, 1H), 4.67 (m, 2H), 4.40 (m, 1H), 4.30 (m, 1H), 4.01 (m, 1H), 3.67 (m, 1H), 2.82 (m, 2H), 2.73 (m, 2H), 1.78 (m, 2H), 1.60 (m, 4H), 1.34 (dd, $J_1$ = 6.4 Hz, $J_2$ = 2.8 Hz, 3H).

*(3S,11aR)-6-hydroxy-3-methyl-N-[(1,2-dimethyl-1H-benzimidazol-5-vl)methyl]-5,7-dioxo-2,3,5,7,11,11a-hexahydro[1,3]oxazolo[3,2-a]pyrido[1,2-d]pyrazine-8-carboxamide (1.1.64):* LC-MS (ESI) 438 (M+H)$^+$; $^1$H NMR (DMSO-$d_6$, 400 MHz) δ 11.44 (brs, 1H), 10.30 (t, $J$= 5.6 Hz, 1H), 8.50 (s, 1H), 7.45 (s, 1H), 7.42 (d, $J$= 8.0 Hz, 1H), 7.16 (dd, $J_1$ = 8.0 Hz, $J_2$ = 0.8 Hz, 1H), 5.39 (dd, $J_1$ = 10.0 Hz, $J_2$= 3.6 Hz, 1H), 4.90 (m, 1H), 4.61 (d, $J$= 5.6 Hz, 2H), 4.39 (dd, $J_1$ = 8.4 Hz, $J_2$ = 7.2 Hz, 1H), 4.29 (sxt, $J$ = 6.4 Hz, 1H), 4.01 (t, $J$ = 11.2 Hz, 1H), 3.70 (s, 3H), 3.66 (dd, $J_1$ = 8.4 Hz, $J_2$ = 6.8 Hz, 1H), 2.50 (s, 3H), 1.34 (d, $J$ = 6.4 Hz, 3H).

*(3S,11aR)-6-hydroxy-3-methyl-N-(quinolin-4-ylmethyl)-5,7-dioxo-2,3,5,7,11, 11a-hexahydro[1,3]oxazolo[3,2-a]pyrido[1,2-d]pyrazine-8-carboxamide (1.1.65)*: LC-MS (ESI) 421 (M+H)$^+$; $^1$H NMR (DMSO-$d_6$, 400 MHz) $\delta$ 11.49 (brs, 1H), 10.50 (t, $J$= 6.0 Hz, 1H), 8.85 (d, $J$= 4.0 Hz, 1H), 8.52 (s, 1H), 8.21 (d, $J$= 8.8 Hz, 1H), 8.06 (d, $J$= 8.0 Hz, 1H), 7.79 (t, $J$= 6.8 Hz, 1H), 7.66 (t, $J$ = 7.6 Hz, 1H), 7.39 (d, $J$= 4.0 Hz, 1H), 5.40 (dd, $J_1$ = 10.0 Hz, $J_2$ = 3.6 Hz, 1H), 5.08 (d, $J$ = 6.0 Hz, 2H), 4.91 (dd, $J_1$ = 12.0 Hz, $J_2$ = 3.6 Hz, 1H), 4.40 (dd, $J_1$ = 8.0 Hz, $J_2$ = 7.2 Hz, 1H), 4.29 (m, 1H), 4.02 (t, $J$ = 11.2 Hz, 1H), 3.67 (dd, $J_1$ = 8.0 Hz, $J_2$ = 6.8 Hz, 1H), 1.33 (d, $J$ = 6.0 Hz, 3H).

*(3aS,6R,13aS)-9-hydroxy-N-[(2,6-difluoropyridin-3-yl)methyl]-6-methyl-8,10-dioxo-1,2,3,5,6,8,10,13a-octahydrocyclopenta[b][1,3]oxazolo[3,2-a]pyrido[1,2-d]pyrazine-11-carboxamide (2.1.1):* LC-MS (ESI) 447 (M+H)$^+$; $^1$H NMR (DMSO-$d_6$, 400 MHz) $\delta$ 12.28 (s, 1H), 10.39 (t, $J$= 6.0 Hz, 1H), 8.41 (s, 1H), 8.08 (q, $J$= 8.4 Hz, 1H), 7.14 (dd, $J_1$ = 8.4 Hz, $J_2$ = 2.0 Hz, 1H), 4.55 (m, 2H), 4.36 (m, 3H), 3.76 (dd, $J_1$ = 8.0 Hz, $J_2$ = 6.0 Hz, 1H), 2.33 (m, 1H), 2.14 (m, 1H), 1.77 (m, 3H), 1.38 (d, $J$= 6.0 Hz, 3H).

*(3aS,6R,13aS)-9-hydroxy-N-[(3,5-difluoropyridin-4-yl]methyl]-6-methyl-8,10-dioxo-1,2,3,5,6,8,10,13a-octahydrocyclopenta[b][1,3]oxazolo[3,2-a]pyrido[1,2-d]pyrazine-11-carboxamide (2.1.2):* LC-MS (ESI) 447 (M+H)$^+$; $^1$H NMR (DMSO-$d_6$, 400 MHz) $\delta$ 12.28 (s, 1H), 10.48 (t, $J$= 6.0 Hz, 1H), 8.50 (s, 2H), 8.38 (s, 1H), 4.66 (m, 2H), 4.34 (m, 3H), 3.75 (dd, $J_1$= 8.0 Hz, $J_2$ = 6.0 Hz, 1H), 2.30 (m, 1H), 2.12 (m, 1H), 1.76 (m, 3H), 1.36 (d, $J$ = 6.0 Hz, 3H).

*(3aS,6R,13aS)-9-hydroxy-6-methyl-N-[(2,4,6-trifluoropyridin-3-yl)methyl]-8,10-dioxo-1,2,3,5,6,8,10,13a-octahydrocyclopenta[b][1,3]oxazolo[3,2-a]pyrido[1,2-d]pyrazine-11-carboxamide (2.1.3):* LC-MS (ESI) 465 (M+H)$^+$; $^1$H NMR (DMSO-$d_6$, 400 MHz) $\delta$ 12.28 (s, 1H), 10.42 (t, $J$ = 5.6 Hz, 1H), 8.38 (s, 1H), 7.34 (d, $J$= 8.0 Hz, 1H), 4.54 (m, 2H), 4.35 (m, 3H), 3.75 (m, 1H), 2.31 (m, 1H), 2.12 (m, 1H), 1.76 (m, 3H), 1.36 (d, $J$ = 6.0 Hz, 3H).

*(3aS,6S,13aS)-9-hydroxy-N-[(2,6-difluoropyridin-3-yl)methyl]-6-methyl-8,10-dioxo-1,2,3,5,6,8,10,13a-octahydrocyclopenta[b][1,3]oxazolo[3,2-a]pyrido[1,2-d]pyrazine-11-carboxamide (2.2.1):* LC-MS (ESI) 447 (M+H)$^+$; $^1$H NMR (DMSO-$d_6$, 400 MHz) $\delta$ 12.29 (s, 1H), 10.40 (t, $J$= 5.8 Hz, 1H), 8.41 (s, 1H), 8.08 (q, $J$= 8.4 Hz, 1H), 7.15 (dd, $J_1$ = 8.4 Hz, $J_2$ = 2.0 Hz, 1H), 4.55 (m, 2H), 4.35 (m, 3H), 3.75 (dd, $J_1$ = 8.0 Hz, $J_2$ = 6.4 Hz, 1H), 2.33 (m, 1H), 2.13 (m, 1H), 1.76 (m, 3H), 1.37 (d, $J$= 6.4 Hz, 3H).

*(3aS,6S,13aS)-9-hydroxy-N-[(3,5-difluoropyridin-4-yl-methyl]-6-methyl-8, 10-dioxo-1,2,3,5,6,8,10,13a-octahydrocyclopenta[b][1,3]oxazolo[3,2-a]pyrido[1,2-d]pyrazine-11-carboxamide (2.2.2):* LC-MS (ESI) 447 (M+H)$^+$; $^1$H NMR (DMSO-$d_6$, 400 MHz) $\delta$ 12.46 (s, 1H), 10.47 (t, $J$ = 5.8 Hz, 1H), 8.49 (s, 2H), 8.42 (s, 1H), 5.43 (dd, $J_1$ = 9.2 Hz, $J_2$= 4.0 Hz, 1H), 5.09 (brs, 1H), 4.66 (m, 3H), 4.60 (brs, 1H), 4.01 (dd, $J_1$ = 12.4 Hz, $J_2$= 9.6 Hz, 1H), 1.94 (brs, 4H), 1.84 (d, $J$= 12.0 Hz, 1H), 1.57 (m, 1H).

*(3aS,6S,13aS)-9-hydroxy-6-methyl-N-[(2,4,6-trifluoropyridin-3-yl)methyl]-8,10-dioxo-1,2,3,5,6,8,10,13a-octahydrocyclopenta[b][1,3]oxazolo[3,2-a]pyrido[1,2-d]pyrazine-11-carboxamide (2.2.3):* LC-MS (ESI) 46e (M+H)$^+$; $^1$H NMR (DMSO-$d_6$, 400 MHz) $\delta$ 12.28 (s, 1H), 10.42 (t, $J$ = 5.8 Hz, 1H), 8.38 (s, 1H), 7.34 (d, $J$= 8.4 Hz, 1H), 4.54 (m, 2H), 4.34 (m, 3H), 3.75 (dd, $J_1$ = 8.0 Hz, $J_2$= 6.0 Hz, 1H), 2.30 (m, 1H), 2.12 (m, 1H), 1.76 (m, 3H), 1.36 (d, $J$ = 6.0 Hz, 3H).

Example 3. General synthetic procedure for annelated 9-hydroxy-1,8-dioxo-1,3,4,8-tetrahydro-2H-pyrido[1,2-a]pyrazine-7-carboxamides of general formula 1.1.

*(3S,11aR)-6-hydroxy-N-[(2,6-difluoropyridin-3-yl)methyl7-3-methyl-5,7-dioxo-2,3,5,7,11,11a-hexahydro[1,3]oxazolo[3,2-a]pyrido[1,2-d]pyrazine-8-carboxamide (1.1.19)* is prepared according to Scheme 3.

Scheme 3. Synthesis of compound 1.1.19.

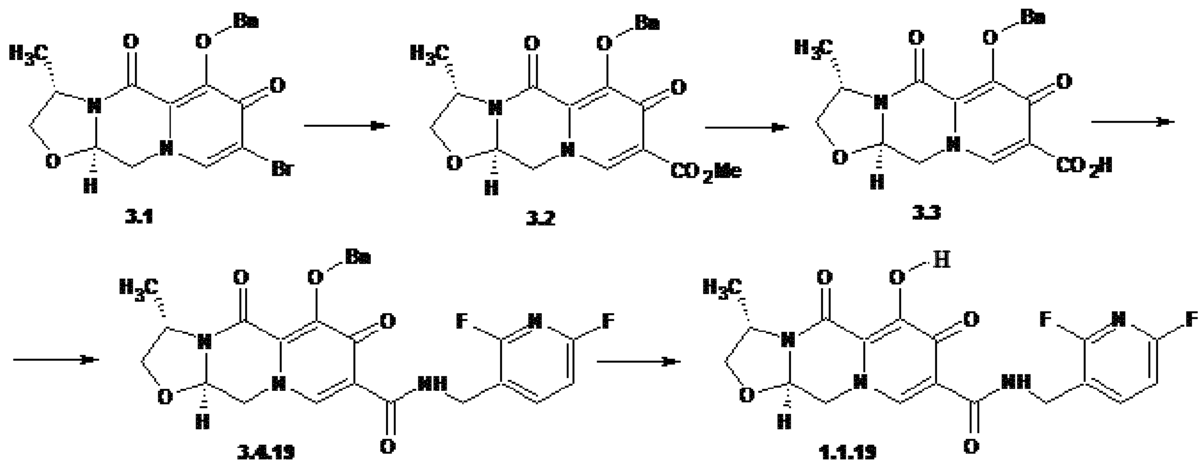

3.1 3.2 3.3

3.4.19 1.1.19

((3S,11a*R*)-6-(benzyloxy)-8-bromo-3-methyl-2,3,11,11a-tetrahydro[1,3]oxazolo[3,2-a]pyrido[1,2-d]pyrazine-5,7-dione (3.1) (3 g, 7.4 mmol) was dissolved in a mixture of THF (250 ml) and methanol (150 ml). The reaction mixture was transferred to an autoclave and triethylamine (1.3 ml, 8.9 mmol) and Pd(dppf)$Cl_2$ (0.05 g, 0.07 mmol) were added. CO was pumped to 15 atm, and the reaction mioxture was heated to 100°C for 3 days. Upon completion of the reaction (LC-MS control), the reaction mass was filtered through celite, evaporated in vacuum, dissolved in dichloromethane, washed with water, dried over sodium sulfate, and evaporated on a rotary evaporator. The residue was subjected to column chromatography on silica gel with dichloromethane - methanol (60 : 1) used as the eluent to yield 1.8 g (63 %) of *methyl (3S,11aR)-6-(benzyloxy)-3-methyl-5,7-dioxo-2,3,5,7,11,11a-hexahydro[1,3]-oxazolo[3,2-a]pyrido[1,2-d]pyrazine-8-carboxylate* (3.2): $^1$H NMR (DMSO-$d_6$, 400 MHz) δ 8.39 (s, 1H), 7.53 (m, 2H), 7.34 (m, 3H), 5.32 (dd, $J_1$ = 10.0 Hz, $J_2$ = 3.6 Hz, 1H), 5.04, 5.17 (ABq, $J_{AB}$ = 10.4 Hz, 2H), 4.67 (dd, $J_1$ = 12.0 Hz, $J_2$ = 3.2 Hz, 1H), 4.29 (m, 2H), 3.94 (dd, $J_1$ = 12.0 Hz, $J_2$ = 10.0 Hz, 1H), 3.63 (dd, $J_1$ = 8.0 Hz, $J_2$ = 6.4 Hz, 1H), 1.27 (d, $J$ = 6.0 Hz).

A solution of LiOH x $H_2O$ (0. 33 g, 7.8 mmol) in 5 ml of water was added to a solution of methyl ether 3.2 (1.5 g, 3.9 mmol) in 15 ml of dioxane. The mixture was then stirred for 3 h at 50°C. Dioxane was evaporated on a rotary evaporator. The residue was diluted with water and acidified with 10% sulfuric acid to pH 3. The precipitate was filtered off, washed with water, and dried in vacuum to yield 1 g (69 %) of *(3S, 11aR)-6-(benzyloxy)-3-methyl-*5,*7-dioxo-2,3,5,*7, *11, 11a-hexahydro[1,3joxazolo[3,2-a]pyrido[1,2-d]pyrazine-8-carboxylic acid* (3.3): $^1$H NMR (DMSO-$d_6$, 400 MHz) δ 15.45 (brs, 1H), 8.75 (s, 1H), 7.54 (m, 2H), 7.36 (m, 3H), 5.38 (dd, $J_1$ = 10.0 Hz, $J_2$ = 3.2 Hz, 1H), 5.15, 5.25 (ABq, $J_{AB}$ = 10.4 Hz, 2H), 4.87 (dd, $J_1$ = 12.0 Hz, $J_2$ = 3.2 Hz, 1H), 4.32 (m, 2H), 4.13 (t, $J$= 11.0 Hz, 1H), 3.66 (dd, $J_1$ = 7.6 Hz, $J_2$ = 6.4 Hz, 1H), 1.28 (d, $J$ = 6.0 Hz).

Triethylamine (1.2 ml, 8.1 mmol) was added to a solution of acid 3.3 (1 g, 2.7 mmol) in 20 ml of dichloromethane. The reaction mixture was stirred at room temperature for 15 minutes and TBTU (0.82 g, 3.3 mmol) was added. The mixture was stirred for another 15 min, then [(2,6-difluoropyridin-3-yl)methyl]amine (0.47 g, 3.3 mmol) was added, and the mixture was stirred for 14 h at room temperature. The reaction mass was washed with 10-% aqueous solution of potassium carbonate and water. The organic layer was dried over sodium sulfate and evaporated on a rotary evaporator. Diethyl ether was added to the residue, and the resulting precipitate was filtered off and dried in vacuum. The product was further used without extra purification. The yield was 0.94 g (70 %) of *(3S,11aR)-6-(benzyloxy)-3-methyl-5,7-dioxo-N-[(2,6-difluoropyridin-3-yl)methyl]-2,3,5,7,11,11a-hexahydro[1,3]oxazolo[3,2-a]pyrido[1,2-d]pyrazine-8-carboxamide* (3.4.19): $^1$H NMR (DMSO-$d_6$, 400 MHz) δ 10.45 (t, J=5.9 Hz, 1H), 8.56 (s, 1H), 8.13-8.00 (m, 1H), 7.57-7.52 (m, 2H), 7.40-7.29 (m, 3H), 7.15 (dd, J1=8.1 Hz, J2=2.3 Hz, 1H), 5.35 (dd, J1=10.0 Hz, J2=3.7 Hz, 1H), 5.21 (d, J=10.6 Hz, 1H), 5.07 (d, J=10.6 Hz, 1H), 4.79 (dd, J1=12.2 Hz, J2=3.7 Hz, 1H), 4.56 (d, J=5.9 Hz, 2H), 4.38-4.22 (m, 2H), 4.08-3.98 (m, 1H), 3.64 (dd, J1=8.1 Hz, J2=6.4 Hz, 1H), 1.27 (d, J=6.1 Hz, 3H).

Compopund 3.4.19 (0.9 g, 1.8 mmol) was dissolved in a mixture of THF (18 ml) and methanol (2 ml), then 10% Pd/C (0.09 g) was added, and the reaction mixture was stirred in a hydrogen atmosphere for 8 h. The reaction mass was passed through celite, the filtrate and then evaporated. The residue was treated with ether, the precipitate was filtered off and dried in vacuum to yield 0.6 g (81 %) of *(3S,11aR)-6-hydroxy-N-[(2,6-difluoro-pyridin-3-yl)methy]-3-methyl-5,7-dioxo-2,3,5,7,11,11a-hexahydror[1,3]oxazolor3,2a]pyrido[1,2-d]pyrazine-8-carboxamide* (1.1.19): LC-MS (ESI) 07 $(M+H)^+$; $^1$H NMR (DMSO-$d_6$, 400 MHz) δ 11.49 (brs, 1H), 10.36 (t, $J$= 5.8 Hz, 1H), 8.45 (s, 1H), 8.06 (dd, $J_1$ = 17.2 Hz, $J_2$ = 8.4 Hz, 1H), 7.14 (dd, $J_1$ = 8.0 Hz, $J_2$= 2.0 Hz, 1H), 5.38 (dd, $J_1$ = 10.0 Hz, $J_2$ = 3.6 Hz, 1H), 4.88 (dd, $J_1$ = 12.0 Hz, $J_2$ = 3.6 Hz, 1H), 4.55 (d, $J$ = 5.6 Hz, 2H), 4.39 (dd, $J_1$ = 8.0 Hz, $J_2$ = 6.8 Hz, 1H), 4.29 (m, 1H), 4.00 (t, $J$ = 11.0 Hz, 1H), 3.67 (dd, $J_1$ = 8.5 Hz, $J_2$= 6.8 Hz, 1H), 1.34 (d, $J$ = 6.4 Hz, 3H); XRD: Simulation of the diffraction pattern of an inhibitor 1.1.19 sample using the Pauli technique is shown in Figure 1. The sample obtained by recrystallization from methanol is single-phase. Unit cell parameters: a = 7.1564(5)Å, b = 10.3470(8)Å, c = 17.795(2)Å, α = 65.469(6)°, β = 106.211(6)°,

γ = 131.825(6)°. The unit cell volume is 893.28(17) Å$^3$. Systematic extinctions and cell volume are consistent with space group P-1.

*Sodium (3S, 11aR)-8-({[(2,6-difluoropyridin-3-yl)methyl]amino}carbonyl)-3-methyl-5,7-dioxo-2,3,5,7, 11, 11a-hexahydro[1,3]oxazolo[3,2-a]pyrido[1,2-d]pyrazin-6-olate* (1.1.20). NaOH (0.05 g, 1.2 mmol) is added to a suspension of (3S,11 a*R*)-6-hydroxy-*N*-[(2,4-difluoro-3-pyridyl)methyl]-3-methyl-5,7-dioxo-2,3,5,7,11,11 a-hexahydro[1,3]oxazole[3,2-*a*]pyrido[1,2-*d*]pyrazine-8-carboxamide (1.1.19) (0.05 g, 1.2 mmol) in 80 ml of ethanol and 20 ml of water at 75°C. The reaction mixture is stirred for 14 h at room temperature. The resulting precipitate is filtered off, washed with absolute ethanol, and dried in vacuum to yield 0.42 g (80 %) of *sodium (3S, 11aR)-8-({[(2,6-difluoropyridin-3-yl)methyl]amino)carbonyl)-3-methyl-5,7-dioxo-2,3,5,7,11, 11a-hexahydro[1,3]oxazolo[3,2-a]pyndo[1,2-d]pyrazin-6-olate* (1.1.20): $^1$H NMR (DMSO-d$_6$, 400 MHz) δ 10.76 (t, *J*= 5.6 Hz, 1H), 8.00 (dd, $J_1$ = 17.2 Hz, $J_2$= 8.0 Hz, 1H), 7.90 (s, 1H), 7.11 (dd, $J_1$ = 8.0 Hz, $J_2$ = 1.6 Hz, 1H), 5.21 (dd, $J_1$ = 10.0 Hz, $J_2$= 4.0 Hz, 1H), 4.59 (dd, $J_1$ = 12.4 Hz, $J_2$= 3.6 Hz, 1H), 4.52 (t, *J*= 5.6 Hz, 2H), 4.25 (m, 2H), 3.74 (t, *J* = 10.8 Hz, 1H), 3.60 (m, 1H), 1.27 (d, *J*= *5.2* Hz, 3H).

The following compounds are obtained in a similar way:

*(3S,11aR)-6-hydroxy-3-methyl-5,7-dioxo-N-(2-thienylmethyl)-2,3,5,7,11,11a-hexahydror[1,3]oxazolo[3,2-a]pyrido[1,2-d]pyrazine-8-carboxamide* (1.1.1): LC-MS (ESI) 376 (M+H)$^+$, $^1$H NMR (DMSO-d$_6$, 400 MHz) δ 11.47 (brs, 1H), 10.33 (t, *J* = 4.8 Hz, 1H), 8.49 (s, 1H), 7.40 (dd, $J_1$ = 8.8 Hz, $J_2$ = 4.8 Hz, 1H), 7.03 (d, *J* = 2.8 Hz, 1H), 6.96 (dd, $J_1$ = 4.8 Hz, $J_2$ = 3.6 Hz, 1H), 5.39 (dd, $J_1$ = 10.0 Hz, $J_2$= 4.0 Hz, 1H), 4.90 (dd, $J_1$ = 12.0 Hz, $J_2$ = 4.0 Hz, 1H), 4.70 (d, *J* = 5.6 Hz, 2H), 4.40 (dd, $J_1$ = 8.4 Hz, $J_2$ = 7.2 Hz, 1H), 4.29 (m, 1H), 4.01 (t, *J* = 11.2 Hz, 1H), 3.67 (dd, $J_1$ = 8.4 Hz, $J_2$ = 7.2 Hz, 1H), 1.34 (d, *J* = 6.0 Hz, 3H);

*(3S,11aR)-6-hydroxy-3-methyl-N-[(4-methyl-4H-1,3-oxazol-2-yl)methyl]-5,7-dioxo-2,3,5,7,11,11a-hexahydror[1,3]oxazolo[3,2-a]pyrido[1,2-d]pyrazine-8-carboxamide (1.1.14):* LC-MS (ESI) 375 (M+H)$^+$, $^1$H NMR (DMSO-d$_6$, 300 MHz, 80 °C) δ 10.49 (brs, 1H), 7.93 (s, 1H), 5.21 (dd, $J_1$ = 9.6 Hz, $J_2$ = 3.6 Hz, 1H), 4.58-4.85 (m, 3H), 4.29 (m, 2H), 3.64 (m, 2H), 2.30 (s, 3H), 1.30 (d, *J* = 5.4 Hz, 3H);

*(3S,11aR)-6-hydroxy-3-methyl-N-[(1,4,5-trimethyl-1H-imidazol-2-yl)methyl]-5,7-dioxo-2,3,5,7,11,11a-hexahydro[1,3]oxazolo[3,2-a]pyrido[1,2-d]pyrazine-8-carboxamide (1.1.10):* LC-MS (ESI) 402 (M+H)$^+$;

*(3S,11aR)-6-hydroxy-3-methyl-N-[(4-methyl-1,2,3-thiadiazol-5-yl)methyl]7-5, 7-dioxo-2,3,5,7,11,11a-hexahydro[1,3]oxazolo[3,2-a]pyrido[1,2-d]pyrazine-8-carboxamide (1.1.11):* LC-MS (ESI) 392 (M+H)$^+$, $^1$H NMR (DMSO-d$_6$, 400 MHz) δ 11.52 (brs, 1H), 10.45 (t, *J*= 5.6 Hz, 1H), 8.49 (s, 1H), 5.39 (dd, $J_1$ = 9.2 Hz, $J_2$ = 3.2 Hz, 1H), 4.89 (m, 1H), 4.81 (d, *J* = 5.6 Hz, 2H), 4.40 (dd, $J_1$ = 7.6 Hz, $J_2$ = 0.4 Hz, 1H), 4.29 (m, 1H), 4.00 (t, *J* = 11.2 Hz, 1H), 3.66 (t, *J* = 7.6 Hz, 1H), 2.67 (s, 3H), 1.34 (d, *J*= 6.0 Hz, 3H);

*(3S,11aR)-6-hydroxy-3-methyl-N-[(3-methyl-1,2,4-oxadiazol-5-yl)methyl]-5,7-dioxo-2,3,5,7,11,11a-hexahydro[1,3]oxazolo[3,2-a]pyrido[1,2-d]pyrazine-8-carboxamide (1.1.13):* LC-MS (ESI) 376 (M+H)$^+$;

*(3S,11aR)-6-hydroxy-3-methyl-N-[(3-fluoropyridin-2-yl)methyl]-5,7-dioxo-2,3,5,7,11,11a-hexahydro[1,3]oxazolo[3,2-a]pyrido[1,2-d]pyrazine-8-carboxamide (1.1.16):* LC-MS (ESI) 389 (M+H)$^+$, $^1$H NMR (DMSO-d$_6$, 400 MHz) δ 10.61 (t, J=5.4 Hz, 1H), 8.57 (s, 1H), 8.42 (d, J=4.5 Hz, 1H), 7.77-7.67 (m, 1H), 7.57-7.50 (m, 2H), 7.47-7.28 (m, 4H), 5.40-5.31 (m, 1H), 5.25-5.17 (m, 1H), 5.11-5.03 (m, 1H), 4.84-4.70 (m, 3H), 4.37-4.23 (m, 2H), 4.08-3.97 (m, 1H), 3.68-3.60 (m, 1H), 1.28 (d, J=6.1 Hz, 3H);

*(3S,11aR)-6-hydroxy-N-[(3,5-difluoropyridin-2-yl)methy]-3-methyl-5,7-dioxo-2,3,5,7,11,11a-hexahydro[1,3]oxazolo[3,2-a]pyrido[1,2-d]pyrazine-8-carboxamide* (1.1.17): LC-MS (ESI) 407 (M+H)$^+$, $^1$H NMR (DMSO-d$_6$, 400 MHz) δ 11.42 (brs, 1H), 10.54 (t, *J* = 5.2 Hz, 1H), 8.49 (d, *J* = 2.4 Hz, 1H), 8.46 (s, 1H), 7.95 (dt, $J_1$ = 11.4 Hz, $J_2$ = 2.4 Hz, 1H), 5.39 (dd, $J_1$ = 10.0 Hz, $J_2$= 4.0 Hz, 1H), 4.88 (dd, $J_1$ = 12.0 Hz, $J_2$ = 4.0 Hz, 1H), 4.71 (d, *J* = 4.8 Hz, 2H), 4.39 (dd, $J_1$ = 8.0 Hz, $J_2$ = 6.8 Hz, 1H), 4.29 (m, 1H), 4.00 (t, *J* = 11.2 Hz, 1H), 3.66 (dd, $J_1$ = 8.0 Hz, $J_2$ = 6.8 Hz, 1H), 1.34 (d, *J* = 6.4 Hz, 3H);

*(3S,11aR)-6-hydroxy-N-[(6-chloropyridin-3-yl)methyl]-3-methyl-5,7-dioxo-2,3,5,7,11,11a-hexahydro[1,3]oxazolo[3,2-a]pyrido[1,2-d]pyrazine-8-carboxamide (1.1.18):* LC-MS (M+1) = 405, $^1$H NMR (DMSO-d$_6$, 400 MHz) δ 10.76 (t, *J* = 5.8 Hz, 1H), 8.35 (s, 1H), 7.92 (s, 1H), 7.75 (d, *J* = 8.0 Hz, 1H), 7.45 (d, *J* = 8.0 Hz, 1H), 5.21 (m, 1H), 4.60 (m, 1H), 4.53 (m, 2H), 4.25 (m, 2H), 3.75 (t, *J*= 10.8 Hz, 1H), 3.59 (m, 1H), 1.26 (d, *J*= 4.4 Hz, 3H);

*Sodium (3S,11aR)-8-({[(2,6-difluoropyridin-3-yl)methyl]amino}carbonyl)-3-methyl-5,7-dioxo-2,3,5,7,11,11a-hexahydro[1,3]oxazolo[3,2-a]pyrido[1,2-d]pyrazin-6-olate (1.1.20):*LC-MS (ESI) 407 (M+H)$^+$, $^1$H NMR (DMSO-d$_6$, 400

MHz) δ 11.49 (brs, 1H), 10.36 (t, $J$= 5.8 Hz, 1H), 8.45 (s, 1H), 8.06 (dd, $J_1$ = 17.2 Hz, $J_2$= 8.4 Hz, 1H), 7.14 (dd, $J_1$ = 8.0 Hz, $J_2$= 2.0 Hz, 1H), 5.38 (dd, $J_1$ = 10.0 Hz, $J_2$= 3.6 Hz, 1H), 4.88 (dd, $J_1$ = 12.0 Hz, $J_2$ = 3.6 Hz, 1H), 4.55 (d, $J$= 5.6 Hz, 2H), 4.39 (dd, $J_1$ = 8.0 Hz, $J_2$ = 6.8 Hz, 1H), 4.29 (m, 1H), 4.00 (t, $J$ = 11.0 Hz, 1H), 3.67 (dd, $J_1$ = 8.5 Hz, $J_2$ = 6.8 Hz, 1H), 1.34 (d, $J$ = 6.4 Hz, 3H);

*(3S,11aR)-6-hydroxy-3-methyl-N-[(2,4,6-trifluoropyridin-3-yl)methyl]-5,7-dioxo-2,3,5,7,11,11a-hexahydro[1,3]oxazolo[3,2-a]pyrido[1,2-d]pyrazine-8-carboxamide (1.1.22):* LC-MS (ESI) 425 $(M+H)^+$, $^1H$ NMR (DMSO-$d_6$, 400 MHz) δ 11.46 (s, 1H), 10.36 (t, $J$ = 6.0 Hz, 1H), 8.43 (s, 1H), 7.33 (d, $J$= 8.4 Hz, 1H), 5.37 (dd, $J_1$ = 10.0 Hz, $J_2$= 4.0 Hz, 1H), 4.85 (dd, $J_1$ = 12.4 Hz, $J_2$ = 4.0 Hz, 1H), 4.55 (d, $J$ = 6.0 Hz, 2H), 4.39 (dd, $J_1$ = 8.4 Hz, $J_2$ = 6.8 Hz, 1H), 4.28 (sxt, $J$ = 6.4 Hz, 1H), 3.99 (dd, $J_1$ = 11.6 Hz, $J_2$ = 10.8 Hz, 1H), 3.66 (dd, $J_1$ = 8.4 Hz, $J_2$ = 6.8 Hz, 1H), 1.33 (d, $J$= 6.4 Hz, 3H);

*(3S,11aR)-6-hydroxy3-methyl-N-[(3-fluoropyridin-4-yl)methyl]-5,7-dioxo-2,3,5,7,11,11a-hexahydro[1,3]oxazolo[3,2-a]pyrido[1,2-d]pyrazine-8-carboxamide (1.1.23):* LC-MS (ESI) 389 $(M+H)^+$, $^1H$ NMR (DMSO-$d_6$, 400 MHz) δ 10.54-10.44 (m, 1H), 8.57 (s, 1H), 8.52 (s, 1H), 8.38 (d, J=4.4 Hz, 1H), 7.55 (d, J=7.2 Hz, 2H), 7.42-7.27 (m, 4H), 5.40-5.30 (m, 1H), 5.23 (d, J=10.4 Hz, 1H), 5.08 (d, J=10.4 Hz, 1H), 4.84-4.74 (m, 1H), 4.65 (d, J=5.6 Hz, 2H), 4.38-4.22 (m, 2H), 4.10-3.96 (m, 1H), 3.70-3.59 (m, 1H), 1.28 (d, J=5.7 Hz, 3H);

*(3S,11aR)-6-hydroxy-N-[(3,5-difluoropyridin-4-yl)methyl]-3-methyl-5,7-dioxo-2,3,5,7,11,11a-hexahydro[1,3]oxazolo[3,2-a]pyrido[1,2-d]pyrazine-8-carboxamide (1.1.24):* LC-MS (ESI) 407 $(M+H)^+$, $^1H$ NMR (DMSO-$d_6$, 400 MHz) 5 11.46 (brs, 1H), 10.43 (t, $J$ = 6.0 Hz, 1H), 8.50 (s, 2H), 8.43 (s, 1H), 5.37 (dd, $J_1$ = 10.0 Hz, $J_2$= 4.0 Hz, 1H), 4.84 (dd, $J_1$ = 12.0 Hz, $J_2$= 4.0 Hz, 1H), 4.67 (d, $J$= 6.0 Hz, 2H), 4.39 (dd, $J_1$ = 8.4 Hz, $J_2$= 6.8 Hz, 1H), 4.28 (m, 1H), 3.99 (dd, $J_1$ = 12.0 Hz, $J_2$ = 6.4 Hz, 1H), 3.66 (dd, $J_1$ = 8.8 Hz, $J_2$ = 6.8 Hz, 1H), 1.33 (d, $J$ = 6.0 Hz, 3H);

*(3S,11aR)-6-hydroxy-3-methyl-N-(pyrazin-2-ylmethyl)-5,7-dioxo-2,3,5,7,11,11a-hexahydro[1,3]oxazolo[3,2-a]pyrido[1,2-d]pyrazine-8-carboxamide (1.1.25):* LC-MS (ESI) 372 $(M+H)^+$, $^1H$ NMR (DMSO-$d_6$, 400 MHz) δ 11.45 (brs, 1H), 10.53 (t, $J$ = 5.6 Hz, 1H), 8.63 (s, 1H), 8.61 (d, $J$ = 2.0 Hz, 1H), 8.54 (d, $J$ = 2.0 Hz, 1H), 8.47 (s, 1H), 5.39 (dd, $J_1$ = 10.0 Hz, $J_2$= 4.0 Hz, 1H), 4.89 (dd, $J_1$ = 12.0 Hz, $J_2$= 4.0 Hz, 1H), 4.71 (d, $J$ = 5.6 Hz, 2H), 4.40 (t, $J$ = 7.6 Hz, 1H), 4.30 (m, 1H), 4.01 (t, $J$ = 11.0 Hz, 1H), 3.67 (dd, $J_1$ = 8.0 Hz, $J_2$ = 7.2 Hz, 1H), 1.34 (d, $J$ = 6.0 Hz, 3H);

*(3S,11aR)-6-hydroxy-3-methyl-N-[(2-chloroimidazo[2,1-b][1,3]thiazol-6-yl)methyl]-5,7-dioxo-2,3,5,7,11,11a-hexahydro[1,3]oxazolo[3,2-a]pyrido[1,2-d]pyrazine-8-carboxamide (1.1.28):* LC-MS (ESI) 450 $(M+H)^+$, $^1H$ NMR (DMSO-$d_6$, 400 MHz) δ 10.31 (brs, 1H), 8.06 (s, 1H), 7.57 (s, 1H), 5.25 (brs, 1H), 4.65 (m, 1H), 4.48 (brs, 2H), 4.28 (brs, 2H), 3.77 (brs, 1H), 3.63 (brs, 1H), 1.29 (brs, 3H);

*(3S,11aR)-6-hydroxy-N-(imidazo[2,1-b][1,3,4]thiadiazol-6-ylmethyl)-3-methyl-5,7-dioxo-2,3,5,7,11,11a-hexahydro[1,3]oxazolo[3,2-a]pyrido[1,2-d]pyrazine-8-carboxamide (1.1.29):* LC-MS (ESI) 417 $(M+H)^+$, $^1H$ NMR (DMSO-$d_6$, 400 MHz) δ 10.64 (brs, 1H), 9.18 (s, 1H), 8.00 (s, 1H), 7.91 (s, 1H), 5.22 (m, 1H), 4.60 (m, 1H), 4.49 (s, 2H), 4.25 (s, 2H), 3.75 (m, 1H), 3.59 (m, 1H), 1.26 (brs, 3H);

*(3S,11aR)-6-hydroxy-3-methyl-N-[(2-methylimidazo[2,1-b][1,3,4]thiadiazol-6-yl)methyl-5,7-dioxo-2,3,5,7,11,11a-hexahydro[1,3]oxazolo[3,2-a]pyrido[1,2-d]pyrazine-8-carboxamide (1.1.30):* LC-MS (ESI) 431 $(M+H)^+$, $^1H$ NMR (DMSO-$d_6$, 400 MHz) δ 11.43 (brs, 1H), 10.27 (t, $J$ = 5.2 Hz, 1H), 8.47 (s, 1H), 7.95 (s, 1H), 5.39 (dd, $J_1$ = 10.0 Hz, $J_2$= 3.6 Hz, 1H), 4.90 (dd, $J_1$ = 12.0 Hz, $J_2$ = 3.6 Hz, 1H), 4.49 (d, $J$ = 5.2 Hz, 2H), 4.39 (dd, $J_1$ = 8.4 Hz, $J_2$ = 7.2 Hz, 1H), 4.29 (m, 1H), 4.01 (t, $J$ = 11.2 Hz, 1H), 3.66 (dd, $J_1$ = 8.4 Hz, $J_2$ = 7.2 Hz, 1H), 2.70 (s, 3H), 1.34 (d, $J$ = 6.0 Hz, 3H);

*(3S,11aR)-6-hydroxy-3-methyl-N-[(1-methyl-benzimidazol-2-yl)methyl]-5,7-dioxo-2,3,5,7,11,11a-hexahydro[1,3]oxazolo[3,2-a]pyrido[1,2-d]pyrazine-8-carboxamide (1.1.42):* LC-MS (ESI) 424 $(M+H)^+$, $^1H$ NMR (DMSO-$d_6$, 400 MHz) δ 11.49 (brs, 1H), 10.60 (t, $J$ = 4.8 Hz, 1H), 8.44 (s, 1H), 7.68 (d, $J$= 8.0 Hz, 1H), 7.63 (d, $J$= 7.6 Hz, 1H), 7.37 (t, $J$= 8.0 Hz, 1H), 7.63 (t, $J$ = 7.6 Hz, 1H), 5.39 (dd, $J_1$ = 10.0 Hz, $J_2$ = 3.6 Hz, 1H), 4.95 (d, $J$ = 4.8 Hz, 2H), 4.88 (m, 1H), 4.40 (dd, $J_1$ = 8.4 Hz, $J_2$ = 6.8 Hz, 1H), 4.30 (m, 1H), 3.99 (t, $J$ = 11.2 Hz, 1H), 3.89 (s, 3H), 3.67 (dd, $J_1$ = 8.4 Hz, $J_2$ = 6.8 Hz, 1H), 1.35 (d, $J$= 6.4 Hz, 3H);

*(3S,11aR)-6-hydroxy-3-methyl-N-[(6-chloro-1-isopropyl-1H-benzimidazol-2-yl)methyl]-5,7-dioxo-2,3,5,7,11,11a-hexahydro[1,3]oxazolo[3,2-a]pyrido[1,2-d]pyrazine-8-carboxamide (1.1.45):* LC-MS (ESI) 486 $(M+H)^+$, $^1H$ NMR (DMSO-$d_6$, 400 MHz) δ 11.47 (brs, 1H), 10.57 (t, $J$= 5.2 Hz, 1H), 8.51 (s, 1H), 7.80 (d, $J$ = 2.0 Hz, 1H), 7.61 (d, $J$ = 8.8 Hz, 1H), 7.20 (dd, $J_1$ = 8.8 Hz, $J_2$ = 2.0 Hz, 1H), 5.40 (dd, $J_1$ = 10.0 Hz, $J_2$= 4.0 Hz, 1H), 4.91 (dd, $J_1$ = 12.0 Hz,

$J_2$ = 4.0 Hz, 1H), 4.86 (d, $J$ = 5.2 Hz, 2H), 4.83 (m, 1H), 4.40 (dd, $J_1$ = 8.4 Hz, $J_2$ = 7.2 Hz, 1H), 4.29 (m, 1H), 4.02 (dd, $J_1$ = 12.0 Hz, $J_2$= 10.0 Hz, 1H), 3.66 (dd, $J_1$ = 8.4 Hz, $J_2$= 7.2 Hz, 1H), 1.54 (d, $J$ = 6.8 Hz, 6H), 1.34 (d, $J$ = 6.0 Hz, 3H);

*(3S,11aR)-N-(2,1,3-benzothiadiazol-5-ylmethyl)-6-hydroxy3-methyl-5,7-dioxo-2,3,5,7,11,11a-hexahydror[1,3]oxazolo[3,2-a]pyrido[1,2-d]pyrazine-8-carboxamide (1.1.47):* LC-MS (ESI) 428 $(M+H)^+$;

*(3S,11aR)-6-hydroxy-3-methyl-N-(guinolin-6-yl)methyl)-5,7-dioxo-2,3,5,7,11,11a-hexahydro[1,3]oxazolo[3,2-a]pyrido[1,2-d]pyrazine-8-carboxamide (1.1.53):* LC-MS (ESI) 421 $(M+H)^+$;

*(3S,11aR)-6-hydroxy-N-(soquinolin-1-ylmethyl)-3-methyl-5,7-dioxo-2,3,5,7,11,11a-hexahydro[1,3]oxazolo[3,2-a]pyrido[1,2-d]pyrazine-8-carboxamide (1.1.55):* LC-MS (ESI) 421 $(M+H)^+$, $^1H$ NMR (DMSO-$d_6$, 300 MHz, 80°C) δ 11.25 (brs, 1H), 10.64 (t, $J$ = 5.2 Hz, 1H), 8.47 (m, 2H), 8.32 (d, $J$ = 8.0 Hz, 1H), 7.99 (d, $J$ = 8.0 Hz, 1H), 7.75 (m, 3H), 5.42 (dd, $J_1$ = 10.0 Hz, $J_2$ = 3.6 Hz, 1H), 5.20 (d, $J$ = 5.2 Hz, 2H), 4.85 (dd, $J_1$ = 12.0 Hz, $J_2$ = 3.6 Hz, 1H), 4.38 (m, 2H), 4.01 (t, $J$= 10.8 Hz, 1H), 3.70 (m, 1H), 1.38 (d, $J$ = 5.6 Hz, 3H);

*(3S,11aR)-6-hydroxy-N-(imidazo[1,2-a]pyridin-3-yl)methyl-3-methyl-5,7-dioxo-2,3,5,7,11,11a-hexahydro[1,3]oxazolo[3,2-a]pyrido[1,2-d]pyrazine-8-carboxamide (1.1.56):* LC-MS (M+1) = 410, $^1H$ NMR (DMSO-$d_6$, 400 MHz) δ 10.67 (t, $J$= 5.6 Hz, 1H), 8.48 (d, $J$ = 6.4 Hz, 1H), 7.96 (s, 1H), 7.57 (m, 2H), 7.23 (t, $J$ = 8.0 Hz, 1H), 6.92 (t, $J$ = 7.2 Hz, 1H), 5.20 (dd, $J_1$ = 9.6 Hz, $J_2$ = 3.6 Hz, 1H), 4.89 (m, 2H), 4.61 (m, 1H), 4.23 (m, 2H), 3.75 (t, $J$= 10.8 Hz, 1H), 3.58 (m, 1H), 1.24 (d, $J$ = 5.6 Hz, 3H);

*(3S,11aR)-6-hydroxy-3-methyl-N-(1,2,4-triazolo[4,3-a]pyridin-3-ylmethyl-5,7-dioxo-2,3,5,7,11,11a-hexahydro[1,3]oxazolo[3,2-a]pyrido[1,2-d]pyrazine-8-carboxamide (1.1.57):* LC-MS (ESI) 411 $(M+H)^+$;

*(3S,11aR)-6-hydroxy-N-(imidazo[1,2-a]pyrimidin-2-ylmethyl)-3-methyl-5,7-dioxo-2,3,5,7,11,11a-hexahydro[1,3]oxazolo[3,2-a]pyrido[1,2-d]pyrazine-8-carboxamide* (1.1.58): LC-MS (ESI) 411 $(M+H)^+$, $^1H$ NMR (DMSO-$d_6$, 400 MHz) δ 11.46 (brs, 1H), 10.41 (t, $J$ = 5.6 Hz, 1H), 8.94 (d, $J$ = 5.6 Hz, 1H), 8.50 (m, 2H), 7.79 (s, 1H), 7.03 (m, 1H), 5.40 (m, 1H), 4.91 (m, 1H), 4.67 (d, $J$ = 5.6 Hz, 2H), 4.39 (dd, $J_1$ = 8.0 Hz, $J_2$= 7.2 Hz, 1H), 4.30 (m, 1H), 4.02 (m, 1H), 3.67 (dd, $J_1$ = 8.0 Hz, $J_2$ = 7.2 Hz, 1H), 1.34 (d, $J$ = 6.0 Hz, 3H);

*(3S,11aR)-6-hydroxy-N-(imidazo[1,2-a]pyrazin-3-ylmethyl)-3-methyl-5,7-dioxo-2,3,5,7,11,11a-hexahydro[1,3]oxazolo[3,2-a]pyrido[1,2-d]pyrazine-8-carboxamide (1.1.59):* LC-MS (ESI) 411 $(M+H)^+$, $^1H$ NMR (DMSO-$d_6$, 300 MHz, 80 °C) δ 10.39 (brs, 1H), 8.98 (s, 1H), 8.59 (d, $J$ = 6.0 Hz, 1H), 8.07 (brs, 1H), 7.88 (d, $J$= 6.0 Hz, 1H), 7.76 (s, 1H), 5.23 (m, 1H), 4.89 (m, 2H), 4.65 (m, 1H), 4.26 (s, 2H), 3.72 (brs, 1H), 3.60 (m, 1H), 1.28 (d, $J$ = 6.0 Hz, 3H);

*(3S,11aR)-6-hydroxy-3-methyl-N-[(6-chloro-1,2,4-triazolo[4,3-b]pyridazin-3-yl)methyl]-5,7-dioxo-2,3,5,7,11,11a-hexahydro[1,3]oxazolo[3,2-a]pyrido[1,2-d]pyrazine-8-carboxamide (1.1.60):* LC-MS (ESI) 446 $(M+H)^+$, $^1H$ NMR (DMSO-$d_6$, 400 MHz) δ 11.50 (brs, 1H), 10.59 (brs, 1H), 8.47 (m, 2H), 7.52 (d, $J$ = 9.6 Hz, 1H), 5.38 (dd, $J_1$ = 10.0 Hz, $J_2$= 4.0 Hz, 1H), 5.04 (d, $J$ = 5.6 Hz, 2H), 4.88 (m, 1H), 4.39 (dd, $J_1$ = 8.2 Hz, $J_2$ = 7.2 Hz, 1H), 4.29 (sxt, $J$ = 6.4 Hz, 1H), 4.00 (t, $J$ = 11.0 Hz, 1H), 3.66 (dd, $J_1$ = 8.2 Hz, $J_2$ = 7.2 Hz, 1H), 1.34 (d, $J$ = 6.4 Hz, 3H);

**Example 4. General synthetic procedure for annelated 9-hydroxy-1,8-dioxo-1,3,4,8-tetrahydro-2H-pyrido[1,2-a]pyrazine-7-carboxamides of general formula 1.2 or 1.3.**

Inhibitors 1.2 and 1.3 are prepared similartly to the synthesis of compounds of general formula 1.1 (Example 3) starting from (4R,12aS)-7-benzyloxy-9-bromo-4-methyl-6,8-dioxo-3,4,6,8,12,12a-hexahydro-2H-pyrido[1',2':4,5]pyrazino-[2,1-b][1,3]oxazine and (2R,5S,13aR)-8-benzyloxy-10-bromo-7,9-dioxo-2,3,4,5,7,9,13,13a-octahydro-2,5-methanopurido[1',2':4,5]pyrazino[2,1-b][1,3]oxazepine, respectively, including as follows:

*(4R,12aS)-7-hydroxy-N-[(2,6-difluoropyridin-3-yl)methyl]-4-methyl-6,8-dioxo-3,4,6,8, 12, 12a-hexahydro-2H-pyrido[1',2':4,5]pyrazino[2,1-b][1,3]oxazine-9-carboxamide* (1.2.1):LC-MS (ESI) 421 $(M+H)^+$, $^1H$ NMR (DMSO-$d_6$, 400 MHz) δ 12.51 (brs, 1H), 10.40 (t, $J$ = 5.8 Hz, 1H), 8.48 (s, 1H), 8.05 (q, $J$ = 8.8 Hz, 1H), 7.14 (dd, $J_1$ = 8.0 Hz, $J_2$= 2.4 Hz, 1H), 5.44 (m, 1H), 4.79 (m, 1H), 4.55 (m, 3H), 4.34 (dd, $J_1$ = 13.6 Hz, $J_2$= 5.6 Hz, 1H), 4.03 (dt, $J_1$ = 12.0 Hz, $J_2$ = 1.6 Hz, 1H), 3.89 (m, 1H), 2.01 (m, 1H), 1.55 (m, 1H), 1.33 (d, $J$ = 7.2 Hz, 3H);

*(4R,12aS)-7-hydroxy-4-methyl-N-[(2,4.6-trifluoropyridin-3-yl)methy]l-6,8-dioxo-3,4,6,8,12,12a-hexahydro-2H-pyrido[1',2':4,5]pyrazino[2,1-b][1,3]oxazine-9-carboxamide (1.2.3):* LC-MS (ESI) 439 $(M+H)^+$, $^1H$ NMR (DMSO-$d_6$, 400

MHz) δ 12.49 (brs, 1H), 10.39 (t, $J$ = 6.0 Hz, 1H), 8.46 (s, 1H), 7.33 (d, $J$ = 8.4 Hz, 1H), 5.43 (dd, $J_1$ = 9.6 Hz, $J_2$ = 4.0 Hz, 1H), 4.78 (p, $J$ = 6.6 Hz, 1H), 4.53 (m, 3H), 4.32 (dd, $J_1$ = 13.6 Hz, $J_2$ = 5.6 Hz, 1H), 4.02 (t, $J$ = 12.0 Hz1H), 3.88 (m, 1H), 2.01 (m, 1H), 1.54 (m, 1H), 1.32 (d, $J$ = 7.2 Hz, 3H);

*(4R,12aS)-7-hydroxy-N-[(3,5-difluoropyridin-4-yl)methyl]-4-methyl-6,8-dioxo-3,4,6,8,12,12a-hexahydro-2H-pyrido[1',2':4,5]pyrazino[2,1-b][1,3]oxazine-9-carboxamide (1.2.4):* LC-MS (ESI) 421 $(M+H)^+$, $^1H$ NMR (DMSO-$d_6$, 400 MHz) δ 12.49 (s, 1H), 10.47 (t, $J$ = 5.6 Hz, 1H), 8.50 (s, 2H), 8.45 (brs, 1H), 5.43 (m, 1H), 4.78 (m, 1H), 4.66 (d, $J$ = 5.6 Hz, 2H), 4.53 (m, 1H), 4.32 (m, 1H), 4.02 (t, $J_1$ = 12.0 Hz, 1H), 3.89 (m, 1H), 2.00 (m, 1H), 1.54 (d, $J$ = 13.6 Hz, 1H), 1.32 (d, $J$ = 7.2 Hz, 3H);

*(2R,5S,13aR)-8-hydroxy-N-[(2,6-difluoropyridin-3-yl)methyl]7,9-dioxo-2,3,4,5,7,9,13,13a-octahydro-2,5-methanopurido[1',2':4,5]pyrazino[2,1-b][1,3]oxazepine-10-carboxamide (1.3. 1):* LC-MS (M+1) = 433, $^1H$ NMR (DMSO-$d_6$, 400 MHz) δ 12.47 (s, 1H), 10.41 (m, 1H), 8.45 (s, 1H), 8.04 (m, 1H), 7.15 (d, $J$ = 7.2 Hz, 1H), 5.44 (m, 1H), 5.10 (brs, 1H), 4.68 (m, 1H), 4.60 (brs, 1H), 4.55 (m, 2H), 4.03 (m, 1H), 1.94 (brs, 4H), 1.84 (m, 1H), 1.58 (m, 1H);

*(2R,5S,13aR)-8-hydroxy-N-[(2,4,6-trifluoropyridin-3-yl)methyl]-7,9-dioxo-2,3,4,5,7,9,13,13a-octahydro-2,5-methanopyrido[1',2':4,5]pyrazino[2,1-b][1,3]oxazepine-10-carboxamide (1.3.2):* LC-MS (ESI) 451 $(M+H)^+$, $^1H$ NMR (DMSO-$d_6$, 400 MHz) δ 12.46 (s, 1H), 10.40 (t, $J$ = 5.6 Hz, 1H), 8.42 (s, 1H), 7.33 (d, $J$ = 8.4 Hz, 1H), 5.43 (dd, $J_1$ = 10.0 Hz, $J_2$ = 4.0 Hz, 1H), 5.09 (brs, 1H), 4.65 (dd, $J_1$ = 12.8 Hz, $J_2$ = 4.0 Hz, 1H), 4.60 (brs, 1H), 4.54 (m, 2H), 4.01 (dd, $J_1$ = 12.4 Hz, $J_2$ = 9.6 Hz, 1H), 1.94 (brs, 4H), 1.84 (d, $J$ = 12.0 Hz, 1H), 1.57 (m, 1H);

*(2R,5S,13aR)-8-hydroxy-N-[(3,5-difluoropyridin-4-yl)methyl]-7,9-dioxo-2,3,4,5,7,9,13,13a-octahydro-2,5-methanopyrido[1',2':4,5]pyrazino[2,1-b][1.3]oxazepine-10-carboxamide (1.3.3):* LC-MS (ESI) 433 $(M+H)^+$, $^1H$ NMR (DMSO-$d_6$, 400 MHz) δ 12.46 (s, 1H), 10.47 (t, $J$ = 5.8 Hz, 1H), 8.49 (s, 2H), 8.42 (s, 1H), 5.43 (dd, $J_1$ = 9.2 Hz, $J_2$ = 4.0 Hz, 1H), 5.09 (brs, 1H), 4.66 (m, 3H), 4.60 (brs, 1H), 4.01 (dd, $J_1$ = 12.4 Hz, $J_2$ = 9.6 Hz, 1H), 1.94 (brs, 4H), 1.84 (d, $J$ = 12.0 Hz, 1H), 1.57 (m, 1H);

**Example 5. A pharmaceutical composition in the form of a tablet.**

Starch (1600 mg), ground lactose (1600 mg), talc (400 mg), and 900 mg of the compound of general formula 1 or 2 were mixed and pressed into a bar. The resulting bar was crushed into granules and sieved through a sieve to collect granules of 14-16 mesh size. The granules thus obtained were formed into tablets of a suitable form weighing 225 or 450 mg each.

**Example 6. A pharmaceutical composition in the form of capsules.**

Compounds of general formula 1 or 2 were thoroughly mixed with lactose powder in a 2:1 ratio. The resulting powdery mixture was packaged in suitable gelatin capsules of 30, 60, or 120 mg in each capsule.

**Example 7. A lyophilized pharmaceutical nanocomposition.**

Zirconium sand (150 ml) (grinding media: 0.5 mm YTZ® Zirconia Grinding and Dispersion Media; Tosoh USA, Inc.) and a solution of poloxamer P338 (10.0 g) and sucrose or mannitol (11.6 g) in 400 ml of phosphate buffered saline (PBS, pH = 7.4) were loaded into a container (Jar). The resulting solution (150 ml) was placed in a jar and zirconium sand (150 ml) (grinding media: 0.5 mm YTZ® Zirconia Grinding and Dispersion Media; Tosoh USA, Inc.) and a compound of general formula 1 or 2 (~ 15 g) were added. The jar was placed on a jar mill (U.S. Stoneware 700 Series), the rotation speed was set at 104 rpm, and the mixture was ground for 24 hours. The end of the process was controlled by particle size distribution measurements on a Malvern Zetasizer Nano ZS instrument. At the end of the grinding process, the rotation was stopped and the mixture was left for 16-20 hours at 2-8°C to let the zirconium sand precipitate. The suspension was filtered through a glass filter with a porosity of 5-15 microns and analyzed to determine the content (concentration) of the compound of general formula 1 or 2. The resulting nanosuspension with a particle size of 200 to 900 nm, preferably 200-250 nm, was poured in sterile conditions at 2 ml in 5-ml sterile glass vials, frozen and lyophilized to yield a pharmaceutical nanocomposition in the form of a lyophilizate.

**Example 8. Injectable drug.**

A previously prepared sterile PBS solution with pH = 6.8 was added to the nanocomposition obtained in Example 7 in the form of a lyophilizate at the rate of 2.2 ml per 5 ml vial. The vials were stoppered with pre-sterilized plugs and

then sealed. The resulting pharmaceutical nanosuspension is ready for use.

**Example 9. Injectable drug.**

A compound of general formula 1 or 2 was sterilized with gamma radiation and subjected to clean milling of wet granules with mannite, polysorbate 20, polyethylene glycol 3350, and water for injection to achieve an average cabotegravir particle size of 200 nm. The nanosuspension was placed in pre-sterilized glass vials. The vials were stoppered with pre-sterilized plugs and then sealed. The resulting pharmaceutical nanosuspension is ready for use.

**Example 10. Anti-HIV activity of compounds of general formula 1 or 2 and the**

**prototype (CAB).**

a) *Inhibitory activity of the lymphocytropic virus strain HIV-1 (IIIB) in the CEM-SS cell line.*

The evaluation was done by ImQuest BioSciences, Inc. (Frederick, MD, USA). CEM-SS cells [Foley G.E. et al. Continuous Culture of Human Lymphoblasts from Peripheral Blood of a Child with Acute Leukemia. Cancer 1965, 18, 522-529] obtained through the NIH AIDS Research and Reference Reagent Program (Manassas, VA, USA) were passaged in T-75 flasks prior to use cell culture medium consisting of RPMI1640 medium (Lonza; Walkersville, MD, USA) supplemented with 10% heat inactivated fetal bovine serum (Gibco; Grand Island, NY), 2 μM L-glutamine (Lonza), 100 U/μl penicillin (Lonza) and 100 μg/μl streptomycin (Lonza) for antiviral analysis. The CEM-SS cells were kept in RPMI 140 medium supplemented with 10% heat inactivated bovine fetal serum, 2 mM glutamine, 100 U/ml penicillin, and 100 μg/ml streptomycin.

A lymphocytropic HIV-1 IIIB strain [Popovic, M. et al. Detection, isolation, and continuous production of cytopathic retroviruses (HTLV-III) from patients with AIDS and pre-AIDS. Science. 1984, 224 (4648), 497-500] was chosen for a virus to be analyzed. The virus was obtained through the NIH AIDS Research and Reference Reagents Program and grown in CEM-SS cells to produce a stock virus pool. A pre-titrated aliquot of the virus was removed from the freezer (-80°C) and allowed to slowly thaw to room temperature in a biological safety cabinet. The virus was diluted in a tissue culture medium so that the amount of virus added in 50 μl per well was sufficient to kill 85 to 95% of the cells 6 days after infection. HIV-1 IIIB was grown and titrated in CEM-SS cells. The inhibitory effect of CEM-SS compounds on HIV-1 compounds was evaluated in CEM-SS cells as described earlier [Nara, P.L. at al. Simple, rapid, quantitative, syncytium-forming microassay for the detection of human immunodeficiency virus neutralizing antibody. AIDS Res. Hum. Retroviruses 1987, 3, 283-302.] in microtiter plate anti-HIV assays to quantitatively assess the ability of a compound to inhibit HIV-induced cell death. Quantification was carried out using 2,3-bis-(2-methoxy-4-nitro-5-sulfenyl)-(2H)-tetrazolium-5-carboxanilide (XTT) tetrazolium, which is metabolized to a stained formazan product through viable cellsю

The compounds given in Table 1 were dissolved at 10 μM in DMSO and stored at - 20°C. Test materials were evaluated using antiviral assays in triplicate in concentrations of 30 and 10 nM. The compounds shown in table 1 were dissolved at 10 μM in DMSO and stored at -20 ° C. Test materials were evaluated using concentrations of 30 and 10 nM in triplicate for antiviral assays. Sixty nanomolar concentrations of each compound (2 times that of the test concentration in the well) were prepared by diluting 10 μM DMSO stock 1:100 in cell culture medium (10 μl of compound added to 990 μl of the medium) followed by 1:10 dilution (10 μl of the compound was added to 90 μl of the medium), then 6 μl was added to 994 μl of the medium; 100 100 μl was added to a 96-well microtiter plate in triplicate to ensure efficacy and one colorimetric well. Twenty nanomolar concentrations of each compound (twice as much as the test concentration in the well) were prepared by diluting 10 μM DMSO with the original 1:100 in cell culture medium (10 μl of the compound added to 990 μl of the medium) followed by diluting 1:100 (10 μl of the compound was added to 900 μl of the medium), then 20 μl was added to 980 μl of the medium; 100 μl was added to a 96-well microtiter plate in triplicate to ensure efficacy and one colorimetric well.

Azidothymidine (AZT) was purchased from Sigma Aldrich (USA) and evaluated as a positive control compound in an antiviral assay using a six-concentration reaction curve on each assay plate.

Test compounds were initially evaluated for HIV-1 IIIB strain in CEM-SS cells at test concentrations of 30 and 10 nM. Antiviral efficacy data are summarized in Table 1. Compounds that were active at 10 and 30 nM were evaluated in an HIV cytoprotection assay at 2 nM. For compounds showing activity at 2 nM, inhibitory activity ($EC_{50}$) was determined. Data on antiviral efficacy are also shown in Table 1.

The AZT control compound was evaluated in parallel with the test compounds presented and gave $EC_{50}$ values ranging from less than 2 nM to 7 nM. When evaluating the antiviral activity of compounds at 2 nM, AZT demonstrated $EC_{50}$ values ranging from 10 to 20 nM.

**Table 1**. Inhibitory activity ($EC_{50}$) against HIV-1 (IIIB strain) demonstrated by compounds of general formula 1 and 2 in the CEM-SS cell line.

| Compound No | $EC_{50}$ (nM) |
|---|---|
| 1.1.1 | 1.44 |
| 1.1.3 | >2 - <10 |
| 1.1.4 | ~8 |
| 1.1.5 | >10 - <30 |
| 1.1.6 | >10 - <30 |
| 1.1.7 | 0.59 |
| 1.1.8 | >2 - <10 |
| 1.1.9 | >30 |
| 1.1.11 | >30 |
| 1.1.12 | >10 - <30 |
| 1.1.14 | -10 |
| 1.1.15 | >10 - <30 |
| 1.1.28 | >30 |
| 1.1.29 | >30 |
| 1.1.30 | >30 |
| 1.1.31 | >2 - <10 |
| 1.1.32 | >2 - <10 |
| 1.1.33 | >10 - <30 |
| 1.1.34 | >2 - <10 |
| 1.1.35 | <2 |
| 1.1.36 | >10 - <30 |
| 1.1.37 | 1.93 |
| 1.1.38 | >30 |
| 1.1.39 | >2 - <10 |
| 1.1.40 | >30 |
| 1.1.52 | -10 |
| 1.1.54 | 2.17 |
| 1.1.55 | >2 - <10, 14.5[a] |
| 1.1.56 | >30 |
| 1.1.58 | >30 |
| 1.1.59 | >30 |
| 1.1.60 | >10 - <30 |
| 1.1.61 | >30 |
| 1.1.62 | >2 - <10 |
| 1.1.63 | >30 |
| 1.1.64 | >30 |
| 1.1.65 | <2 |

(continued)

| Compound No | $EC_{50}$ (nM) |
|---|---|
| 1.1.16 | >10 - <30, 10.5[a] |
| 1.1.17 | 0.24, 1.5[a] |
| 1.1.18 | >10 - <30 |
| 1.1.19 | 0.24, 1.9[a] |
| 1.1.20 | 4.8 |
| 1.1.21 | >30 |
| 1.1.23 | >10 - <30 |
| 1.1.24 | 2.63 |
| 1.1.26 | >10 - <30 |
| 1.1.27 | >30 |
| 1.1.41 | >10 - <30 |
| 1.1.42 | >30 |
| 1.1.43 | >30 |
| 1.1.44 | >30 |
| 1.1.45 | >30 |
| 1.1.46 | >30 |
| 1.1.48 | 0.43 |
| 1.1.49 | >2 - <10 |
| 1.1.50 | >2 - <10 |
| 1.1.51 | >2 - <10 |
| 1.2.1 | 5.15 |
| 1.2.3 | >2 - <10 |
| 1.2.4 | >2 - <10 |
| 1.3.1 | >2 - <10 |
| 1.3.2 | >2 - <10 |
| 1.3.3 | >2 - <10 |
| 2.1.1 | >2 - <10 |
| 2.1.2 | >2 - <10 |
| 2.1.3 | >2 - <10 |
| 2.2.1 | -10 |
| 2.2.3 | >2 - <10 |
| [(a)] Inhibitory activity of HIV-1 (NL4.3 strain) carrying the GFP reporter gene (NL4.3-GFP) in the SupT1 cell line. | |

*6. Inhibitory activity of HIV-1 (NL4.3 strain) carrying the GFP reporter gene (NL4.3-GFP) in the SupT1 cell line.*

The evaluation was done by RetroVirox Inc. (San Diego, CA, USA). SupT1 cells were infected with the HIV NL4.3 strain carrying the green fluorescent protein gene (NL4.3-GFP). The virus sample was produced by transfection of 293T cells with proviral DNA. The sample was frozen 48 hours after transfection and stored until use. To enhance the efficiency of infection, a suspension of SupT1 cells was precipitated from the infectious mixture by centrifugation. Test substances were added to the cells immediately before adding the virus. After 2 hours of incubation, the infectious

mixture was replaced with a fresh culture medium containing test samples. The effectiveness of the infection was determined after 45 hours by counting the percentage of fluorescent cells in comparison with uninfected cell cultures. The cytotoxicity of the test compounds was determined in parallel on the same, but not infected, SupT1 cell line using the XTT reagent. Serial tenfold dilutions of the samples (starting from 10 μM when determining antiviral activity or from 100 μM for determining cytotoxicity). As a negative control, 0.1% DMSO was used. The values of $EC_{50}$ were calculated. The quality of the tests was determined using the following controls: signal-to-noise ratio, integrase inhibitor raltegravir (1 μM), and test reproducibility. The results are presented in Table 1.

**Example 11. Evaluation of thermodynamic solubility of compounds of general formula 1 or 2.**

The test compound of general formula 1 or 2 (2 mg) was weighed into a glass vial. The pION universal buffer (0.5 ml) with pH 7.4 or deionized water was added to appropriate vials. The vials were tightly closed with a lid, placed on a rotating shaker, and incubated for 24 hours to balance the solution and the solid phase at the saturation point. Each resulting solution (200 μl) was taken (in duplicate) from the vials, placed in a 96-well MultiScreen Solubility Filter Plate (Millipore), and filtered using vacuum (10"Hg) into a polypropylene plate with a U-shaped bottom. If necessary, the filtrates were diluted with an appropriate buffer (water). Then, 120 μl of the filtarate (or a diluted filtrate) was transferred to a 96-well plate with a UV-transparent bottom and 60 μl/well of acetonitrile and 20 μl/well of DMSO were added and thoroughly mixed with a multichannel pipette (acetonitrile and DMSO were added to obtain a required composition of calibration standards, as described below). The optical density of the resulting solutions was measured on a multifunction Infinite 200 PRO plate reader in the wavelength range from 240 nm to 400 nm in steps of 10 nm. The value of $OD_{\lambda}$ for solutions was assessed at a wavelength chosen for plotting a calibration curve.

The concentration of a substance in the solution after the solubility test was evaluated using a calibration curve. A stock solution in DMSO with a concentration of 2 mg/ml was prepared from a separate sample. Using the serial dilutions (step 2), six 10-fold solutions in DMSO were prepared, which were then diluted 10 times in an appropriate buffer with 30% acetonitrile to ensure complete solubility of the test compound in the mixture. The range of final concentrations of the calibration curve was 0.2; 0.1; 0.05; 0.025; 0.0125; 0.00625, and 0 mg/ml. Calibration solutions were prepared in a 96-well UV plate as follows: 20 μl of a 10-fold stock solution in DMSO, 60 μl of acetonitrile, and 120 μl of the appropriate buffer were added to the well and stirred. The optical density of the resulting solutions was measured on a multifunction Infinite 200 PRO plate reader in the wavelength range from 240 nm to 400 nm with a step of 10 nm. The opimal wavelength typically corresponding to the absorption maximum was chosen, and a linear dependence of $OD_{\lambda}$ at a given wavelength on the concentration of the compound was obtained.

The concentration of the compound in the filtrate after the solubility test was calculated according to the formula:

$$\text{shake-flask solubility} = (OD_{\lambda}\ \text{filtrate} - OD_{\lambda}\ \text{blank}) / (\text{slope} \times 1.67 \times \text{filtrate dilution})$$

wherein slope means the slope of the calibration curve; 1.67 is the filtrate : acetonitrile+DMSO dilution ratio; filtrate dilution is an additional dilution of the filtrate, if applicable. Data on the thermodynamic solubility of compounds of general formulas 1 and 2 are presented in Table 2.

**Table 2.** Thermodynamic solubility of compounds of general formulas 1 and 2

| Compound | Wavelength, nm (water/pH 7.4) | Water | | pH 7.4 | |
|---|---|---|---|---|---|
| | | Solubility, mg/ml | | Solubility, mg/ml | |
| | | value | SD | value | SD |
| **Cabotegravir** (CAB) | 290/280 | 0.008 | 0.000 | 0.005 | 0.000 |
| 1.1.1 | 320/290 | 0.033 | 0.000 | 0.024 | 0.000 |
| 1.1.7 | 320/280 | 0.318 | 0.053 | 0.318 | 0.015 |
| 1.1.17 | 320/290 | 0.131 | 0.004 | 0.196 | 0.011 |
| 1.1.19 | 280/280 | 0.269 | 0.003 | 0.232 | 0.002 |
| 1.1.20 | 310/280 | 2.679 | 0.090 | 1.684 | 0.056 |
| 1.1.23 | 320/280 | 0.078 | 0.000 | 0.091 | 0.002 |
| 1.1.24 | 320/280 | 0.347 | 0.011 | 0.380 | 0.002 |

(continued)

| Compound | Wavelength, nm (water/pH 7.4) | Water | | pH 7.4 | |
|---|---|---|---|---|---|
| | | Solubility, mg/ml | | Solubility, mg/ml | |
| | | value | SD | value | SD |
| 1.1.36 | 320/290 | 0.056 | 0.000 | 0.070 | 0.000 |
| 1.1.37 | 310/270 | 0.021 | 0.002 | 0.021 | 0.001 |
| 1.1.48 | LC-MSMS | 0.00285 | 0.0000118 | 0.0032 | 0.0000118 |
| 1.1.51 | 320/270 | 0.031 | 0.001 | 0.008 | 0.000 |
| 1.1.54 | 320/300 | 0.010 | 0.000 | 0.006 | 0.000 |
| 1.2.1 | 320/280 | 0.057 | 0.003 | 0.080 | 0.000 |

**Example 12. The pharmacokinetics of compounds of general formula 1.1.**

The research was carried out on male Sprague-Dawley rats obtained from Charles River Laboratories (Germany).

The studies were carried out on male Sprague-Dawley rats obtained from Charles River nursery (Germany). The age of the animals at the time of administration was 8-10 weeks. The animals were pre-catheterized so that all blood samples during the study of each substance could be taken from the same animals. Each substance was tested in 3 animals at each route of administration.

The solution of test substances with a concentration of 0.2 mg/ml in 10% DMSO/10% Solutol/80% 0.05M N-methylglucamine was administered to rats intravenously in the tail vein at a dose of 1 mg/kg. Blood samples were collected at 5, 15, 30 min, 1, 2, 4, 8, and 24 hours after administration. Intragastric administration involved gavage using a solution with a concentration of 0.5 mg/ml in 0.5% methylcellulose at a dose of 5 mg/kg. Blood samples were collected at 15, 30 min, 1, 2, 4, 8, and 24 hours after administration. Blood (0.2 ml) was collected in polypropylene tubes containing 20 μl of 5% NaEDTA. Blood plasma was separated by centrifugation at 1500 x g for 10 min and stored until testing at a temperature of -80°C.

A pure acetonitrile-water (1:1) mixture (5 μl) was added to 45 μl of test samples. The samples were vortexed thoroughly for 10 seconds. Tolbutamide (100 μl) chilled to +4°C (200 ng/ml in MeCN) was added to the samples asnd the mixture was vortexed. The samples were precipitated on ice at +4°C for 15 minutes and then centrifuged at 2700 g for 10 min at +4°C. Supernatants (100 μl each) were transferred to a blank plate for HPLC/MS/MS analysis.

The HPLC/MS/MS analysis was performed using an Agilent 1290 Infinity system cupled to an AB Sciex QTrap 6500 mass spectrometer.

Pharmacokinetic analysis of plasma concentration vs time data was performed by a model-independent method using the Phoenix ™ WinNonlin® 6.3 software (Pharsight Corp.). The following pharmacokinetic parameters were calculated (Table 3): maximum concentration in the liver ($C_{max}$) and time to reach it ($T_{max}$), half-life ($T_{1/2}$), the area under the PC curve ($AUC_{0-t}$, $AUC_{0-inf}$), clearance (Cl), and elimination constant (kel).

**Table 3.** Pharmacokinetics of compound 1.1.19 and CAB in rat plasma when administered intravenously (IV) and orally (Oral).

| Parameter | Unit of measuremen t | IV (dose: 1 mg/kg) | | Oral (dose: 5 mg/kg) | |
|---|---|---|---|---|---|
| | | 1.1.1 9 | CAB **Cabotegravi r** | 1.1.1 9 | CA B |
| $AUC_{INF}$ | h·ng/ml | 367627 | 232346 | 282070 | 158201 |
| $AUC_{last}$ | h·ng/ml | 202189 | 140844 | 176506 | 62277 |
| $C_{max}$ | ng/ml | 18550 | 16983 | 11488 | 5213 |
| $C_{8h}$ | ng/ml | | | 7913 | 4338 |
| $C_{24h}$ | ng/ml | | | 4435 | 1845 |
| kel | 1/h | 0.03 | 0.04 | 0.04 | 0.04 |
| M $RT_{last}$ | h | 9.46 | 9.19 | 9.42 | 8.05 |

(continued)

| Parameter | Unit of measuremen t | IV (dose: 1 mg/kg) | | Oral (dose: 5 mg/kg) | |
|---|---|---|---|---|---|
| | | 1.1.1 9 | CAB **Cabotegravi r** | 1.1.1 9 | CA B |
| $T_{1/2}$ | h | 21.2 | 18.3 | 16.5 | 19.1 |
| $T_{max}$ | h | 0.08 | 0.08 | 3 | 4.67 |
| F | % | | | 17.5 | 8.8 |

**Example 13. Evaluation of stability in biological media of compositions comprising compounds of general formulas 1 and 2.**

*a) Stability in blood plasma.* A test compound at a final concentration of 1 μM was incubated in pooled human blood plasma (Innovative Research). Incubation was carried out in a VorTemp 56 shaking incubator at 37°C with stirring at 300 rpm. At certain intervals (0, 0.25, 0.5, 1, 2, 4, 6, 8, and 24 h30 μl of samples were taken, and the reaction was stopped by adding 180 μl of cold acetonitrile containing an internal standard to the selected sample. The samples were then centrifuged for 10 min at 3000 rpm, and 150 μl of the supernatant was taken for analysis. Incubation was performed in duplicate, each sample was measured twice. Samples were analyzed by HPLC-MS/MS developed for each prodrug tested using a 1290 Infinity II chromatographic system (Agilent Technologies) coupled to a QTRAP5500 tandem mass spectrometer (AB Sciex). When developing conditions for mass spectrometric detection, the solutions of tested compounds in a 1:1 acetonitrile-water mixture with a concentration of 100 ng/ml were analyzed by direct injection into the mass spectrometer by a syringe pump using electrospray ionization in positive ion mode. When scanning in the total ion current mode (MS1), the molecular ion for each compound was determined, the main product ions were recorded in the MS2 mode. Then, the MS/MS technique was optimized in the MRM mode to achieve maximum sensitivity. In the quantitative processing of chromatograms, the most intense MRM transition was used for the analyte and the internal standard. Chromatographic separation was performed on a YMC Triart C18, 50 x 2.mm, 1.9 μm column in a gradient elution mode in the mobile phase with a composition of 0.1% formic acid in water - 0.1% formic acid in acetonitrile. Tolbutamide (Fluka) was used as an internal standard. The half-life ($T_{1/2}$) was calculated from the kinetics of the loss of the tested prodrug in the antiviral composition during incubation in the biological medium.

In calculations, the values of the areas of chromatographic peaks of substances in the experimental samples normalized to the signal of the internal standard were used. The elimination rate constant (k is the slope of the linear section) was calculated from the linear dependence of the log-normalized areas of chromatographic peaks on time. Then, the half-life was calculated: $T_{1/2}$ = 0.693/k. It was found (Figure 2) that the compounds of general formula 1.1 are stable in human and rat plasma.

*b) Stability in the S9 fraction.* The reaction mixture was prepared in 0.1 M potassium phosphate buffer (pH 7.4 BD Gentest) in a total final volume of 250 μl and contained 1 mM of NADPH tetrasodium salt (AppliChem), 7 mM of glucose-6-phosphate sodium salt (Sigma), 1.5 U/ml of glucose-6-phosphate dehydrogenase (Sigma), 3.3 mM of $MgCl_2$ (Sigma), 5 mM of uridine-5-diphosphate-glucuronic acid trisodium salt (UDPGA, Sigma), and 1 μM of the test compound. The metabolic reaction was initiated by adding a suspension of the S9 human (BD Gentest) or rat liver fraction, the final protein concentration was 1 mg/ml. The reaction mixture was incubated at 37°C on a shaker (Vortemp56) with stirring at 400 rpm. At certain time intervals (0, 0.25, 0.5, 1, 2, 4, 6, 8, 24 h), 30-μl samples were taken, and the reaction was stopped by adding to the selected sample 180 μl of cold acetonitrile containing the internal standard. Proteins were precipitated on ice for 15 min, the samples were centrifuged for 10 min at 3000 rpm, and 150 μl of the supernatant was collected for analysis. Incubation was performed in duplicate with measuring each sample twice. It was established (Figure 3) that the compounds of general formula 1.1 are stable in the S9 fraction of human or rat liver.

**Example 14. Plasma protein binding of compounds 1.1.17 and 1.1.19.**

To assess protein binding, pooled samples of blood plasma diluted with phosphate buffer up to 50% were used. The test was carried out in a teflon coated 48-well dialysis plate. Each well contained two separate chambers separated by a vertical semipermeable dialysis membrane with 8 kDa pores. A plasma sample with 1 μM of the test compound was placed into one of the chambers, and a buffer solution (pH= 7.2) was placed in the other chamber. Over time, at 37°C while rocking, passive diffusion of the unbound compound occurred and after 4 hours the equilibrium state between the chambers with plasma and the buffer solution was reached. The amount of free fraction was evaluated by HPLC-MS/MS following precipitation of the proteins by acetonitrile. Also, the study involved evaluation of stability of the substance during the 4-hour period and passive permeability through the dialysis membrane in the buffer by mass balance.

Warfarin was used as the control compound. The results are presented in Table 4, from which it follows that the compounds of general formula 1.1 have a high degree of binding to human and rat plasma proteins.

**Table 4.** Binding of compounds **1.1.17** and **1.1.19** to human and rat plasma proteins

| Type | Compound | Permeability, % | Extraction, % | Bound, % | Binding class |
|---|---|---|---|---|---|
| Human | **1.1.17** | 84.8 | 94.6 | 98.8 | High |
| Rat | **1.1.17** | 90.9 | 108 | 99.9 | High |
| Human | **1.1.19** | 84.0 | 95.0 | 99.0 | High |
| Rat | **1.1.19** | 83.8 | 94.5 | 98.9 | High |

**Industrial applicability**

This invention can be applied in human and veterinary medicine.

**Claims**

1. Annelated 9-hydroxy-1,8-dioxo-1,3,4,8-tetrahydro-2H-pyrido[1,2-a]pyrazine-7-carboxamide of general formulas 1 and 2, or its stereoisomer, pharmaceutically acceptable salt, solvate, crystalline or polycrystalline form thereof

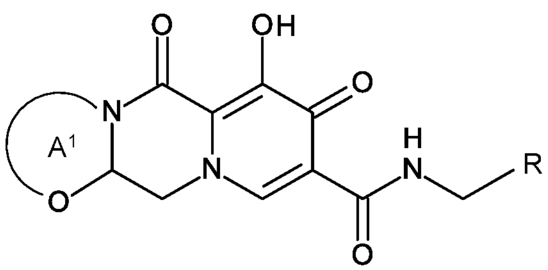

,

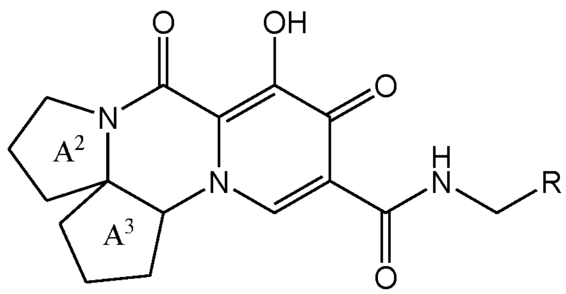

**1** **2**

wherein

ring $A^1$ is an optionally methyl substituted 5-7 membered saturated heterocycle or heterobicycle,
ring $A^2$ is an optionally methyl substituted 5-6 membered saturated or partially monocyclic heterocycle,
ring $A^3$ is a 5-6 membered monocyclic saturated cycloalkane and tetrahydro-2H-pyran,
R is a 5-7-membered, optionally substituted with one, two or three optionally identical substituents, monocyclic or bicyclic heterocyclic radical containing 1-4 heteroatoms selected from the series O, S, and N, except (2*S*,5*R*,13a*S*)-8-hydroxy-7,9-dioxo-*N*-{[3-(trifluoromethyl)-pyridin-2-yl]methyl}-2,3,4,5,7,9,13,13a-octahydro-2,5-methanopurido[1',2':4,5]pyrazino[2,1-*b*][1,3]oxazepine-10-carboxamide (formula A4) and (1*R*,4*S*,12a*R*)-*N*-[(3,5-difluoropyridin-2-yl)methyl]-7-hydroxy-6,8-dioxo-1,2,3,4,6,8,12,12a-octahydro-1,4-methanodipyrido[1,2-a:1',2'-d]pyrazine-9-carboxamide (formula A5).

2. The compound of claim 1 wherein the heterocyclic radical R is thienyl, furyl, pyrazolyl, isoxazolyl, thiazolyl, oxazolyl, imidazolyl, thiadiazolyl, [1,2,5]oxadiazolyl, [1,2,4]oxadiazolyl, [1,2,4]triazolyl, tetrazolyl, pyridinyl, pyridazinyl, pirimidinyl, pyrazinyl, 1,2,3-triazinyl, 1,2,4-triazinyl, and 1,3,5-triazinyl, imidazo[2,1-b]thiazolyl, imidazo[2,1-b][1,3,4]thiadiazolyl, benzothiophenyl, benzofuranyl, indolyl, 1,3-benzodioxol-5-yl, 2,3-dihydro-1,4-benzodioxin-6-yl, 1,3-benzothiazolyl, 1,3-benzoxazolyl, benzimidazolyl, 1,3-dihydro-2trifluorobenzimidazolyl, 2,1,3-benzothiadiazolyl, 2,1,3-benzoxadiazolyl, quinolinyl, isoquinolinyl, imidazo[1,2-a]pyridinyl, 1,2,4-triazolo[4,3-a]pyridinyl, imidazo[1,2-a]pirimidinyl, imidazo[1,2-a]pyrazinyl, 1,2,4-triazolo[4,3-b]pyridazinyl, 4,5,6,7-tetrahydrobenzothiophenyl, 5,6,7,8-tetrahydro-[1,2,4]triazolo[4,3-a]pyridinyl, 1,4,5,6,7,8-hexahydrocyclohepta[c]pyrazolyl, 5,6,7,8-tetrahydro-4H-cyclohepta[d]thiazolyl, 5,6,7,8-tetrahydro-4*H*-cyclohepta[*d*]isothiazol-3-yl, 5,6,7,8-tetrahydro-4*H*-cyclohepta[*d*]isothiazol-3-yl, [1,2,4]triazolo[4,3-*b*]pyridazin-3-yl.

3. The compound of claim 1 or 2 wherein the heterocyclic radical R is 2-thienyl, 2-furyl, 1H-pyrazol-3-yl, 1H-pyrazol-4-yl, 1H-pyrazol-5-yl, isoxazol-4-yl, thiazol-2-yl, 1,3-oxazol-2-yl, imidazol-2-yl, 1,2,3-thiadiazol-5-yl, 1,2,5-oxadiazol-3-yl, 1,2,4-oxadiazol-5-yl, 1H-1,2,4-triazol-3-yl, 1H-1,2,3,4-tetrazol-5-yl, 2-pyridyl, 3-pyridyl, 4-pyridyl, pyridazin-4-yl, pyrimidin-4-yl, pyrazin-2-yl, imidazo[2,1-b]thiazol-6-yl, imidazo[2,1-b][1,3,4]thiadiazol-6-yl, benzothiophen-5-yl, benzofuran-2-yl, 1H-indol-5-yl, 1,3-benzodioxol-5-yl, 2,3-dihydro-1,4-benzodioxin-6-yl, 1,3-benzothiazol-2-yl, 1,3-benzoxazol-2-yl, 1H-benzimidazol-2-yl, 1,3-dihydro-2-oxobenzimidazol-5-yl, 2,1,3-benzothiadiazol-5-yl, 2,1,3-benzoxadiazol-5-yl, quinolin-2-yl, quinolin-3-yl, quinolin-4-yl, quinolin-5-yl, quinolin-6-yl, quinolin-7-yl, quinolin-8-yl, 1-isoquinolinyl, imidazo[1,2-a]pyridin-3-yl, 1,2,4-triazolo[4,3-a]pyridin-3-yl, imidazo[1,2-a]pyrimidin-2-yl, imidazo[1,2-a]pyrazin-3-yl, 1,2,4-triazolo[4,3-b]pyridazin-3-yl, 4,5,6,7-tetrahydrobenzothiophen-2-yl, 5,6,7,8-tetrahydro-[1,2,4]triazolo[4,3-a]pyridin-3-yl, 1,4,5,6,7,8-hexahydrocyclohepta[c]pyrazol-3-yl, 5,6,7,8-tetrahydro-4*H*-cyclohepta[*d*][1,3]thiazol-2-yl, 5,6,7,8-tetrahydro-4*H*-cyclohepta[*d*]isothiazol-3-yl, 5,6,7,8-tetrahydro-4*H*-cyclohepta[*d*]isothiazol-3-yl, [1,2,4]triazolo[4,3-*b*]pyridazin-3-yl.

4. The compound according to any one of claims 1, 2, or 3 wherein the heterocyclic radical R is substituted with one, two, or three substituents selected from C1-C3 alkyl or halogen atoms, preferably, F, Cl, and Br.

5. The compound according to any one of claims 1, 2, 3, or 4 of general formula 1.1, 1.2 or 1.3, its stereoisomer, pharmaceutically acceptable salt, solvate, crystalline or polycrystalline form thereof

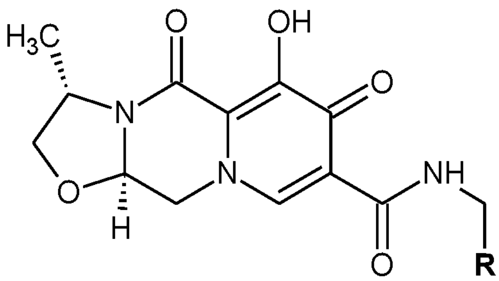

**1.1**

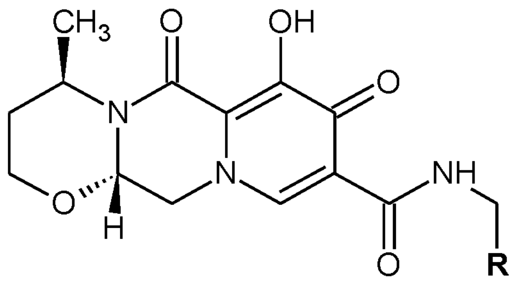

**1.2**

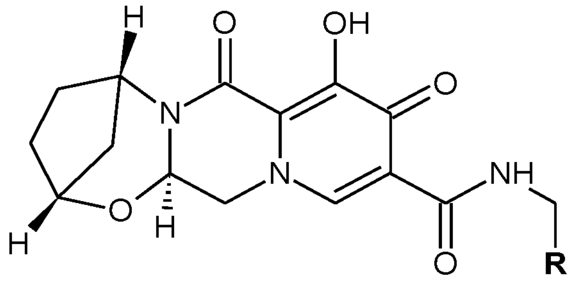,

**1.3**

wherein R is as given above.

6. The compound according to claim 1 of general formula 2.1 or 2.2, its stereoisomer, pharmaceutically acceptable salt, solvate, crystalline or polycrystalline form thereof

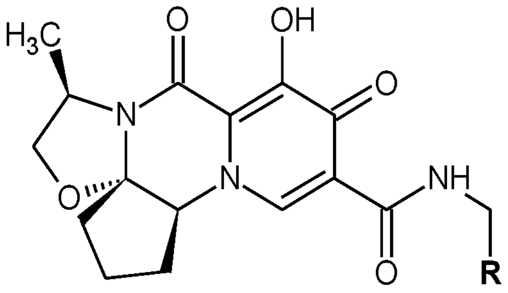

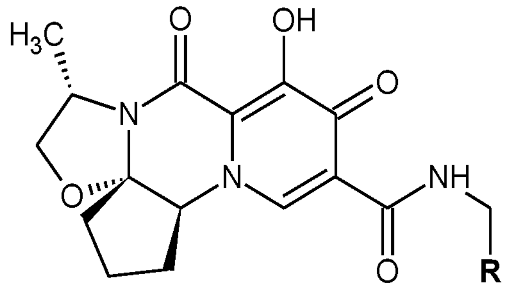

**2.1** **2.2** ,

wherein R is as given above.

**7.** The compound according to claim 1 selected from the series:

| |
|---|
| (3*S*,11*aR*)-6-hydroxy-3-methyl-5,7-dioxo-*N*-(2-thienylmethyl)-2,3,5,7,11,11*a*-hexahydro[1,3] oxazolo[3,2-a] pyrido[1,2-*d*]pyrazine-8-carboxamide (1.1.1), |
| (3*S*,11*aR*)-*N*-[(5-bromo-2-thienyl)methyl]-6-hydroxy-3-methyl-5,7-dioxo-2,3,5,7,11,11*a*-hexahydro[1,3]oxazolo [3,2-a]pyrido[1,2-*d*]pyrazine-8-carboxamide (1.1.2), |
| (3*S*,11*aR*)-6-hydroxy-3-methyl-*N*-[(5-methyl-2-furyl)methyl]-5,7-dioxo-2,3,5,7,11,11*a*-hexahydro[1,3]oxazolo [3,2-a]pyrido[1,2-*d*]pyrazine-8-carboxamide (1.1.3), |
| (3*S*,11*aR*)-6-hydroxy-*N*-[(3,5-dimethyl-1H-pyrazol-4-yl)methyl]-3-methyl-5,7-dioxo-2,3,5,7,11,11*a*-hexahydro [1,3]oxazolo[3,2-*a*]pyrido[1,2-*d*]pyrazine-8-carboxamide (1.1.4), |
| (3*S*,11*aR*)-6-hydroxy-3-methyl-*N*-[(1,3,5-trimethyl-1H-pyrazol-4-yl)methyl]-5,7-dioxo-2,3,5,7,11,11*a*-hexahydro [1,3]oxazolo[3,2-*a*]pyrido[1,2-*d*]pyrazine-8-carboxamide (1.1.5), |
| (3*S*,11*aR*)-6-hydroxy-*N*-[(1-ethyl-3,5-dimethyl-1H-pyrazol-4-yl)methyl]3-methyl-5,7-dioxo-2,3,5,7,11,11*a*-hexahydro[1,3]oxazolo[3,2-*a*]pyrido[1,2-*d*]pyrazine-8-carboxamide (1.1.6), |
| (3*S*,11a*R*)-6-hydroxy-*N*-[(4,5-диchloro-1-methyl-1H-pyrazol-3-yl)methyl]3-methyl-5,7-dioxo-2,3,5,7,11,11*a*-hexahydro[1,3]oxazolo[3,2-*a*]pyrido[1,2-*d*]pyrazine-8-carboxamide (1.1.7), |
| (3*S*,11*aR*)-6-hydroxy-3-methyl-*N*-(thiazol-2-ylmethyl)-5,7-dioxo-2,3,5,7,11,11*a*-hexahydro-[1,3]oxazolo[3,2-a] pyrido[1,2-*d*]pyrazine-8-carboxamide (1.1.8), |
| (3*S*,11*aR*)- 6-hydroxy-3-methyl-*N*-[(4-methyl-1,3-oxazol-2-yl)methyl]-5,7-dioxo-2,3,5,7,11,11*a*-hexahydro[1,3] oxazolo[3,2-*a*]pyrido[1,2-*d*]pyrazine-8-carboxamide (1.1.9), |
| (3*S*,11*aR*)-6-hydroxy-3-methyl-*N*-[(1,4,5-trimethyl-1H-imidazol-2-yl)methyl]-5,7-dioxo-2,3,5,7,11,11*a*-hexahydro [1,3]oxazolo[3,2-a]pyrido[1,2-*d*]pyrazine-8-carboxamide (1.1.10), |
| (3*S*,11*aR*)-6-hydroxy-3-methyl-*N*-[(4-methyl-1,2,3-thiadiazol-5-yl)methyl]-5,7-dioxo-2,3,5,7,11, 11*a*-hexahydro [1,3]oxazolo[3,2-a]pyrido[1 ,2-*d*]pyrazine-8-carboxamide (1.1.11), |
| (3*S*,11*aR*)-6-hydroxy-3-methyl-*N*-[(4-methyl-1,2,5-oxadiazol-3-yl)methyl]-5,7-dioxo-2,3,5,7,11,11*a*-hexahydro [1,3]oxazolo[3,2-*a*]pyrido[1,2-*d*]pyrazine-8-carboxamide (1.1.12), |
| (3*S*,11*aR*)-6-hydroxy-3-methyl-*N*-[(3-methyl-1,2,4-oxadiazol-5-yl)methyl]-5,7-dioxo-2,3,5,7,11, 11*a*-hexahydro [1,3]oxazolo[3,2-a]pyrido[1 ,2-*d*]pyrazine-8-carboxamide (1.1.13), |
| (3*S*,11*aR*)-6-hydroxy-3-methyl-*N*-[(4-methyl-4H-1,2,4-triazol-3-yl)methyl]-5,7-dioxo-2,3,5,7,11,11*a*-hexahydro [1,3]oxazolo[3,2-*a*]pyrido[1,2-*d*]pyrazine-8-carboxamide (1.1.14), |
| (3*S*,11*aR*)-6-hydroxy-3-methyl-*N*-(pyridyl-2-ylmethyl)-5,7-dioxo-2,3,5,7,11,11a-hexahydro[1,3]-oxazolo[3,2-a] pyrido[1,2-*d*]pyrazine-8-carboxamide (1.1.15), |
| (3*S*,11*aR*)-6-hydroxy-3-methyl-*N*-[(3-fluoropyridin-2-yl)methyl]-5,7-dioxo-2,3,5,7,11,11a-hexahydro[1,3]oxazolo [3,2-a]pyrido[1,2-*d*]pyrazine-8-carboxamide (1.1.16), |

(continued)

| |
|---|
| (3*S*,11*aR*)-6-hydroxy-*N*-[(3,5-difluoropyridin-2-yl)methyl]-3-methyl-5,7-dioxo-2,3,5,7,11,11*a*-hexahydro[1,3]oxazolo[3,2-a]pyrido[1,2-*d*]pyrazine-8-carboxamide (1.1.17), |
| (3*S*,11*aR*)-6-hydroxy-*N*-[(6-chloropyridin-3-yl)methyl]-3-methyl-5,7-dioxo-2,3,5,7,11,11a-hexahydro[1,3]oxazolo[3,2-a]pyrido[1,2-*d*]pyrazine-8-carboxamide (1.1.18), |
| (3*S*,11*aR*)-6-hydroxy-*N*-[(2,6-difluoropyridin-3-yl)methyl]-3-methyl-5,7-dioxo-2,3,5,7,11,11a-hexahydro[1,3]oxazolo[3,2-a]pyrido[1,2-*d*]pyrazine-8-carboxamide (1.1.19), |
| Sodium (3S, 11aR)-8-({[(2,6-difluoropyridin-3-yl)methyl]amino}carbonyl)-3-methyl-5,7-dioxo-2,3,5,7,11, 11a-hexahydro[1,3]oxazolo[3,2-a]pyrido[1,2-d]pyrazin-6-olate (1.1.20), |
| (3*S*,11*aR*)-6-hydroxy-*N*-[(2,4-difluoropyridin-3-yl)methyl]-3-methyl-5,7-dioxo-2,3,5,7,11,11*a*-hexahydro[1,3]oxazolo[3,2-*a*]pyrido[1,2-*d*]pyrazine-8-carboxamide (1.1.21), |
| (3*S*,11*aR*)-6-hydroxy-3-methyl-*N*-[(2,4,6-trifluoropyridin-3-yl)methyl]-5,7-dioxo-2,3,5,7,11,11*a*-hexahydro[1,3]oxazolo[3,2-*a*]pyrido[1,2-*d*]pyrazine-8-carboxamide (1.1.22), |
| (3*S*,11*aR*)-6-hydroxy-3-methyl-*N*-[(3-fluoropyridin-4-yl)methyl]-5,7-dioxo-2,3,5,7,11,11*a*-hexahydro[1,3]oxazolo[3,2-*a*]pyrido[1,2-*d*]pyrazine-8-carboxamide (1.1.23), |
| (3*S*,11*aR*)-6-hydroxy-*N*-[(3,5-difluoropyridin-4-yl)methyl]-3-methyl-5,7-dioxo-2,3,5,7,11,11*a*-hexahydro[1,3]oxazolo[3,2-*a*]pyrido[1,2-*d*]pyrazine-8-carboxamide (1.1.24), |
| (3*S*,11*aR*)-6-hydroxy-3-methyl-*N*-(pyridazin-4-ylmethyl)-5,7-dioxo-2,3,5,7,11,11*a*-hexahydro[1,3]oxazolo[3,2-*a*]pyrido[1,2-*d*]pyrazine-8-carboxamide (1.1.25), |
| (3*S*,11*aR*)-6-hydroxy-3-methyl-*N*-[(2-methylpyrimidin-4-yl)methyl]-5,7-dioxo-2,3,5,11,11*a*-hexahydro[1,3]oxazolo[3,2-*a*]pyrido[1,2-*d*]pyrazine-8-carboxamide (1.1.26), |
| (3*S*,11*aR*)-6-hydroxy-3-methyl-*N*-(pyrazin-2-ylmethyl)-5,7-dioxo-2,3,5,7,11,11*a*-hexahydro[1,3]oxazolo[3,2-*a*]pyrido[1,2-*d*]pyrazine-8-carboxamide (1.1.27), |
| (3*S*,11*aR*)-6-hydroxy-3-methyl-*N*-[(2-chloroimidazo[2,1-b][1,3]thiazol-6-yl)methyl]-5, 7-dioxo-2,3,5,7,11,11*a*-hexahydro[1,3]oxazolo[3,2-*a*]pyrido[1,2-*d*]pyrazine-8-carboxamide (1.1.28), |
| (3*S*,11*aR*)-6-hydroxy-*N*-(imidazo[2,1-b][1,3,4]thiadiazol-6-ylmethyl)-3-methyl-5,7-dioxo-2,3,5,7,11,11*a*-hexahydro[1,3]oxazolo[3,2-*a*]pyrido[1,2-*d*]pyrazine-8-carboxamide (1.1.29), |
| (3*S*,11*aR*)-6-hydroxy-3-methyl-*N*-[(2-methylimidazo[2,1-b][1,3,4]thiadiazol-6-yl)methyl]-5,7-dioxo-2,3,5,7,11,11*a*-hexahydro[1,3]oxazolo[3,2-*a*]pyrido[1,2-*d*]pyrazine-8-carboxamide (1.1.30), |
| (3*S*,11*aR*)-*N*-(1-benzothien-5-ylmethyl)-6-hydroxy-3-methyl-5, 7-dioxo-2,3,5,7,11,11*a*-hexahydro[1,3]oxazolo[3,2-*a*]pyrido[1,2-*d*]pyrazine-8-carboxamide (1.1.31), |
| (3*S*,11*aR*)-*N*-(1-benzofuran-2-ylmethyl)-6-hydroxy-3-methyl-5,7-dioxo-2,3,5,7,11,11*a*-hexahydro[1,3]oxazolo[3,2-*a*]pyrido[1,2-*d*]pyrazine-8-carboxamide (1.1.32), |
| (3*S*,11*aR*)-6-hydroxy-3-methyl-*N*-[(1-methyl-1H-indol-5-yl)methyl]-5,7-dioxo-2,3,5,7,11,11*a*-hexahydro[1,3]oxazolo[3,2-a]pyrido[1,2-*d*]pyrazine-8-carboxamide (1.1.33), |
| (3*S*,11*aR*)-*N*-(1,3-benzodioxol-5-ylmethyl)-6-hydroxy-3-methyl-5, 7 -dioxo-2,3,5, 7,11,11*a*-hexahydro[1,3]oxazolo[3,2-*a*]pyrido[1,2-*d*]pyrazine-8-carboxamide (1.1.34), |
| (3*S*,11*aR*)-*N*-[(6-bromo-1,3-benzodioxol-5-yl)methyl]-6-hydroxy-3-methyl-5, 7-dioxo-2,3,5,7,11,11*a*-hexahydro[1,3]oxazolo[3,2-*a*]pyrido[1,2-*d*]pyrazine-8-carboxamide (1.1.35), |
| (3*S*,11*aR*)-6-hydroxy-3-methyl-*N*-[(7-methyl-2,3-dihydro-1,4-benzodioxin-6-yl)methyl]-5,7-dioxo-2,3,5,7,11,11*a*-hexahydro[1,3]oxazolo[3,2-*a*]pyrido[1,2-*d*]pyrazine-8-carboxamide (1.1.36), |
| (3*S*,11*aR*)-*N*-(1,3-benzothiazol-2-ylmethyl)-6-hydroxy-3-methyl-5,7-dioxo-2,3,5,7,11,11*a*-hexahydro[1,3]oxazolo[3,2-*a*]pyrido[1,2-*d*]pyrazine-8-carboxamide (1.1.37), |
| (3*S*,11*aR*)-6-hydroxy-3-methyl-*N*-[(5-chloro-1,3-benzoxazol-2-yl)methyl]-5,7-dioxo-2,3,5,7,11,11*a*-hexahydro[1,3]oxazolo[3,2-*a*]pyrido[1,2-*d*]pyrazine-8-carboxamide (1.1.38), |
| (3*S*,11*aR*)-6-hydroxy-3-methyl-*N*-[(6-chloro-1,3-benzoxazol-2-yl)methyl]-5,7-dioxo-2,3,5,7,11,11*a*-hexahydro[1,3]oxazolo[3,2-*a*]pyrido[1,2-*d*]pyrazine-8-carboxamide (1.1.39), |

(continued)

| |
|---|
| (3*S*,11*aR*)-6-hydroxy-3-methyl-*N*-[(5-methyl-1H-benzimidazol-2-yl)methyl]-5,7-dioxo-2,3,5,7,11,11*a*-hexahydro [1,3]oxazolo[3,2-*a*]pyrido[1,2-*d*]pyrazine-8-carboxamide (1.1.40), |
| (3*S*,11*aR*)-6-hydroxy-3-methyl-*N*-[(5-фТоро-1H-benzimidazol-2-yl)methyl]-5,7-dioxo-2,3,5,7,11,11*a*-hexahydro [1,3]oxazolo[3,2-*a*]pyrido[1,2-*d*]pyrazine-8-carboxamide (1.1.41), |
| (3*S*,11*aR*)-6-hydroxy-3-methyl-*N*-[(1-methyl-benzimidazol-2-yl)methyl]-5,7-dioxo-2,3,5,7,11,11*a*-hexahydro[1,3] oxazolo[3,2-*a*]pyrido[1,2-*d*]pyrazine-8-carboxamide (1.1.42), |
| (3*S*,11*aR*)-6-hydroxy-3-methyl-*N*-[(1-methyl-6-chlorobenzimidazol-2-yl)methyl]-5,7-dioxo-2,3,5,7,11,11*a*-hexahydro[1,3]oxazolo[3,2-*a*]pyrido[1,2-*d*]pyrazine-8-carboxamide (1.1.43), |
| (3*S*,11aR)-6-hydroxy-3-methyl-N-{[1isopropyl-1H-benzimidazol-2-yl]methyl}-5,7-dioxo-2,3,5,7,11,11a-hexahydro[1,3]oxazolo[3,2-a]pyrido[1,2-d]pyrazine-8-carboxamide_(1.1.44), |
| (3*S*,11*aR*)-6-hydroxy-3-methyl-*N*-[(6-chloro-1-isopropyl-1H-benzimidazol-2-yl)methyl]-5,7-dioxo-2,3,5,7,11,11*a*-hexahydro[1,3]oxazolo[3,2-a]pyrido[1,2-*d*]pyrazine-8-carboxamide (1.1.45), |
| (3*S*,11*aR*)-6-hydroxy-*N*-[(2-oxo-2,3-dihydro-1H-benzimidazol-5-yl)methyl]-3-methyl-5,7-dioxo-2,3,5,7,11,11*a*-hexahydro[1,3]oxazolo[3,2-*a*]pyrido[1,2-*d*]pyrazine-8-carboxamide (1.1.46), |
| (3*S*,11*aR*)-*N*-(2,1,3-benzothiadiazol-5-ylmethyl)-6-hydroxy-3-methyl-5,7-dioxo-2,3,5,7,11,11*a*-hexahydro[1,3] oxazolo[3,2-*a*]pyrido[1,2-*d*]pyrazine-8-carboxamide (1.1.47), |
| (3*S*,11*aR*)-*N*-(2,1,3-benzoxadiazol-5-ylmethyl)-6-hydroxy-3-methyl-*5*,7-dioxo-2,3,5,7,11,11*a*-hexahydro[1,3] oxazolo[3,2-*a*]pyrido[1,2-*d*]pyrazine-8-carboxamide (1.1.48), |
| (3*S*,11*aR*)-6-hydroxy-3-methyl-*N*-(quinolin-2-ylmethyl)-5,7-dioxo-2,3,5,7,11,11*a*-hexahydro[1,3]oxazolo[3,2-*a*] pyrido[1,2-*d*]pyrazine-8-carboxamide (1.1.49), |
| (3*S*,11*aR*)-6-hydroxy-3-methyl-*N*-(quinolin-3-ylmethyl)-5,7-dioxo-2,3,5,7,11,11*a*-hexahydro[1,3]oxazolo[3,2-*a*] pyrido[1,2-*d*]pyrazine-8-carboxamide (1.1.50), |
| (3*S*,11*aR*)-6-hydroxy-3-methyl-*N*-(2-methylquinolin-4-ylmethyl)-5,7-dioxo-2,3,5, 7,11,11*a*-hexahydro[1,3] oxazolo[3,2-*a*]pyrido[1,2-*d*]pyrazine-8-carboxamide (1.1.51), |
| (3*S*,11*aR*)-6-hydroxy-3-methyl-*N*-(quinolin-5-ylmethyl)-5,7-dioxo-2,3,5,7,11,11*a*-hexahydro[1,3]-oxazolo[3,2-*a*] pyrido[1,2-*d*]pyrazine-8-carboxamide (1.1.52), |
| (3*S*,11*aR*)-6-hydroxy-3-methyl-*N*-(quinolin-6-ylmethyl)-5,7-dioxo-2,3,5,7,11,11*a*-hexahydro[1,3]oxazolo[3,2-*a*] pyrido[1,2-*d*]pyrazine-8-carboxamide (1.1.53), |
| (3*S*,11*aR*)-6-hydroxy-3-methyl-*N*-(quinolin-8-ylmethyl)-5,7-dioxo-2,3,5,7,11,11*a*-hexahydro[1,3]oxazolo[3,2-*a*] pyrido[1,2-*d*]pyrazine-8-carboxamide (1.1.54), |
| (3*S*,11*aR*)-6-hydroxy-*N*-(isoquinolin-1-ylmethyl)-3-methyl-5,7-dioxo-2,3,5,7,11,11*a*-hexahydro[1,3]oxazolo[3,2-a]pyrido[1,2-*d*]pyrazine-8-carboxamide (1.1.55), |
| (3*S*,11*aR*)-6-hydroxy-*N*-(imidazo[1,2-*a*]pyridin-3-ylmethyl)-3-methyl-5, 7-dioxo-2,3,5, 7,11,11*a*-hexahydro[1,3] oxazolo[3,2-*a*]pyrido[1,2-*d*]pyrazine-8-carboxamide (1.1.56), |
| (3*S*,11*aR*)-6-hydroxy-3-methyl-*N*-(1,2,4-triazolo[4,3-a]pyridin-3-ylmethyl)-5,7-dioxo-2,3,5,7,11,11*a*-hexahydro [1,3]oxazolo[3,2-*a*]pyrido[1,2-*d*]pyrazine-8-carboxamide (1.1.57), |
| (3*S*,11*aR*)-6-hydroxy-*N*-(imidazo[1,2-a]pyrimidin-2-ylmethyl)-3-methyl-5, 7 -dioxo-2,3,5,7,11,11*a*-hexahydro[1,3] oxazolo[3,2-*a*]pyrido[1,2-*d*]pyrazine-8-carboxamide (1.1.58), |
| (3*S*,11*aR*)-6-hydroxy-*N*-(imidazo[1,2-a]pyrazin-3-ylmethyl)-3-methyl-5, 7 -dioxo-2,3,5, 7,11,11*a*-hexahydro[1,3] oxazolo[3,2-*a*]pyrido[1,2-*d*]pyrazine-8-carboxamide (1.1.59), |
| (3*S*,11*aR*)-6-hydroxy-3-methyl-*N*-[(6-chloro-1,2,4-triazolo[4,3-b]pyridazin-3-yl)methyl]-5,7-dioxo-2,3,5,7,11,11*a*-hexahydro[1,3]oxazolo[3,2-*a*]pyrido[1,2-*d*]pyrazine-8-carboxamide (1.1.60), |
| (3*S*,11*aR*)-6-hydroxy-3-methyl-*N*-(5,6,7,8-tetrahydro-[1,2,4]triazolo[4,3-a]pyridin-3-ylmethyl)-5,7-dioxo-2,3,5,7,11,11*a*-hexahydro[1,3]oxazolo[3,2-*a*]pyrido[1,2-*d*]pyrazine-8-carboxamide (1.1.61), |
| (3*S*,11*aR*)-6-hydroxy-*N*-(1,4,5,6,7,8-hexahydrocyclohepta[c]pyrazol-3-ylmethyl)-3-methyl-5,7-dioxo-2,3,5,7,11,11*a*-hexahydro[1,3]oxazolo[3,2-a]pyrido[1,2-*d*]pyrazine-8-carboxamide (1.1.62), |

(continued)

| |
|---|
| (3*S*,11a*R*)-6-hydroxy-3-methyl-5,7-dioxo-*N*-(5,6,7,8-tetrahydro-4*H*-cyclohepta[d*]*[1,3]thiazol-2-ylmethyl)-2,3,5,7,11,11*a*-hexahydro[1,3]oxazolo[3,2-*a*]pyrido[1,2-*d*]pyrazine-8-carboxamide (1.1.63), |
| (3*S*,11*aR*)-6-hydroxy-3-methyl-*N*-[(1,2-dimethyl-1H-benzimidazol-5-yl)methyl]-5,7-dioxo-2,3,5,7,11,11*a*-hexahydro[1,3]oxazolo[3,2-*a*]pyrido[1,2-*d*]pyrazine-8-carboxamide (1.1.64), |
| (3*S*,11*aR*)-6-hydroxy-3-methyl-*N*-(quinolin-4-ylmethyl)-5,7-dioxo-2,3,5,7,11,11*a*-hexahydro[1,3]oxazolo[3,2-*a*]pyrido[1,2-*d*]pyrazine-8-carboxamide (1.1.65), |
| (4*R*,12a*S*)-7-hydroxy-*N*-[(2,6-difluoropyridin-3-yl)methyl]-4-methyl-6,8-dioxo-3,4,6,8,12,12a-hexahydro-2*H*-pyrido[1',2':4,5]pyrazino [2,1-*b*][1,3]oxazine-9-carboxamide (1.2.1), |
| (4*R*,12a*S*)-7-hydroxy-*N*-[(2,4-difluoropyridin-3-yl)methyl]-4-methyl-6,8-dioxo-3,4,6,8,12,12a-hexahydro-2*H*-pyrido[1',2':4,5]pyrazino [2,1-*b*][1,3]oxazine-9-carboxamide (1.2.2), |
| (4*R*,12a*S*)-7-hydroxy-4-methyl-*N*-[(2,4.6-trifluoropyridin-3-yl)methyl]-6,8-dioxo-3,4,6,8,12,12a-hexahydro-2*H*-pyrido[1',2':4,5]pyrazino[2,1-*b*][1,3]oxazine-9-carboxamide (1.2.3), |
| (4*R*,12a*S*)-7-hydroxy-*N*-[(3,5-difluoropyridin-4-yl)methyl]-4-methyl-6,8-dioxo-3,4,6,8,12,12a-hexahydro-2*H*-pyrido[1',2':4,5]pyrazino[2,1-*b*][1,3]oxazine-9-carboxamide (1.2.4), |
| (2*R*,5*S*,13a*R*)-8-hydroxy-*N*-[(2,6-difluoropyridin-3-yl)methyl]-7,9-dioxo-2,3,4,5,7,9,13,13a-octahydro-2,5-methanopyrido[1',2':4,5]pyrazino[2,1-*b*][1,3]oxazepine-10-carboxamide (1.3.1), |
| (2*R*,5*S*,13a*R*)-8-hydroxy-*N*-[(2,4,6-triffuoropyridin-3-yl)methyl]-7,9-dioxo-2,3,4,5,7,9,13,13a-octahydro-2,5-methanopyrido[1',2':4,5]pyrazino[2,1-*b*][1,3]oxazepine-10-carboxamide (1.3.2), |
| (2*R*,5*S*,13a*R*)-8-hydroxy-*N*-[(3,5-difluoropyridin-4-yl)methyl]-7,9-dioxo-2,3,4,5,7,9,13,13a-octahydro-2,5-methanopyrido[1',2':4,5]pyrazino[2,1-*b*][1,3]oxazepine-10-carboxamide (1.3.3), |
| (3a*S*,6*R*,13a*S*)-9-hydroxy-*N*-[(2,6-difluoropyridin-3-yl)methyl]-6-methyl-8,10-dioxo-1,2,3,5,6,8,10,13a-octahydrocyclopenta[*b*][1,3]oxazolo[3,2-*a*]pyrido[1,2-*d*]pyrazine-11-carboxamide (2.1.1), |
| (3a*S*,6*R*,13a*S*)-9-hydroxy-*N*-[(3,5-difluoropyridin-4-yl)methyl]-6-methyl-8,10-dioxo-1,2,3,5,6,8,10,13a-octahydrocyclopenta[*b*][1,3]oxazolo[3,2-*a*]pyrido[1,2-*d*]pyrazine-11-carboxamide (2.1.2), |
| (3a*S*,6*R*,13a*S*)-9-hydroxy-6-methyl-*N*-[(2,4,6-trifluoropyridin-3-yl)methyl]-8,10-dioxo-1,2,3,5,6,8,10,13a-octahydrocyclopenta[*b*][1,3]oxazolo[3,2-*a*]pyrido[1,2-*d*]pyrazine-11-carboxamide (2.1.3), |
| (3a*S*,6*S*,13a*S*)-9-hydroxy-*N*-[(2,6-difluoropyridin-3-yl)methyl]-6-methyl-8,10-dioxo-1,2,3,5,6,8,10,13a-octahydrocyclopenta[*b*][1,3]oxazolo[3,2-*a*]pyrido[1,2-*d*]pyrazine-11-carboxamide (2.2.1), |
| (3a*S*,6*S*,13a*S*)-9-hydroxy-*N*-[(3,5-difluoropyridin-4-yl)methyl]-6-methyl-8,10-dioxo-1,2,3,5,6,8,10,13a-octahydrocyclopenta[*b*][1,3]oxazolo[3,2-*a*]pyrido[1,2-*d*]pyrazine-11-carboxamide (2.2.2), |
| (3a*S*,6*S*,13a*S*)-9-hydroxy-6-methyl-*N*-[(2,4,6-trifluoropyridin-3-yl)methyl]-8,10-dioxo-1,2,3,5,6,8,10,13a-octahydrocyclopenta[*b*][1,3]oxazolo[3,2-*a*]pyrido[1,2-*d*]pyrazine-11-carboxamide (2.2.3), or any stereoisomer, any pharmaceutically acceptable salt, any solvate, or any crystalline or polycrystalline form thereof. |

**8.** A method to produce compounds of general formula 1 or 2 according to claim 1 and stereoisomers thereof by the interaction of the corresponding bromides of general formula 3 or 4 and heterocyclylmethylamines in dimethyl sulfoxide in the presence of CO and $Pd(PPh_3)_4$ at elevated temperature followed by debenzylation of the resulting compound 5 or 6

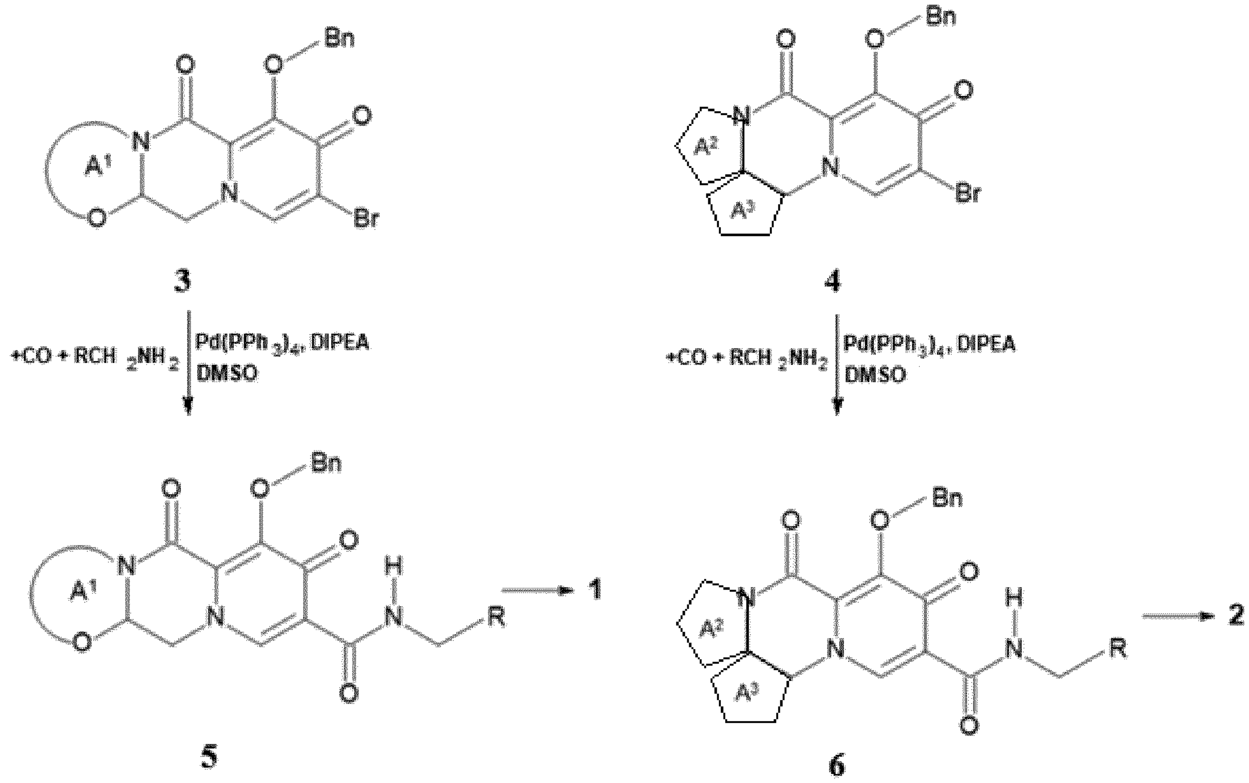


wherein $A^1$, $A^2$, $A^3$, R, and Bn are as given above.

**9.** A method to produce compounds of general formula 1 or 2 and stereoisomers thereof by acylation of heterocyclylmethylamines by the corresponding acids of the compounds of general formula 7 or 8 followed by debenzylation of the resulting compound of formula 5 or 6

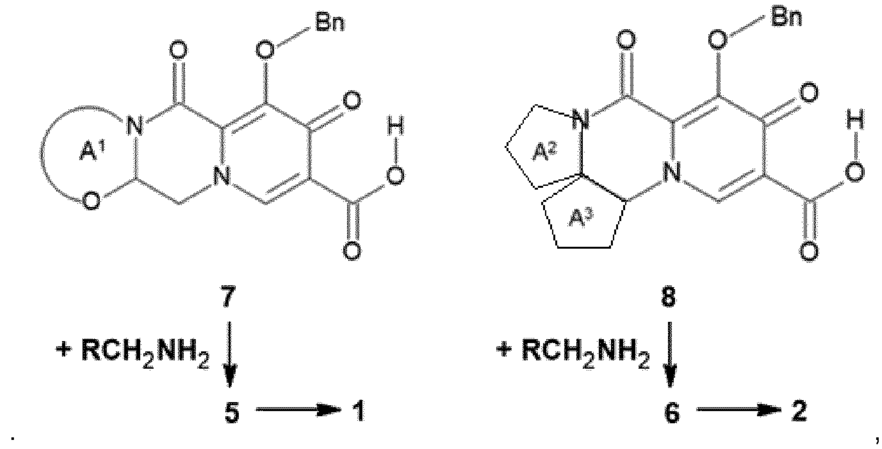


wherein $A^1$, $A^2$, $A^3$, and Bn are as given above.

**10.** A pharmaceutical composition containing a compound of general formula 1 or 2 according to any one of claims 1-7 in a pharmaceutically effective amount and a pharmaceutically acceptable excipient.

**11.** The pharmaceutical composition according to claim 10 containing one or more additional therapeutic agents.

**12.** The pharmaceutical composition according to claim 10 containing an anti-HIV agent as an additional therapeutic agent.

**13.** The pharmaceutical composition according to claim 12 containing one or more additional therapeutic agents selected from the group consisting of HIV protease inhibitors, HIV non-nucleoside reverse transcriptase inhibitors, HIV nucleoside or nucleotide reverse transcriptase inhibitors, HIV capsid assembly inhibitors and combinations thereof.

**14.** The pharmaceutical composition according to any one of claims 11-13 containing a first additional therapeutic agent selected from the group consisting of abacavir sulfate, tenofovir, tenofovir disoproxil fumarate, tenofovir alafenamide, tenofovir alafenamide hemifumarate, tenofovir cyclobutylalafenamide, tenofovir cyclobutylalafenamide hemifumarate, and tenofovir cyclobutylalafenamide fumarate, elsulfavirine, and VM-1500A, GS-CA1 and a second additional therapeutic agent selected from the group consisting of emtricitabine and lamivudine.

**15.** The pharmaceutical composition according to claim 11 containing one or more anti-HBV agents as additional therapeutic agents.

**16.** The pharmaceutical composition according to claim 11 containing one or more anti-HCV agents as additional therapeutic agents.

**17.** The pharmaceutical composition according to claim 11 containing one or more anti-HBV agents and one or more anti-HCV agents as additional therapeutic agents.

**18.** The pharmaceutical composition according to claim 10 in the form of a lyophilizate obtained by freeze drying a nanosuspension of a compound of general formula 1 or 2 in a pharmaceutically effective amount according to any one of claims 1-7 with a particle size of 200 to 900 nm, preferably 200 nm, containing pharmaceutically acceptable excipients.

**19.** The method to prepare a pharmaceutical composition according to claim 18, said method consisting in grinding wet granules of the compound of general formula 1 or 2 according to any one of claims 1-7 with excipients and water to obtain a particle size of 200 to 900 nm, preferably 200 nm followed by lyophilization of the resulting suspension.

**20.** The method according to claim 19 wherein excipients are selected from mannite, polysorbate, polyethylene glycol, poloxamer, mannitol, and sucrose.

**21.** A pharmaceutical nanosuspension to be used as an injectable drug for long-term maintenance therapy of HIV infection containing the pharmaceutical composition according to claim 18, a phosphate buffered saline, and water for injection.

**22.** A method to prepare a pharmaceutical nanosuspension by mixing the pharmaceutical composition of claim 18, a phosphate buffered saline (PBS) with pH = 6.8, and water for injection.

**23.** An injectable drug for long-term maintenance therapy of HIV infection containing the pharmaceutical composition according to claim 18, a phosphate buffered saline, and water for injection.

**24.** A method to prepare an injectable drug consisting in mixing the pharmaceutical composition of claim 18, a phosphate buffered saline (PBS) with a pH of 6.8, and water for injection.

**25.** A method for the prevention and treatment of HIV infection in a HIV-infected or HIV-exposed individual by administering to said individual a therapeutically effective amount of the compound of general formula 1 or 2 according to any one of claims 1-7, or the pharmaceutical composition according to any one of claims 10-18, or the pharmaceutical nanosuspension according to claim 21, or the injectable drug according to claim 23.

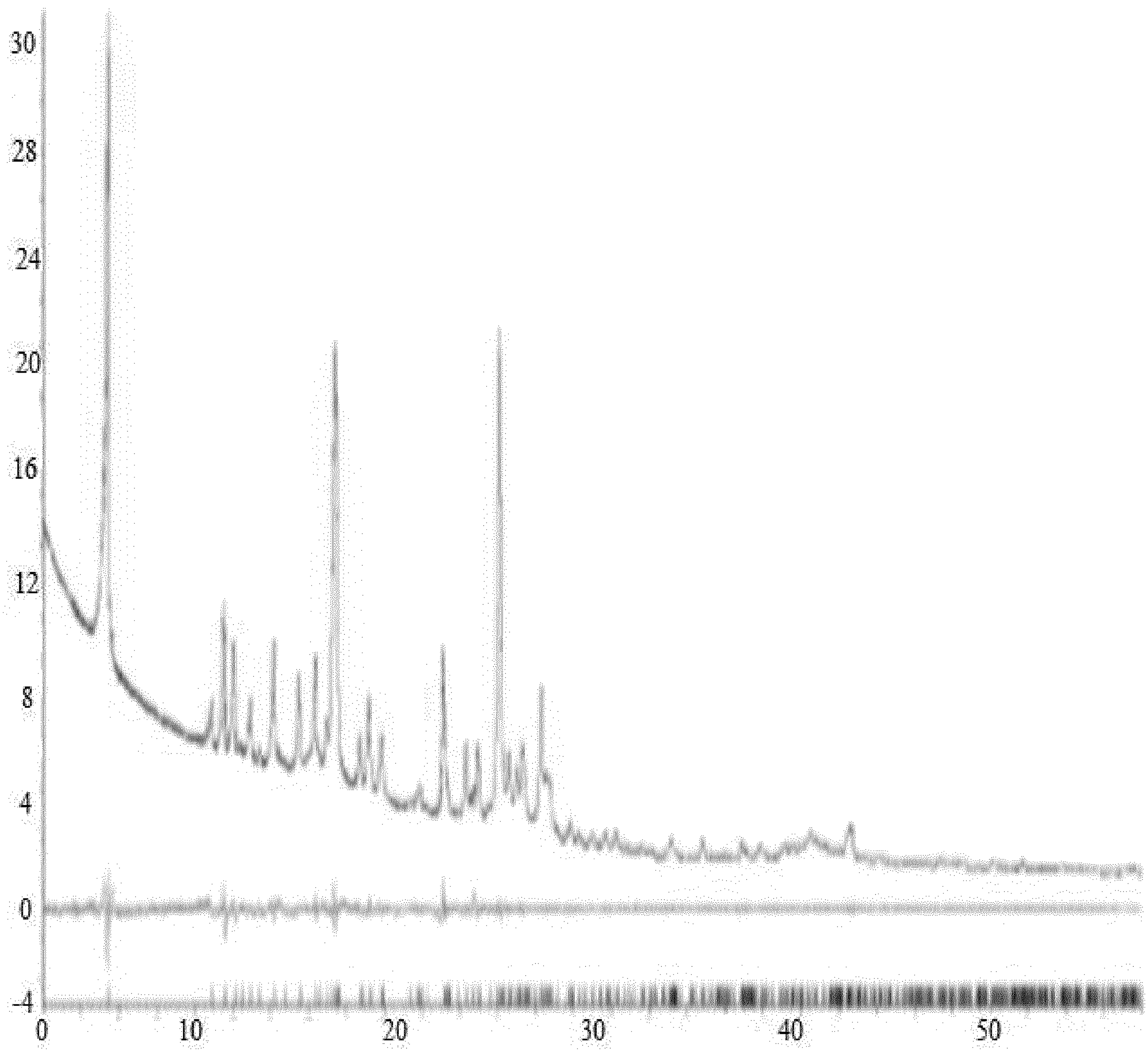


Figure 1

**Human plasma**

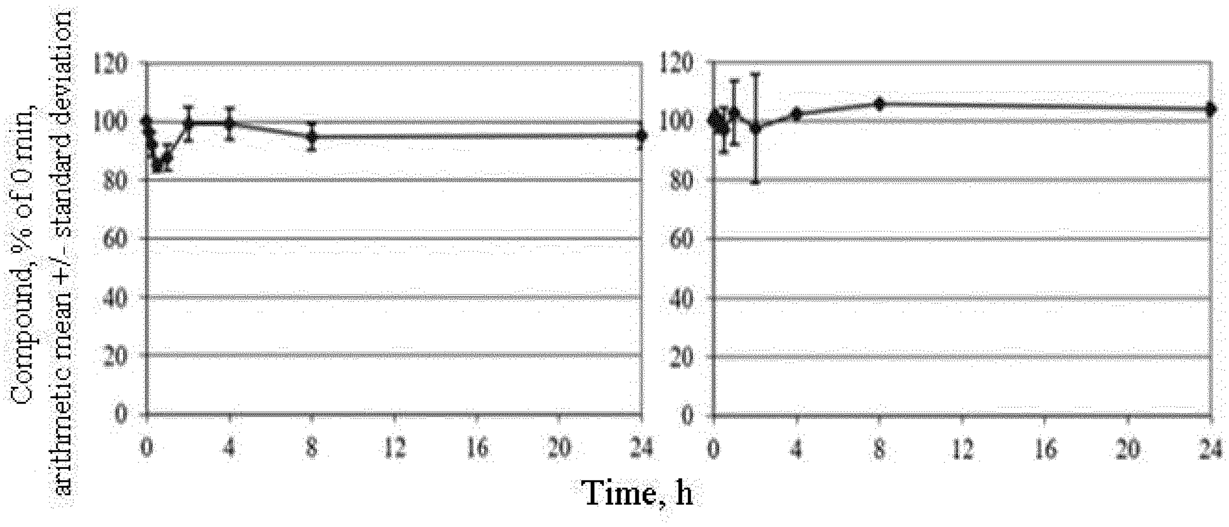


**1.1.16** **1.1.17**

Compound, % of 0 min, arithmetic mean +/- standard deviation

Time, h

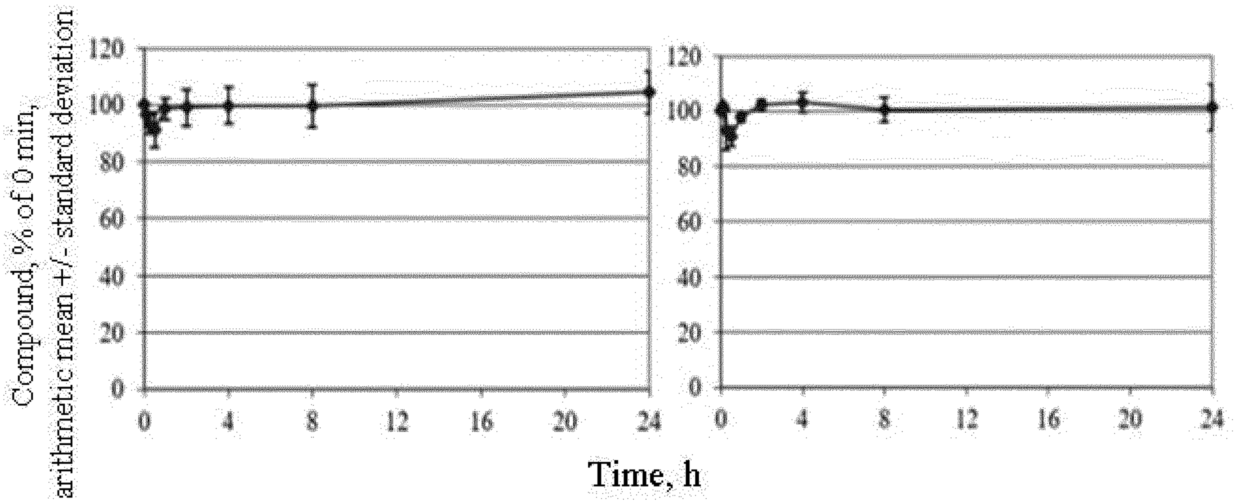


**1.1.19** **1.1.23**

Compound, % of 0 min, arithmetic mean +/- standard deviation

Time, h

Figure 2a

**Rat plasma**

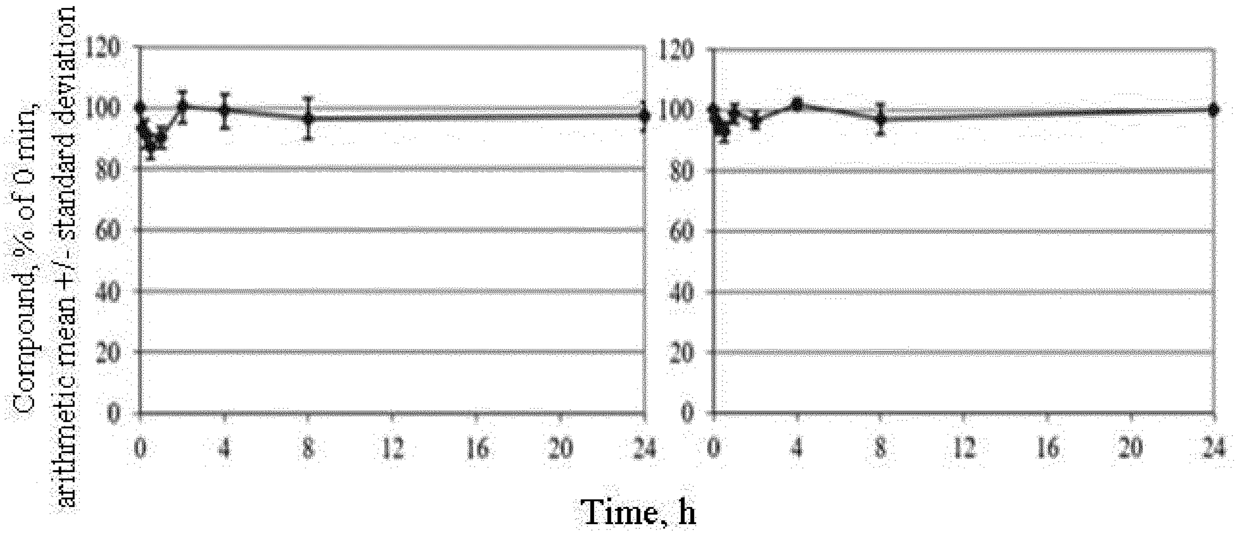


**1.1.16** **1.1.17**

Compound, % of 0 min, arithmetic mean +/- standard deviation

Time, h

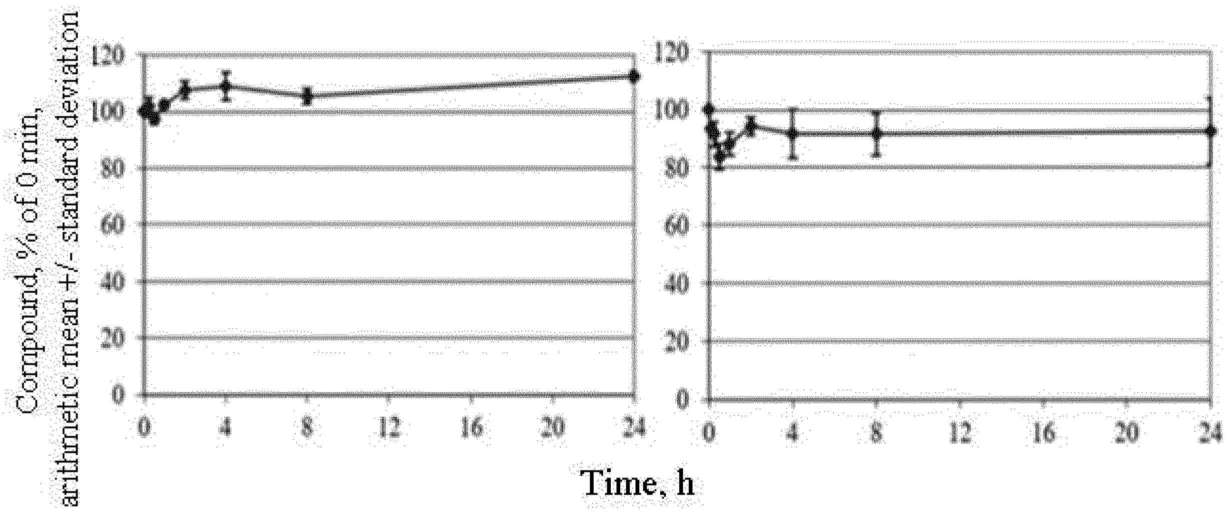


**1.1.19** **1.1.23**

Compound, % of 0 min, arithmetic mean +/- standard deviation

Time, h

Figure 2b

**Human liver S9 fraction**

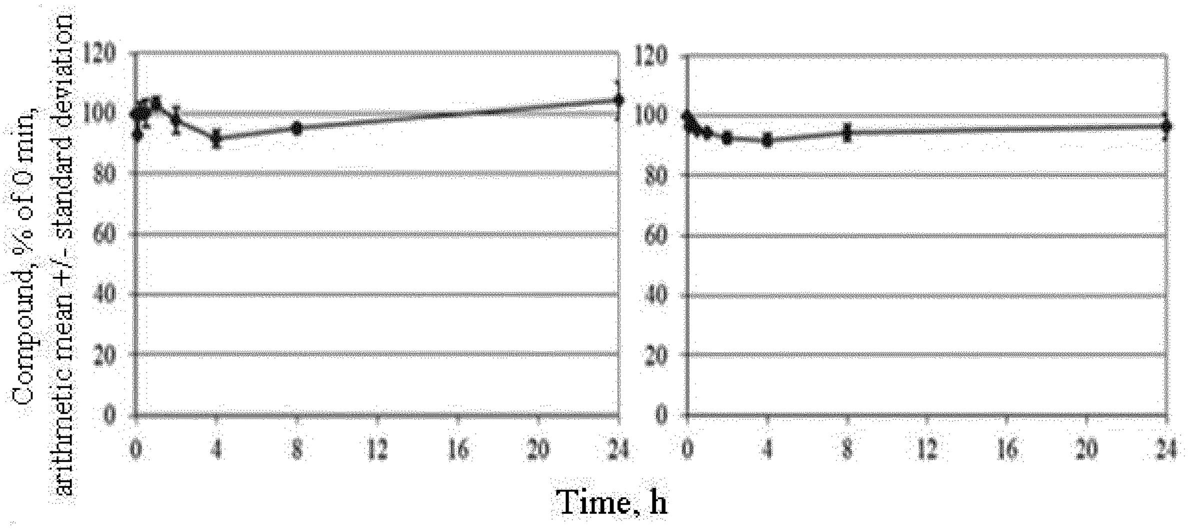


**1.1.16** **1.1.17**

Compound, % of 0 min, arithmetic mean +/- standard deviation

Time, h

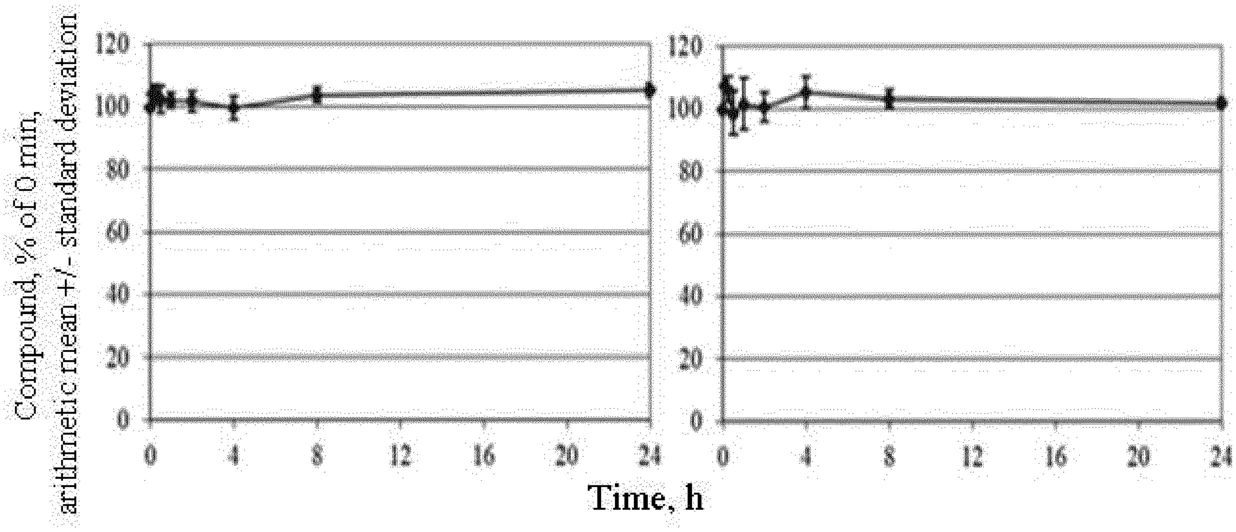


**1.1.19** **1.1.23**

Compound, % of 0 min, arithmetic mean +/- standard deviation

Time, h

Figure 3a

**Rat liver S9 fraction**

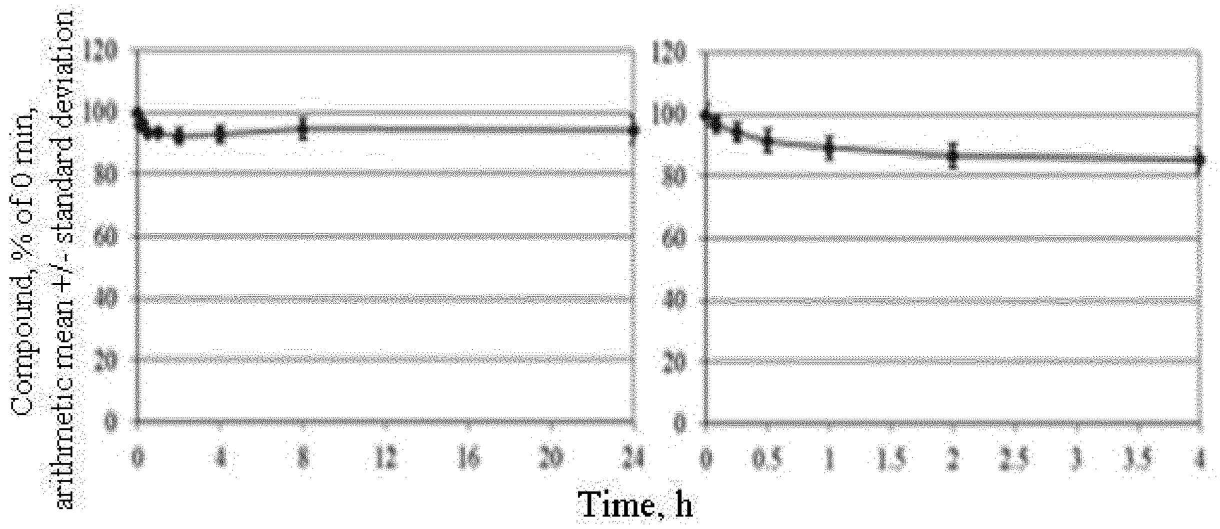


**1.1.16** **1.1.17**

Compound, % of 0 min, arithmetic mean +/- standard deviation

Time, h

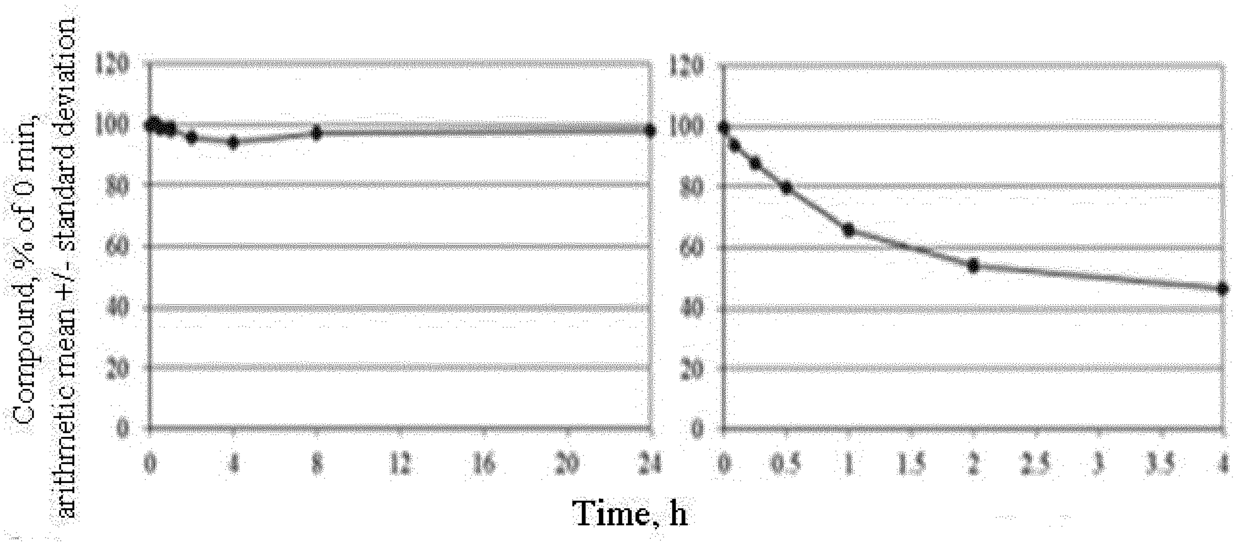


**1.1.19** **1.1.23**

Compound, % of 0 min, arithmetic mean +/- standard deviation

Time, h

Figure 3b

**INTERNATIONAL SEARCH REPORT**

International application No.
PCT/RU 2020/000118

A. CLASSIFICATION OF SUBJECT MATTER

see the supplemental sheet

According to International Patent Classification (IPC) or to both national classification and IPC

B. FIELDS SEARCHED

Minimum documentation searched (classification system followed by classification symbols)

C07D 498/14, 498/18, A61K 31/5365, 31/537, 31/553, 31/4985, A61P 31/18

Documentation searched other than minimum documentation to the extent that such documents are included in the fields searched

Electronic data base consulted during the international search (name of data base and, where practicable, search terms used)

EAPATIS, ESPACENET, PatSearch (RUPTO internal), Information Retrieval System of FIPS, USPTO, PATENTSCOPE, Google, Reaxys, STNext

C. DOCUMENTS CONSIDERED TO BE RELEVANT

| Category* | Citation of document, with indication, where appropriate, of the relevant passages | Relevant to claim No. |
|---|---|---|
| X | US 2013/0096109 A1 (SHIONOGI & SO., LTD.) 18.04.2013, the claims, examples 1-2, abstract, tables 1-2, compound A-7, tables 1-3, compound A-11, A- 12, A-13, tables 1-4 compound A-18, tables 1-5, compound A-20, A-22, tables 1-6 compound A-24, A-25, A-26, A-27, tables 1-7, compound A-29, A-30, table 1-9, compound A-39, A-41, tables 1-10, compound A-43, A-44, A-45, tables 1-12, compound A-52, A-54, tables 1-13, compound A-56, A-57, A-59, tables 1-14, compound A-66, paragraph [0055] | 1-3, 7, 9, 10, 25 |
| Y | | 8, 11-24 |
| X, D | WO 2014/100323 A1 (GILEAD SCIENCES, INC.) 26.06.2014, p. 3, 23 compound PA, examples 1-3, 49, claims 1, 47, 95-108, p. 12, lines 17-20, page 30, lines 17-27, pages 31-39 | 1-3,7, 9-17, 25 |
| Y, D | | 6,8, 11-25 |

[X] Further documents are listed in the continuation of Box C.  [ ] See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 07 August 2020 (07.08.2020) | 10 September 2020 (10.09.2020) |
| Name and mailing address of the ISA/ RU | Authorized officer |
| Facsimile No. | Telephone No. |

Form PCT/ISA/210 (second sheet) (April 2005)

**INTERNATIONAL SEARCH REPORT**

International application No.
PCT/RU 2020/000118

C (Continuation). DOCUMENTS CONSIDERED TO BE RELEVANT

| Category* | Citation of document, with indication, where appropriate, of the relevant passages | Relevant to claim No. |
|---|---|---|
| X, D | WO 2016/161382 A1 (GILEAD SCIENCES, INC.) 06.10.2016, the claims, abstract, p. 35-39 | 1-3, 6, 8-25 |
| Y | RU 2620269 C1 (FEDERALNOE GOSUDARSTVENNOE BIUDZHETNOE UCHREZHDENIE INSTITUT ELEMENTOOGRANICHESKIKH SOEDINENII IM. A.N. NESMEIANOVA ROSSIISKOI AKADEMII NAUK (INEOS RAN) 24.05.2017, plan 4 | 8 |
| Y | RU 2552324 C2 (KIAZMA INK.) 10.06.2015, abstract, claims 1, 3-6 | 18-24 |
| Y | WO 2017/075369 A1 (PFIZER INC.) 04.05.2017, paragraphs [0091], [0098],[0164], [0173], claims 30, 53, 54 | 18-24 |
| A | EA 200702080 A1 (SMITHKLINE BEECHAM CORPORATION et al.) 28.04.2008, the claims | 1-25 |

Form PCT/ISA/210 (continuation of second sheet) (April 2005)

**INTERNATIONAL SEARCH REPORT**

International application No.
PCT/RU 2020/000118

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☒ Claims Nos.: 4, 5
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

INTERNATIONAL SEARCH REPORT

International application No.
PCT/RU 2020/000118

CLASSIFICATION OF SUBJECT MATTER

***C07D 498/14*** *(2006.01)*
***C07D 498/18*** *(2006.01)*
***A61K 31/5365*** *(2006.01)*
***A61K 31/537*** *(2006.01)*
***A61K 31/553*** *(2006.01)*
***A61K 31/4985*** *(2006.01)*
***A61P 31/18*** *(2006.01)*

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005102989 A
- RU 2389719
- WO 2010028968 A
- RU 2659388
- RU 2647576
- WO 2018035359 A
- EP 1422218 A
- WO 2006116764 A
- US 8129385 B
- US 8778943 B
- EP 3260457 A
- WO 2014100323 A
- EP 3196201 A
- WO 2016161382 A
- RU 2644256
- WO 2012074437 A
- US 9428491 B
- RU 2674576
- WO 2013006738 A
- US 20130165489 A
- WO 2013091096 A1
- WO 2009062285 A
- US 20140221380 A
- US 20140221378 A
- WO 2010130034 A
- WO 2013159064 A
- WO 2012145728 A
- WO 2012003497 A
- WO 2012003498 A
- WO 2013006792 A
- WO 2004096286 A
- WO 2006110157 A
- WO 2006015261 A


### Non-patent literature cited in the description


- **WEISS R.A.** How does HIV cause AIDS. *Science,* 1993, vol. 260 (5112), 1273-1279
- **DOUEK D.C. ; ROEDERER M. ; KOUP R.A.** Emerging Concepts in the Immunopathogenesis of AID. *Annu. Rev. Med.,* 2009, vol. 60, 471-84
- **BARRÉ-SINOUSSI F.** Isolation of a T-lymphotropic retrovirus from a patient at risk for acquired immune deficiency syndrome (AIDS). *Science,* 1983, vol. 220 (4599), 868-871
- **GALLO R. C.** Isolation of human T-cell leukemia virus in acquired immune deficiency syndrome (AIDS). *Science,* 1983, vol. 220 (4599), 865-867
- **HASSOUNAH S.A. et al.** *AAC,* 2017, vol. 61 (12), e01695-17
- **YOSHINAGA T. et al.** *AAC,* 2015, vol. 59 (1), 397-406, https://aac.asm.org/content/aac/59/1/397full.pdf; https://aac. asm.org/content/ 61/12/e01695-17.long
- **TSIANG M. et al.** *AAC,* 2016, vol. 60 (12), 7086-7097, https://aac.asm.org/content/60/ 12/7086
- **TREZZA C et al.** Formulation and pharmacology of long-acting cabotegravir. *Curr. Opin. HIV AIDS.,* 2015, vol. 10 (4), 239-245
- **T.D. MCPHERSON et al.** Cabotegravir in the treatment and prevention of Human Immunodeficiency Virus-1. *Expert Opin Investig Drugs.,* 2018, vol. 27 (4), 413-420
- **C.D. ANDREWS et al.** Cabotegravir long acting injection protects macaques against intravenous challenge with SIVmac251. *AIDS,* 2017, vol. 31 (4), 461-467
- **NOGRADY'S.** Medicinal Chemistry: A Biochemical Approach. Oxford University Press, 1985, 388-392
- **IVACHTCHENKO, A. V. et al.** Discovery of Novel Highly Potent Hepatitis C Virus NS5A Inhibitor (AV-4025). *J. Med. Chem.,* 2014, vol. 57, 7716-7730
- **V. SORIANO et al.** Treatment of hepatitis C with new fixed dose combinations. *Expert Opin Pharmacother,* August 2017, vol. 18 (12), 1235-1242
- Remington. The Science and Practice of Pharmacy. Philadelphia College of Pharmacy and Science, 2000
- **FOLEY G.E. et al.** Continuous Culture of Human Lymphoblasts from Peripheral Blood of a Child with Acute Leukemia. *Cancer,* 1965, vol. 18, 522-529
- **POPOVIC, M. et al.** Detection, isolation, and continuous production of cytopathic retroviruses (HTLV-III) from patients with AIDS and pre-AIDS. *Science,* 1984, vol. 224 (4648), 497-500

- **NARA, P.L.** Simple, rapid, quantitative, syncytium-forming microassay for the detection of human immunodeficiency virus neutralizing antibody. *AIDS Res. Hum. Retroviruses,* 1987, vol. 3, 283-302